# EUROPEAN PATENT SPECIFICATION

(11) **EP 1 933 926 B1**
(45) Date of publication and mention of the grant of the patent: **24.10.2012**
(21) Application number: 06779437.0
(22) Date of filing: 15.09.2006
(51) Int. Cl.: A61M 1/00, A61B 17/70, A61M 3/02

(54) **APPARATUS WITH ACTIVES FROM TISSUE**
GERÄT MIT WIRKSTOFFEN AUS GEWEBE
APPAREIL FAISANT INTERVENIR DES SUBSTANCES ACTIVES EN PROVENANCE DE TISSUS

(30) Priority: 15.09.2005 GB 0518826
(43) Date of publication of application: 25.06.2008
(60) Divisional application: 10006422.9
(73) Proprietor: Smith & Nephew, PLC, London WC2N 6LA (GB)
(72) Inventor: BLOTT, Patrick, Lewis, York YO42 4ER (GB); GREENER, Bryan, York YO42 4BG (GB); HARTWELL, Edward, Yerbury, Hull HU15 1EP (GB); LEE-WEBB, Julian, York YO23 3TJ (GB); NICOLINI, Derek, Brough HU15 2ET (GB)
(74) Representative: Connors, Martin L.H.
(86) International application number: PCT/GB2006/003425
(87) International publication number: WO 2007/031765

(56) References cited:
- WO-A-93/09727
- WO-A-2005/046761
- WO-A-2005/105176

## Description

The present invention relates to apparatus and a medical wound dressing for aspirating, irrigating and/or cleansing wounds.

It relates in particular to such an apparatus, wound dressing that can be easily applied to a wide variety of, but in particular chronic, wounds, to cleanse them of materials that are deleterious to wound healing, and adding such materials using cells or tissue, whilst retaining materials that are beneficial in some therapeutic aspect, in particular to wound healing, and adding such materials using cells or tissue.

Before the present invention, aspirating and/or irrigating apparatus therefor were known, and tended to be used to remove wound exudate during wound therapy. In known forms of such wound therapy, the offtake from the wound, especially when in a highly exuding state, is voided to waste, e.g. to a collection bag.

Materials deleterious to wound healing are removed in this way.

However, materials that are beneficial in promoting wound healing, such as growth factors, extracellular matrix components and fragments thereof, and other physiologically active components of the exudate from a wound are lost to the site where they can be potentially of most benefit, i.e. the wound bed, when such therapy is applied.

Additionally, before the present invention, known aspirating and/or irrigating apparatus was only used to remove materials that are deleterious to healing from wound exudate during wound therapy, and not for the delivery from cells or tissue of further materials that are beneficial in promoting wound healing. Examples of the latter include materials from cells or tissue, such as growth factors, extracellular matrix components and fragments thereof, selective proteases or fibrinolytic factors and combinations thereof.

Such known forms of wound dressing and aspiration and/or irrigation therapy systems often create a wound environment under the dressing that thus may result in the loss of optimum performance of the body's own tissue healing and slow healing and/or in weak new tissue growth that does not have a strong three-dimensional structure adhering well to and growing from the wound bed. This is a significant disadvantage, in particular in chronic wounds.

Document WO 2005/046761 A1 describes an apparatus for cleansing and applying therapy or prophylaxis to wounds, in which irrigant fluid containing a physiologically active material from a reservoir connected to a conformable wound dressing and wound exudate from the dressing are recirculated by a device for moving fluid through a flow path which passes through the dressing and a means for fluid cleansing and back to the dressing. The cleansing means (which may be a single-phase, e.g. microfiltration, system or a two-phase, e.g. dialytic system) removes materials deleterious to wound healing, and the cleansed fluid, still containing materials that are beneficial in promoting wound healing, is returned to the wound bed.

It thus would be desirable to provide a system of therapy which
a) can remove materials deleterious to wound healing, whilst
b) retaining materials that are beneficial in promoting wound healing, and adding such materials, e.g. using cells or tissue to be, in contact with the wound bed.

Dialysis is a known method of treating bodily fluids such as blood ex vivo, to cleanse them of materials that are deleterious to the body systemically. Removal of such materials by contact with the dialysate is the prime purpose of dialysis, whilst also retaining materials such as blood, cells and proteins. Other materials that may have an additional positive therapeutic action are potentially lost to the system through the dialysis membrane, which is also permeable to them. The balance of such materials in the bodily fluid in recirculation may thus be further depleted.

It would be desirable to provide a system of therapy that can remove materials deleterious to wound healing, without substantially diluting materials that are beneficial in promoting wound healing, and whilst adding such materials using cells or tissue to be in contact with the wound bed, and which can continuously supply and recirculate such materials to the wound simultaneously.

Dialysis for treating bodily fluids is also a systemic therapy, since the treated fluid is returned to within the body.

This is in contrast to a topical therapy in which the treated fluid is recycled outside the body, e.g. to a wound.

Dialysis also requires large amounts either of bodily fluids, such as blood, or of dialysate, and consequently the relevant devices tend not to be portable.

Even when in a highly exuding state, chronic wounds produce relatively little fluid to be treated compared with internal bodily systems and relatively little materials that are beneficial in some therapeutic aspect to be retained in the wound and/or its environment.

It is an object of the present invention
a) to obviate at least some of the abovementioned disadvantages of known aspiration and/or irrigation therapy systems, and
b) to provide a system of therapy which can remove materials deleterious to wound healing, whilst retaining materials that are beneficial in promoting wound healing, and whilst adding such materials using cells or tissue to be, in contact with the wound bed.

It is a further object of the present invention
a) to obviate at least some of the abovementioned disadvantages of known dialysis systems, and
b) to provide a system of therapy which can remove materials deleterious to wound healing, whilst retaining materials that are beneficial in promoting wound healing, and whilst adding such materials using cells or tissue to be, in contact with the wound bed,
c) without affecting the body systemically.

It is a yet further object of the present invention
a) to obviate at least some of the abovementioned disadvantages of known dialysis systems, and
b) to provide a system of therapy which can remove materials deleterious to wound healing, whilst retaining materials that are beneficial in promoting wound healing, and whilst adding such materials using cells or tissue to be, in contact with the wound bed, and
c) is portable.

It is a further object of the present invention
a) to obviate at least some of the disadvantages of known dialysis systems, and
b) to provide a system of therapy which can remove materials deleterious to wound healing from wound exudate, whilst retaining materials that are beneficial in promoting wound healing, and
c) further supplies fluids containing active amounts of materials that are beneficial in promoting wound healing using cells or tissue to pass into and/or through the wound in contact with the wound bed.

Vascular supply to, and circulation in, tissue underlying and surrounding the wound is often compromised.

It is a further object of the present invention to provide a system of therapy that retains therapeutically active amounts of materials that are beneficial in reversing this effect and supplies such materials using cells or tissue, whilst removing deleterious materials, thereby promoting wound healing.

According to the present invention there is provided an apparatus (1;21) for aspirating, irrigating and/or cleansing wounds, which comprises a fluid flowpath, comprising
i) a wound dressing (2), having a backing layer (3) and
   at least one inlet pipe (16) for connection to a fluid supply tube (7), which passes through and/or under the backing layer and at least one outlet pipe (9) for connection to a fluid offtake tube (10), which passes through and/or under the backing layer,
   at least one inlet pipe being connected to a fluid recirculation tube (13), and at least one outlet pipe being connected to a fluid offtake tube; and
ii) a means for fluid cleansing (17) having at least one inlet port connected to a fluid offtake tube and at least one outlet port connected to a fluid recirculation tube;
iii) a device for moving fluid (18;926;918) through the wound dressing and the means for fluid cleansing (17); and characterised in that the apparatus further comprises means for supplying physiologically active agents from cells or tissue to the wound, wherein said means comprises
   (a) an irrigant reservoir connected to;
   (b) a container that contains a cell or tissue component, in turn connected to;
   (c) a supply tube in the flowpath
such that fluid may be supplied to fill the flowpath and supply a physiologically active agents from cells or tissue to the wound and recirculated by the device through the flow path.

The aspirating and irrigating may be sequentially or simultaneously.

Where any pipe is described in connection with the operation of the apparatus as being connected or for connection to a (mating end of a) tube, e.g. a fluid supply tube, fluid recirculation tube or fluid offtake tube, the pipe and the tube may form a single integer in the flow path through which the circulating fluid from the wound passes.

The prolonged delivery of such physiologically active components in therapeutically active amounts in a precise and time-controlled manner, together with
a) the removal of materials deleterious to wound healing from wound exudate,
b) without substantially diluting materials that are beneficial in promoting wound healing (including such materials that have been added using cells or tissue) in contact with the wound bed, and
c) the continuously supply and recirculation of such materials to the wound,
promotes greater wound healing than
i) by treatment with the fluid physiologically active component(s) alone, or
ii) by topical bolus delivery.

Advantages over topical bolus delivery include greater bioavailability to all areas of the wound surface, prolonged delivery between dressing changes and optimal dosing. For example, factors such as TGFβ show different effects at high and low concentrations.

Consequently, undesirable effects may be the result of an unnecessarily high dose to ensure prolonged residence between topical applications.

Supply to the wound bed under a positive pressure may be advantageous, as application of a positive pressure to the wound under the backing layer may make it possible to flood the tissue underlying the wound with one or more physiologically active components, added using cells or tissue, in therapeutically' active amounts, to promote greater wound healing, than by treatment with static fluid physiologically active component(s) alone.

It is believed that by using the apparatus for irrigating and/or aspirating wounds of the present invention cyclically and/or with reversal of flow, the effects may be further enhanced.

The means for supplying physiologically active agents from cells or tissue to the wound often conveniently comprises
a) an irrigant reservoir connected to
b) a container that contains a cell or tissue component, in turn connected to
c) a supply tube into the flowpath.

The supply of physiologically active agents from cells or tissue will often occur into the conformable wound dressing.

In use, irrigant is passed from the reservoir through the container that contains the cells or tissue and exits from it containing one or more physiologically active component materials that are beneficial in promoting wound healing that are expressed by the cells or tissue.

The modified irrigant (including such physiologically active agents as have been added from the cells or tissue) is moved by the device for moving fluid through the supply tube and dressing to the wound. Then in admixture with wound exudate it is moved along the flow path, through the offtake tube.

Thus, one embodiment of the apparatus for irrigating, cleansing and/or aspirating wounds of the present invention is characterised in that the means for supplying physiologically active agents from cells or tissue to the wound comprises
a) an irrigant reservoir connected to
b) a container that contains a cell or tissue component, in turn connected to
c) a supply tube.

In use, irrigant is passed from the reservoir through the container that contains the cells or tissue and exits from it containing one or more physiologically active component materials that are beneficial in promoting wound healing that are expressed by the cells or tissue. The modified irrigant (including such physiologically active agents as have been added from the cells or tissue) is moved by a device for moving fluid through the supply tube and dressing to the wound. Then in admixture with wound exudate it is moved along the flow path, through the offtake tube.

In another embodiment of the apparatus for irrigating, cleansing and/or aspirating wounds, the means for supplying physiologically active agents from cells or tissue to the wound comprises
a) an irrigant reservoir, and
b) a container that contains a cell or tissue component,
d) both connected in parallel to a supply tube for supplying physiologically active agents from cells or tissue and irrigant to the wound under the action of at least one device for moving fluid through the wound.

In this embodiment of the apparatus, the irrigant reservoir and the container that contains a cell or tissue component may be, e.g. connected to the supply tube by a Y-junction.

In use, irrigant is passed from the reservoir to the supply tube, and a fluid (which may be a nutrient medium for the cells or tissue) containing one or more physiologically active component materials that are beneficial in promoting wound healing that are expressed by the cells or tissue is passed from the container that contains the cells or tissue to the supply tube. The irrigant in admixture with such physiologically active agents as have been added from the cells or tissue is moved by a device for moving fluid through the wound to and through the wound.

In yet another embodiment of the apparatus for irrigating, cleansing and/or aspirating wounds, the means for supplying physiologically active agents from cells or tissue to the wound comprises
a) an irrigant reservoir, connected to
b) a first supply tube for supplying irrigant to the wound under the action of at least one device for moving fluid through the wound, and
c) a container that contains a cell or tissue component, connected to
d) a second supply tube for supplying physiologically active agents from the cells or tissue the wound dressing.

In use, irrigant is passed from the reservoir to the first supply tube for supplying irrigant to the wound. The fluid containing one or more physiologically active component materials that are beneficial in promoting wound healing that are expressed by the cells or tissue is passed from the container that contains the cells or tissue to the second supply tube for supplying physiologically active agents from the cells or tissue to the wound dressing. Each is moved by a device for moving fluid through the wound to and through the wound. The irrigant is admixed in the wound space with the physiologically active agents that have been added from the cells or tissue.

In a further embodiment of the apparatus for irrigating, cleansing and/or aspirating wounds of the present invention, the means for supplying physiologically active agents from cells or tissue to the wound comprises
a) an irrigant reservoir connected to
b) a container that contains a cell or tissue component, under the backing layer, and which communicates with the wound via at least one channel or conduit for supplying physiologically active agents from cells or tissue and irrigant to the wound under the action of at least one device for moving fluid through the wound.

The container that contains a cell or tissue component may be integral with the other components of the dressing, in particular the backing layer. Alternatively, it may be permanently or demountably attached to them/it, with an adhesive film, for example, or by heat-sealing.

In use, irrigant is passed from the reservoir through the container that contains the cells or tissue and exits from it into the wound space under the backing layer proximal face containing one or more physiologically active component materials that are beneficial in promoting wound healing that are expressed by the cells or tissue.

In yet a further embodiment of the apparatus for irrigating, cleansing and/or aspirating wounds of the present invention, the means for supplying physiologically active agents from cells or tissue to the wound comprises
a) a first irrigant reservoir connected to
b) a supply tube for supplying irrigant to the wound under the action of at least one device for moving fluid through the wound, and
c) a second irrigant reservoir connected to
d) a container that contains a cell or tissue component, under the backing layer, and which communicates with the wound via at least one channel or conduit for supplying physiologically active agents from cells or tissue and irrigant to the wound under the action of at least one device for moving fluid through the wound.

The container that contains a cell or tissue component may be integral with the other components of the dressing, in particular the backing layer. Alternatively, it may be permanently or demountably attached to them/it, with an adhesive film, for example, or by heat-sealing.

In use, irrigant is passed from the first reservoir to the supply tube for supplying irrigant to the wound. Irrigant is also passed from the second reservoir to the container.

The fluid containing one or more physiologically active component materials that are beneficial in promoting wound healing that are expressed by the cells or tissue is passed from the container that contains the cells or tissue to the second supply tube for supplying physiologically active agents from the cells or tissue to the wound dressing. Each is moved by a device for moving fluid through the wound to and through the wound.

The irrigant is admixed in the wound space with the modified irrigant containing physiologically active agents that have been added from the cells or tissue.

All of these embodiments of the means for supplying physiologically active agents from cells or tissue to the wound may use cells or tissues of two or more different types. In such systems, a first input cell or tissue type is often contained in a first container, and a second input cell or tissue type is often contained in a second container.

The two input cell or tissue types and containers may feed physiologically active agents in parallel to the dressing and to the wound bed under the action of at least one device for moving fluid through the wound.

In this embodiment of the apparatus, the containers that contain the cell or tissue components may be, e.g. connected to a single supply tube by a Y-junction, and thence to the wound dressing, or they may, e.g. be connected to it by separate supply tubes, the two flows of physiologically active agents from cells or tissue optionally with irrigant and/or nutrient medium for the cells being optionally mutually admixed in the wound space under the wound dressing.

In an alternative layout of this means for supplying physiologically active agents from cells or tissue to the wound, the first container, in which the first input cell or tissue type is contained, is in fluid communication in series with the second container, in which the second cell or tissue type is contained.

Thus, they feed their physiologically active agents in series to the dressing and to the wound bed under the action of at least one device for moving fluid through the wound. In this layout of the means for supplying physiologically active agents from cells or tissue, the two containers effectively function as a single container.

As noted above, irrigant and/or nutrient medium for the cells or tissue is often fed through the containers of the cell or tissue components and thence to the wound dressing. In use, these layouts of the means for supplying physiologically active agents from cells or tissue to the wound will function in the apparatus exactly as for their analogues with a single cell or tissue type.

The container is often in the form of a hollow body such as e.g. a canister, cartridge or cassette, with a chamber or compartment that contains a cell or tissue component, through which the irrigant is passed.

Where the container that contains a cell or tissue component lies outside the backing layer, the structure will often be made of glass, and/or synthetic polymeric materials. For example, such a structure may be a glass cylinder defining a chamber with axial inlet and outlet ports for throughflow, which contains cells or tissue on a scaffold.

Where the container that contains a cell or tissue component lies under the backing layer, the structure will often be made of a conformable synthetic polymeric material.

Such a structure may still be a structure defining a chamber with an inlet port, which contains cells or tissue on a scaffold, and which communicates with the wound via at least one channel or conduit.

The latter is/are for supplying physiologically active agents from cells or tissue and irrigant to the wound under the action of at least one device for moving fluid through the wound.

Where the container that contains a cell or tissue component is integral with the other components of the dressing, in particular the backing layer, it will usually be of the same polymeric material as the components. Where, alternatively, it is permanently or demountably attached to them/it, with an adhesive film, for example, or by heat-sealing, it may be of a different polymeric material.

It may contain a cell or tissue component that is not bound to an insoluble and immobilised substrate over and/or through which the irrigant and/or wound exudate from the wound dressing passes.

Any such structure may contain a cell or tissue component that is not bound to an insoluble and immobilised substrate over and/or through which the irrigant and/or wound exudate from the wound dressing passes.

It then also appropriately comprises two or more integers which are permeable to the wound exudate or a mixture with irrigant, but have apertures, holes, openings, orifices, slits or pores of sufficiently small cross-dimension to hold the cell or tissue component, and to retain particulates, e.g. cell debris, in the hollow body. Each of the integers may then effectively form a macroscopic and/or microscopic filter.

Alternatively, it may contain a cell or tissue component that is bound to an insoluble and immobilised substrate over and/or through which the irrigant and/or wound exudate from the wound dressing passes, e.g. a scaffold.

This will often be of a material that is not (cyto)toxic and is biocompatible and inert to any components that are beneficial in promoting wound healing, including natural and synthetic polymeric materials.

This may typically be in the form of a conformable film, sheet or membrane, often with apertures, holes, openings, orifices, slits or slots of small cross-dimension.

It may then effectively form a structure which is a mesh, grid, lattice, net or web.

The container for cells or tissue may then not need to comprise two or more integers which are permeable to the wound exudate or a mixture with irrigant to hold the cell or tissue component in the hollow body, but they may be desirable to retain particulates, e.g. cell debris.

The integer that contains the tissue or cell component will normally be mounted within a device constructed to maintain the viability and activity of the cells. This would include but not be limited to means for supplying nutrition and regulating the exchange of gases and maintaining an optimum temperature.

The means for supplying nutrition may comprise a conventional nutrient medium for the cells or tissue containing one or more physiologically active component materials that are beneficial in promoting cell proliferation in the cells or tissue in the container that contains the cells or tissue and/or the expression by such cells or tissue of one or more physiologically active component materials that are beneficial in promoting wound healing.

To achieve therapeutically effective amounts of materials that are beneficial in promoting wound healing, a fluid flow though and/or over the cells or tissue may have to be maintained over multiple cycles, with significant dwell times and/or over significant periods of time.

Thus, in those embodiments of the means for supplying physiologically active agents from cells or tissue to the wound described above, the container that contains a cell or tissue component may be provided with
a) means for recycling nutrient medium for the cells or tissue from and back to a nutrient medium reservoir, e.g. a loop comprising the reservoir, connected to the container that contains the cells or tissue, with a pump, and in particular
b) means for switching fluid flow between recycling around the loop comprising the reservoir and the container and supply to the relevant supply tube.

Such means for switching fluid flow may comprise at least one one-way valve in the loop and in the fluid supply tube, or a two way valve connecting the supply tube and the loop.

In use, nutrient medium for the cells or tissue is recycled from and back to a nutrient medium reservoir in the loop comprising the reservoir and the container that contains the cells or tissue, with a pump, over multiple cycles, with significant dwell times and/or over significant periods of time until the cell proliferation in the cells or tissue in the container that contains the cells or tissue and/or the expression by such cells or tissue of one or more physiologically active component materials that are beneficial in promoting wound healing have achieved the desired levels.

Recycling nutrient medium for the cells or tissue from and back to the nutrient medium reservoir is then stopped, and supply to the relevant supply tube is started.

This may be achieved by stopping the pump and/or closing a one-way valve in the loop and opening on in the supply tube, or by switching a two way valve connecting the supply tube and the loop.

The necessary desired levels of physiologically active component materials, valves, pumps, number of cycles, dwell times and/or time periods will be apparent to the skilled person.

As noted above, in another embodiment of the apparatus of this first aspect of the present invention for aspirating, irrigating and/or cleansing wounds, a particular advantage is that the means for supplying physiologically active agents from cells or tissue to the wound lies within the wound dressing.

In use, irrigant is passed from the reservoir through the cells or tissue component for supplying physiologically active agents to the wound which lies within the wound dressing, and exits from it containing one or more component physiologically active component materials that are beneficial in promoting wound healing that are expressed by the cells or tissue.

The modified irrigant (including such physiologically active agents as have been added from the cells or tissue) in admixture with wound exudate is moved by the device for moving fluid through the offtake tube along the flow path.

Thus, one embodiment of the apparatus for irrigating, cleansing and/or aspirating wounds of the present invention is characterised in that it the means for supplying physiologically active agents from cells or tissue to the wound comprises
a) an irrigant reservoir fluidically connected to
b) a wound dressing that contains a cell or tissue component.

The wound dressing backing layer, which is capable of forming a relatively fluid-tight seal or closure over a wound, and the wound bed define a wound space, which contains cells or tissue. As noted above for a separate container, the wound space may contain a cell or tissue component that is not bound to an insoluble and immobilised substrate over and/or through which the irrigant and/or wound exudate from the wound passes.

It then also appropriately comprises two or more integers which are permeable to the wound exudate or a mixture with irrigant, but have apertures, holes, openings, orifices, slits or pores of sufficiently small cross-dimension to hold the cell or tissue component, and to retain particulates, e.g. cell debris, in the hollow body.

Each of the integers may then effectively form a macroscopic and/or microscopic filter.

Alternatively, it may contain a cell or tissue component that is bound to an insoluble and immobilised substrate over and/or through which the irrigant and/or wound exudate from the wound passes, e.g. a scaffold.

This will often be of a material, and may typically be in the form, noted above as amongst those that are suitable for such components of a separate container that contains a cell or tissue component.

The wound space may contain a cell or tissue component at any appropriate point in contact with the irrigant and/or wound exudate, and the component may be as appropriate, adhered or otherwise secured to any integer of the wound dressing, e.g. the dressing backing layer or a wound filler, or it may be a separate structures, permanently unattached.

It may often lie in contact with the wound bed. Where it does so, it may be advantageous if it is
a) bound to an insoluble and immobilised substrate over and/or through which the irrigant and/or wound exudate from the wound passes, or
b) not bound to an insoluble and immobilised substrate, but comprised in two or more integers which are permeable to the wound exudate or a mixture with irrigant, and
c) comprises a biodegradable mesh, grid, lattice, net or web, with apertures, holes, openings, orifices, slits or pores of small cross-dimension in contact with the wound bed.

The cell or tissue component in contact with continuously supplied and recirculated irrigant and/or wound exudate has the ability to add elements beneficial to wound healing to the irrigant, but the same elements also aid proliferation of wound bed cells into the apertures, holes, openings, orifices, slits or pores of small cross-dimension of the biodegradable mesh, grid, lattice, net or web, which is also beneficial to wound healing.

The tissue component has the ability to elaborate or express materials beneficial to wound healing to the irrigant to modify the irrigant.

As described in further detail hereinafter, such elements beneficial to wound healing may be biochemical, e.g. enzymatic or physical antagonists to elements detrimental to wound healing in the exudate and/or exudate and irrigant.

An additional embodiment of the apparatus for irrigating, cleansing and/or aspirating wounds of the present invention is characterised in that the physiologically active components that have been added using cells or tissue in amounts to promote wound healing comprise materials that are beneficial in promoting wound healing by removing materials or by regulating, limiting or inhibiting processes deleterious to wound healing.

Depending on the particular type of wound being treated and the particular cells or tissue used in the present apparatus for aspirating, irrigating and/or cleansing wounds, the deleterious materials to be removed may include proteases, such as serine proteases, e.g. elastase and thrombin; cysteine proteases; matrix metalloproteases, e.g. collagenase; and carboxyl (acid) proteases; inhibitors of angiogenesis such as thrombospondin-1 (TSP-1), Plasminogen activator inhibitor, or angiostatin (plasminogen fragment)
pro-inflammatory cytokines such as tumour necrosis factor alpha (TNFα) and interleukin 1 beta (IL-1β), and
inflammatories, such as lipopolysaccharides, and e.g. histamine.

Again, depending on the particular type of wound being treated and the particular cells or tissue used in the present apparatus for aspirating, irrigating and/or cleansing wounds, the beneficial materials to be added may include antagonists to the materials deleterious to wound healing in the wound exudate, such as, for example
enzymes or others, such as protease inhibitors, such as serine protease inhibitors, cysteine protease inhibitors; matrix metalloprotease inhibitors; and carboxyl (acid) protease inhibitors;
binders and/or degraders, such as anti-inflammatory materials to bind or destroy lipopolysaccharides, e.g. peptidomimetics;

They further include peptides (including cytokines, e.g. bacterial cytokines, such as α-amino-γ-butyrolactone and L-homocarnosine); and
other physiologically active components.

Examples of antagonists to such materials also include
natural proteins or recombinant-produced protein, proteinase inhibitors, such as tissue inhibitors of metalloproteinases (TIMP 1 to 4) and alpha 1-antitrypsin (AAT), aprotinin, α-2-macroglogulin;
antibodies or other molecules at inappropriate levels that inhibit or inactivate processes or materials deleterious to wound healing, such as matrix metalloproteinases (MMPs), neutrophil elastase, inhibitors of new blood vessel formation (angiogenesis) such as thrombospondin or kallistatin and combinations thereof.

The irrigant may alternatively or additionally, where appropriate, deliver a steady supply of natural proteins or recombinant-produced protein debriding agents to remove and limit eschar, necrotic cells and tissues from the wound bed.

Examples of such include stretoptokinase, plasmin, trypsin, collagenases, and other selective proteases or fibrinolytic factors and combinations thereof.

The irrigant supplied to the wound dressing, preferably may alternatively or additionally, where appropriate, contain materials added using cells or tissue such as
antioxidants, such as ascorbic acid or stable derivatives thereof and
free radical scavengers, such as gutathione or natural proteins or recombinant-produced proteins such as superoxide dismutase (SOD) or free radical generators to balance the oxidative stress and oxidant potential of the wound bed in order to maximise the opportunity for wound healing.

The active material may however act beneficially on the wound bed and have the ability to aid wound healing, as it is passed and recirculated by the device through the flow path, through biochemical, enzymatic or physical means without any such role as a biochemical, enzymatic or physical antagonist.

Examples of such components (however supplied) also include: autologous, allogeneic or xenogeneic blood or blood products, such as platelet lysates, plasma or serum
natural proteins or recombinant-produced protein growth factors, such as platelet derived growth factor (PDGF), vascular endothelial growth factor (VEGF), transforming growth factor alpha (TGFα) or transforming growth factor beta (TGFβ-1, 2 or 3), basic-fibroblast growth factor (b-FGF also known as FGF2), epidermal growth factor (EGF), granulocyte-macrophage colony-stimulating factor (GM-CSF); insulin like growth factor-1 (IGF-1) and keratinocyte growth factor 2 KGF2 (also known as FGF7);
natural purified proteins or recombinant produced protein cytokines such as the interleukin 1β (IL1β), or interleukin 8 (IL-8) and
other physiologically active agents whether present normally in acute or chronic wounds, that can be augmented in the irrigant fluid to be of benefit to the wound bed, when such therapy is applied, and combinations thereof.

The irrigant supplied to the wound dressing may alternatively or additionally, where appropriate, contain materials added using cells or tissue such as nutrients for wound cells to aid proliferation or migration or the synthesis of matrix components or factors beneficial to wound healing, such as sugars, amino acids, purines, pyrimidines, vitamins, metal ions or minerals.

The irrigant supplied to the wound dressing may alternatively or additionally, where appropriate supply materials to achieve the delivery of nucleic acid molecules as active genes or gene-containing vectors (DNA, RNA or modified versions thereof), as naked molecules, molecules complexed with nucleic acid binding carriers, molecules within liposomes or as virus vectors to give steady, measured delivery of gene therapeutic molecules to wound bed cells.

In the means for supplying physiologically active agents from cells or tissue to the wound, the irrigant from the reservoir that passes into and through the cell or tissue component often conveniently comprises cell culture medium species, e.g. trace elements and/or other nutrients such as amino acids, sugars, low molecular weight tissue building blocks, purines, pyrimidines, vitamins, metal ions or minerals, and/or gases, such as air, nitrogen, oxygen and/or nitric oxide, to aid proliferation of the cells or tissue in the means and/or steady, measured expression and supply of physiologically active agents.

In such case, materials that are listed above are also suitable therapeutic molecules to supply to wound bed cells to aid proliferation of the cells or tissue, and/or which are otherwise beneficial to wound healing.

In such case, it may be desirable to provide a system in which the irrigant from the reservoir that passes into and through the cell or tissue component comprises cell culture medium species and thereafter is supplied to the wound bed via a supply tube into the flowpath wherever appropriate, so that such cell culture medium species pass with the irrigant to the wound bed.

The irrigant from the reservoir may be used to maintain an optimum temperature of the cells or tissue and/or for regulating the exchange of gases in a conventional manner apparent to the skilled person. It is necessary for such a system to also irrigate the wound at a practical rate with the physiologically active components in therapeutically active amounts

Automated, programmable systems which can regulate the wound irrigant parameters and functions listed above in a precise and time-controlled manner are amongst those that are particularly suitable for use.

The tissue component may be an ex vivo (autologous, allogeneic or xenogenic) uncultured tissue explant.

Alternatively the tissue component may be formed from separated or partially separated cells which have either been used without a period of culture or they may have been cultured in vitro.

The process of culture may involve growth and proliferation or just incubation in culture.

The source tissues may be tissue from any organ such as skin, muscle, bone, neural, connective tissue, intestinal, liver or amniotic tissue and other organs or combinations thereof, whose cells and tissue retain the appropriate properties.

The cells or tissue may be fully viable or viable, but rendered non-dividing through irradiation or chemical treatment, or rendered non-viable after an appropriate period of culture.

Alternatively, the cells or tissue may be genetically modified to increase production of a particular material, e.g. a protein that is beneficial in promoting wound healing, such as a growth factor, an extracellular matrix component or fragments thereof, and other physiologically active components, or a biochemical, e.g. enzymatic or physical antagonists to elements detrimental to wound healing in the exudate and/or exudate and irrigant.

The tissue component that provides the active material that acts beneficially on the wound bed and/or cleanses the exudate and/or exudate and irrigant of materials detrimental to wound healing may consist of a co-culture.

A co-culture encompasses the in vitro or ex vivo culture of two or more cell types or tissue explants. This might be with one or both input cells or tissues fully viable or viable, but rendered non-dividing, through irradiation or chemical treatment, or rendered non-viable after an appropriate period of culture. Alternatively, the cells or tissue may be genetically modified to increase production of a particular material, e.g. a protein that is beneficial in promoting wound healing, such as a growth factor, an extracellular matrix component or fragments thereof, and other physiologically active components, or a biochemical, e.g. enzymatic or physical antagonists to elements detrimental to wound healing in the exudate and/or irrigant.

The input cells or tissues may be intimately mixed or intermingled, or they may be present as layers one on the other.

In some systems a semi permeable membrane or matrix between the component cells or tissues allows communication through biochemicals or proteins or other signals, but no cell apposition between the input cell types. In further systems modified irrigant is collected from one input cell or tissue type and given to the second input cell or type and given back to the first input cell type (sequentially or continuously) to generate the optimal output.

The cell or tissue component may be activated either singly or repeatedly through the delivery of biochemical, protein, enzymatic or physical means or through electromagnetic irradiation, ultrasonic or electrical stimulation.

The means for fluid cleansing is often in the form of a hollow body such as a container, e.g. a canister, cartridge or cassette, with a chamber or compartment, through which the wound exudate or a mixture of wound exudate and irrigant (or modified irrigant) is passed and recirculated by the device through the flow path. The structures noted above will often be made of glass, and/or synthetic polymeric materials. For example, such a structure may be a glass cylinder defining a chamber with axial inlet and outlet ports for throughflow.

The apparatus of the invention for aspirating, irrigating and/or cleansing wounds is provided with means for fluid cleansing, which may be
a) a single-phase system, such as an ultrafiltration unit, or a chemical absorption and/or adsorption unit; or
b) a two-phase system, such as a dialysis unit, or a biphasic extraction unit.

In the former, circulating fluid from the wound and the container for cells or tissue and the fluid reservoir passes through a self-contained system in which materials deleterious to wound healing are removed and the cleansed fluid, still containing materials that are beneficial in promoting wound healing (including such materials that have been added using cells or tissue) is returned via the recirculation tube to the wound bed. No other fluid phase is supplied or passes into such means for fluid cleansing.

In the two-phase system, the circulating fluid from the wound and the means for supplying physiologically active agents from cells or tissue to the wound passes through a system in which it is in indirect or (less usually, direct) contact with a second fluid (dialysate) phase.

Materials deleterious to wound healing are removed into the second phase, and the cleansed circulating fluid, still containing materials that are beneficial in promoting wound healing (including such materials that have been added using cells or tissue), is returned via the recirculation tube to the wound bed. Such systems are described in further detail hereinafter in connection with the means for fluid cleansing.

The means for fluid cleansing may as desired be a 'single-phase system'. The single-phase system may be of any conventional type.

Examples of the means for fluid cleansing in such a system include a macro- or microfiltration unit, which appropriately comprises one or more macroscopic and/or microscopic filters. These are to retain particulates, e.g. cell debris and micro-organisms, allowing proteins and nutrients to pass through.

Alternatively, they also include an ultrafiltration unit, such as a one in which the cleansing integer is a filter for materials deleterious to wound healing, for example a high throughput, low protein-binding polymer film, sheet or membrane which is selectively impermeable to materials deleterious to wound healing, which are removed, and the cleansed fluid, still containing materials that are beneficial in promoting wound healing, and adding such materials using cells or tissue is passed by it.

The membrane may preferably be of a hydrophilic polymeric material, such as a cellulose acetate - nitrate mixture, polyvinylidene chloride, and, for example hydrophilic polyurethane.

Examples of less preferred materials include hydrophobic materials also including polyesters, such as polycarbonates, PTFE, and polyamides, e.g. 6-6 and 6 - 10, and hydrophobic polyurethanes, and quartz and glass fibre. It has microapertures or micropores, the maximum cross-dimension of which will largely depend on the species that are to be selectively removed in this way and those to which it is to be permeable.

The former may be removed with microapertures or micropores, e.g. typically with a maximum cross-dimension in the range of 20 to 700 micron, e.g. 20 to 50 nm (for example for undesired proteins), 50 to 100 nm, 100 to 250 nm, 250 to 500 nm and 500 to 700 nm.

The filter integer may be a flat sheet or a membrane of a polymeric material in a more convoluted form, e.g. in the form of elongate structure, such as pipes, tubules, etc.

The system may be a chemical adsorption unit, for example one in which a particulate, such as a zeolite, or a layer, e.g. of a functionalised polymer has sites on its surface that are capable of removing materials deleterious to wound healing on passing the circulating fluid from the wound and the container for cells or tissue and the fluid reservoir over them.

The materials may be removed, e.g. by destroying or binding the materials that are deleterious to wound healing by, for example chelators and/or ion exchangers, degraders, which may be enzymes.

Examples of such also include less specific chemical adsorption units, for example one in which a physical absorbent, such as activated carbon or a zeolite, has non-specific sites on its surface that are capable of removing materials deleterious to wound healing on passing the circulating fluid from the wound and the container for cells or tissue and the fluid reservoir over them.

The cleansing integer, for example the polymer film, sheet or other chemical absorption and/or adsorption means, etc should of course be capable of removing materials deleterious to wound healing at a practical rate for a given capacity of the apparatus flow path and the flow rate of irrigant.

Alternatively, where appropriate the means for fluid cleansing may as desired be a 'two-phase systems', such as a dialysis unit, or a biphasic liquid extraction unit. Where the apparatus of the invention for aspirating, irrigating and/or cleansing is provided with means for fluid cleansing is a single-phase system, it may be of any conventional type.

In examples of the means for fluid cleansing that is a two-phase system, circulating fluid from the wound and the fluid reservoir is indirect or (less usually, direct) contact with a second fluid (dialysate) phase, usually a liquid.

Thus, in one form, a biphasic liquid extraction unit, the second fluid phase is (usually) a liquid that is immiscible with the circulating fluid from the dressing, over a surface of which the circulating fluid passes in direct contact with the cleansing fluid. Materials deleterious to wound healing are removed into the dialysate, and the cleansed fluid, still containing materials that are beneficial in promoting wound healing (including such materials that have been added using cells or tissue) is returned via the recirculation tube to the wound bed.

Examples of such means for fluid cleansing include those wherein the second fluid (dialysate) phase is perfluorodecalin and like materials

Alternatively and more usually, where appropriate it may be provided in a form in which the two fluids (recirculation fluid and dialysate) are separated by a significantly two-dimensional integer, for example a polymer film, sheet or membrane or hollow fibre or filament that is permeable to materials in the circulating fluid in the apparatus.

Again, materials deleterious to wound healing are removed into the dialysate, and the cleansed fluid, still containing materials that are beneficial in promoting wound healing (including such materials that have been added using cells or tissue) is returned via the recirculation tube to the wound bed.

In this form in which the two-phase system, such as a dialysis unit, is provided, typically in use the dialysate moves past the circulating fluid in the apparatus in a co- or preferably counter-current direction.

Pumps, such as peristaltic pumps, and/or valves control the direction of the two fluid flows.

However, the cleansing fluid may less usually be static, although this may not provide a system with sufficient (dynamic) surface area to remove materials deleterious to wound healing from wound exudate at a practical rate.

Typical dialysate flow rates in a dialytic means for fluid cleansing in the present apparatus for aspirating, irrigating and/or cleansing wounds are those used in the conventional type of two-phase system, such as a dialysis unit for systemic therapy.

The integer may be a film, sheet or membrane, often of the same type, and of the same (generally uniform) thickness, as those used in conventional two-phase system, such as a dialysis unit for systemic therapy.

The film, sheet or membrane may be substantially flat, and depending on any pressure differential across it may require other materials on or in it to stiffen, reinforce or otherwise strengthen it.

However, this may not provide a system with sufficient functional surface area to remove materials deleterious to wound healing from wound exudate at a practical rate.

To be suitable for use, in particular in chronic wound dialysis, with relatively high concentrations of materials that are deleterious to wound healing, it may be advantageous to provide a system in which the film, sheet or membrane of a polymeric material is in a more convoluted form.

This may be in the form of elongate structures, such as pipes, tubes hollow fibres or filaments or tubules of a round cross-section, e.g. elliptical or circular, e.g. in a parallel array with spaces therebetween.

The wound irrigant and/or wound exudate may recirculate through the inside and the cleansing fluid may pass into the spaces between adjacent pipes, tubes or tubules in a co- or preferably counter-current direction, or vice versa.

Again, materials deleterious to wound healing are removed into the dialysate, and the cleansed fluid, still containing materials from the wound that are beneficial in promoting wound healing (including added elements beneficial to wound healing to the exudate and irrigant or modified irrigant), is returned via the recirculation tube to the wound.

Examples of suitable materials for the film, sheet or membrane include natural and synthetic polymeric materials.

The membrane may be of one or more hydrophilic polymeric materials, such as a cellulose derivative, e.g. regenerated cellulose, a cellulose mono-, di- or tri- esters, such as cellulose mono-, di- or tri-acetate, benzyl cellulose and Hemophan, and mixtures thereof.

Examples of other materials include hydrophobic materials, such as aromatic polysulphones, polyethersulphones, polyetherether-sulphones, polyketones, polyetherketones and polyetherether-ketones, and sulphonated derivatives thereof, and mixtures thereof.

Examples of other materials include hydrophobic materials, such as polyesters, such as polycarbonates,
polyamides, e.g. 6-6 and 6 - 10;
polyacrylates, including, e.g. poly(methyl methacrylate),
polyacrylonitrile
and copolymers thereof, for example acrylonitrile - sodium metallosulphonate copolymers; and
poly(vinylidene chloride).

Suitable materials for the present membranes include thermoplastic polyolefins, such as polyethylene e.g. high-density polyethylene, polypropylene, copolymers thereof, for example with vinyl acetate and polyvinyl alcohol, and mixtures thereof.

The membrane should have a molecular weight cut off (MWCO) chosen to allow perfusion of species deleterious to wound healing that have been targeted for removal from the wound.

For example, perfusion of the serine protease elastase (molecular weight 25900 Dalton) would require a membrane with MWCO >25900 Dalton. The MWCO threshold can be varied to suit each application between 1 and 3000000 Dalton.

It may be desired to provide a system of therapy which can remove materials deleterious to wound healing from wound exudate, while
a) retaining the relevant antagonists, for example degrading enzymes, or sequestrating agents, on the dialysate side of the membrane,
b) supplying such materials if they are beneficial to wound healing into the exudate and irrigant (or modified irrigant), and/or
c) supplying into the exudate and irrigant (or modified irrigant) other materials that are beneficial to wound healing.

A particular advantage of option a) in the two-phase system, is where an antagonist that removes materials deleterious to wound healing from wound exudate is (cyto)toxic or bioincompatible, or not inert to any components that are beneficial in promoting wound healing.

The system does not allow any significant amounts of antagonist to diffuse freely out of the dialysate into the irrigant fluid. The active material can however act beneficially on the fluid.

As an example of option a), the antagonist to elastase, alpha-1-antitrypsin (AAT) (molecular weight 54000 Dalton) may occur in the dialysate component and removes elastase (which is deleterious to wound healing). A membrane with MWCO >25900 Dalton does not allow any significant amounts of the inhibitor, which is beneficial in promoting chronic wound healing, to diffuse freely out of the dialysate and it remains there.

As an example of option b), a less conventional type of two-phase system may be used as the means for fluid cleansing. In this type, the polymer film, sheet or membrane is not an integer selectively permeable to materials deleterious to wound healing.

It will also permit a broad spectrum of components of the exudate from a wound and/or irrigant fluid that may be larger or smaller molecules, but are beneficially involved in wound healing to pass freely to and fro through it. Some species will pass from the dialysate to the irrigant and/or wound exudate and back.

The target materials deleterious to wound healing pass into the dialysate from the exudate through the non-selectively permeable polymer film, sheet or membrane. Unlike the other components of the exudate from a wound and/or irrigant fluid, the target materials deleterious to wound healing come into contact with the dialysate and/or antagonists, binders and/or degraders, optionally on an integer with at least one surface in the dialysate, and are removed by the appropriate antagonists, binders and/or degraders.

Thus, unlike the other components of the exudate from a wound and/or irrigant fluid the target materials are constantly removed from the dialysate, and very little of these species will pass from the dialysate into the irrigant and/or wound exudate.

A steady state concentration equilibrium is not set up, even if the species are constantly 'topped up' from the wound dressing.

If (preferably) none of the dialysate is voided to waste, e.g. to a collection bag, a steady state concentration equilibrium of the untargeted species is eventually set up between the dialysate and the irrigant and/or wound exudate, which is topped up' from the wound dressing.

Circulating wound fluid aids in removal from recirculation of the materials deleterious to wound healing from wound exudate, and in the quicker attainment of this equilibrium of these materials.

The cleansed fluid, still containing materials from the wound that are beneficial in promoting wound healing (including elements beneficial to wound healing added to the exudate and irrigant or modified irrigant), is returned to the recirculation tube and to the where materials beneficial in promoting wound healing can be potentially of most benefit, i.e. the wound bed.

Specifically, a membrane with MWCO >54000 Dalton will allow significant amounts of elastase that is deleterious to chronic wound healing to diffuse freely into the dialysate and eventually to be removed by alpha-1-antitrypsin (AAT) (molecular weight 54000 Dalton) that may occur in the dialysate component. This inhibitor/antagonist to elastase (which is beneficial to wound healing) can diffuse freely into the exudate and eventually pass to the wound bed, where it can act beneficially on it.

As an example of option c), a membrane with a suitable MWCO will allow significant amounts of solutes or disperse phase species to pass from the dialysate into the irrigant and/or wound exudate through the polymer film, sheet or membrane. This property may be used to perfuse materials beneficial to wound healing into the irrigant and/or exudate from a dialysate. In this less conventional type of infusion feed, a broad spectrum of species will usually pass into the exudate and/or irrigant fluid from the dialysate.

These include materials that are beneficial to wound healing.

Such materials include cytokines, enzymes, growth factors, and others having beneficial effects in causing chemotaxis.

These also include materials that are added elements beneficial to wound healing, such as
ionic species, such as bicarbonate;
vitamins, such as ascorbic acid (vitamin C) and vitamin E, and stable derivatives thereof, and mixtures thereof; to relieve oxidative stress on the wound bed;
pH buffering agents, such as potassium dihydrogen phosphate/ disodium hydrogen phosphate,
local analgesics/anaesthetics, such as lidocaine/lignocaine hydrochloride and xylocaine (adrenoline lidocaine) and/or anti-inflammatories, to reduce wound pain or inflammation or pain associated with the dressing
nutrients to aid proliferation of wound cells, such as amino acids, sugars, low molecular weight tissue building blocks and trace elements; and other cell culture medium species; and
gases, such as air, nitrogen, oxygen and/or nitric oxide.

All such use of the present apparatus is, e.g. favourable to the wound healing process in chronic wounds, such as diabetic foot ulcers, and especially decubitus pressure ulcers.

Where it is desired to remove several different materials that are deleterious to wound healing, it may be advantageous to provide a system of modules in series, each of which removes a different material. This allows incompatible materials to be used on the same fluid and/or wound exudates.

Both the single-phase system and two-phase system may be in modular form that is relatively easily demountable from the apparatus of the invention. The system may suitably comprise one or more such modules.

Preferably any such system is a conventional automated, programmable system which can cleanse the wound irrigant and/or wound exudate with minimal supervision.

The means for fluid cleansing using cells or tissue may additionally, where appropriate, comprise one or more macroscopic and/or microscopic filters. These are to retain particulates, e.g. cell debris and micro-organisms, allowing proteins and nutrients to pass through.

The conduits through which respectively
a) the irrigant and/or wound exudate passes from the wound dressing and
b) the cleansed fluid,
   still containing materials from the wound that are beneficial in promoting wound healing,
   with added elements beneficial to wound healing to the exudate and irrigant (or modified irrigant), and/or
   modified through biochemical, enzymatic or physical means to contain elements beneficial to wound healing,
   is returned to the recirculation tube, and
c) (in the case where the means is provided in the form of a two-phase system, such as an dialysis unit) through which the cleansing using cells or tissue fluid enters and exits the means
preferably have means for, on module disconnection and withdrawal,
i) switching off the flow and
ii) providing an immediate fluid-tight seal or closure over the ends of the conduits and the cooperating tubes in the rest of the apparatus of the invention so exposed,
to prevent continuing passage of irrigant and/or exudate and cleansed fluid, and cleansing using cells or tissue fluid.

The means for flow switching between supply and recirculation may take any form that enables the wound simultaneously to be
a) put into communication with the fluid reservoir but
b) closed to the fluid recirculation tube, and
c) vice versa.

Thus, If there is only one inlet pipe that passes through and/or under the wound-facing face of the wound dressing, the means for supplying physiologically active agents from cells or tissue to the wound is often connected to the flow path via means for flow switching as desired between a fluid recirculation tube or a fluid supply tube.

In this case, the means for flow switching between supply and recirculation may be a regulator, such as a T- valve.

This is connected in turn to two parts of a fluid recirculation tube or a fluid offtake tube and the fluid supply tube, such that the desired flow switching between supply and recirculation is achieved.

The means for supplying physiologically active agents from cells or tissue to the wound often comprises
a) an irrigant reservoir connected to
b) a container that contains a cell or tissue component, through which the irrigant is passed to form modified irrigant, in turn connected to a supply tube.

If there are two or more inlet pipes, these may each be connected respectively to
a) a fluid supply tube, in turn connected to a means for supplying physiologically active agents from cells or tissue to the wound, and
b) a fluid recirculation tube,
respectively having a first regulator and a second regulator, such as a valve or other control device for admitting fluids into the wound.

The desired flow switching between supply and recirculation is achieved by respectively having the first regulator open when the second regulator is shut, and vice versa.

Again, the means often comprises
i) an irrigant reservoir connected to
ii) a container that contains a cell or tissue component, through which the irrigant is passed to form modified irrigant

The means for bleeding the flowpath may be situated in any appropriate part of the apparatus that is in contact with the irrigant and/or wound exudate, but is usually within the offtake and/or recirculation tubes. However, it is often as far downstream of and away from the reservoir and the fluid supply tube as possible, so that it may be used to prime the whole of the flowpath from the fluid reservoir via the fluid supply tube.

It may be a regulator, such as a valve or other control device, e.g. a T-valve that is turned to switch between bleed and recirculation, for bleeding fluids from the apparatus, e.g. to a waste reservoir, such as a collection bag.

Alternatively, flow switching between supply and recirculation may not be desired, but rather concomitant bleeding and/or recirculation is desired.

The latter may occur when the volume of irrigant and/or wound exudate in recirculation is increased by continuing addition to it of
a) wound exudate, and/or
b) fluid passing from a cleansing fluid through a selectively permeable integer, for example in a system such as a dialysis unit.

The means for bleeding the offtake and/or recirculation tubes may then be provided in the form of a regulator, such as a simple valve or other control device for admitting or blocking the passage of irrigant and/or exudate through a bleed line branching from the recirculation path.

In use, typically, the means for flow switching between supply and recirculation tubes is set to admit fluid into the wound from the fluid reservoir but to close the wound to the fluid recirculation tube.

Then, any means for bleeding the offtake and/or recirculation tubes are/is opened and the device for moving fluid through the wound and means for fluid cleansing is started.

The capacity of the apparatus flow path and the flow rate of irrigant and/or wound exudate from the wound will largely determine whether it is appropriate to run the device to prime the apparatus throughout the whole length of the apparatus flow path, i.e. to displace any existing fluid reservoir (often air) from the fluid recirculation path, and for how long it should be run.

Typically, there is a preponderance of irrigant from the fluid reservoir over wound exudate in recirculation, so that use of the device for moving fluid through the wound is appropriate for this purpose.

It is allowed to run until the apparatus is primed throughout the whole length of the apparatus flow path.

Then, typically the means for bleeding the offtake and/or recirculation tubes is closed, and the means for flow switching between supply and recirculation tubes is set to close the wound to the fluid reservoir but to admit fluid into the wound from the fluid recirculation tube.

If the means for fluid cleansing is a two-phase system, such as a dialysis unit, or a biphasic extraction unit, the cleansing fluid is typically set in motion in contact with the surface of the selectively permeable integer, for example the polymer film, sheet or membrane. Of course, the cleansing fluid may less usually be static, and then this step is omitted.

As noted below in more detail, the volume of irrigant and/or wound exudate from the wound in recirculation may be increased by continuing addition to it of
a) wound exudate, and/or
b) fluid passing from a cleansing fluid through a selectively permeable integer, for example the polymer film, sheet or membrane of a two-phase system, such as an dialysis unit.

Additionally or alternatively, it may be desired to apply a negative pressure to the wound by means of a device for moving fluid through the wound and means for fluid cleansing applied to the fluid in recirculation in the fluid recirculation tube downstream of and away from the wound dressing.

In such case, it may be desirable to provide a system in which concomitant bleeding and/or recirculation is possible, and to make the necessary adjustments to maintain the desired balance of fluid in recirculation by means of the means for bleeding the offtake and/or recirculation tubes.

The volume of irrigant and/or wound exudate from the wound in recirculation may be decreased by continuing loss from it of fluid passing from a cleansing fluid through a selectively permeable integer, for example in a system such as a dialysis unit.

Additionally or alternatively, it may be desired to apply a positive pressure to the wound by means of a device for moving fluid through the wound and means for fluid cleansing applied to the fluid in recirculation in the fluid recirculation tube upstream of and towards the wound dressing.

The means for flow switching between supply and recirculation may be similarly provided in a form in which concomitant supply and/or recirculation is possible, and to make the necessary adjustments to maintain the desired balance of fluid in recirculation by means of the means for flow switching.

It will be appreciated that where a positive or negative pressure is to be applied to the wound, at least one hollow body in the recirculation flow path to and from the wound bed should have sufficient resilience against the pressure to allow any significant compression or decompression of the irrigant fluid to occur.

In all embodiments of the apparatus, the type and material of such bodies (which are defined by a film, sheet or membrane) that are described by way of example herein to be suitable for use in the present invention will be largely capable of this function.

Thus, examples of suitable materials for bodies defined by a film, sheet or membrane, such as inlet or offtake and/or recirculation tubes and structures such as bags, chambers and pouches, filled with irrigant fluid, e.g. the backing layer of the wound dressing are suitably elastically resilient thermoplastic materials that are potentially capable of this function when pressure is applied in this way.

The present invention in this aspect provides several advantages.

One is that application of a positive pressure to the wound under the backing layer may make it possible to flood the tissue underlying the wound with one or more physiologically active components.

This may be effected in therapeutically active amounts, to promote greater wound healing than by treatment with the fluid physiologically active component(s) alone.

Such physiologically active components of the exudate that are beneficial to wound healing (including such materials that have been added using cells or tissue) may be e.g. enzymes or other species.

It is believed that using the apparatus for aspirating, irrigating and/or cleansing wounds of the present invention cyclically the effects may be further enhanced.

Circulating wound fluid aids in movement of biological signalling molecules involved in wound healing (including such materials that have been added using cells or tissue) to locations in the wound bed that are favourable to the wound healing and/or to cells that would otherwise not be exposed to them, e.g. in a highly exuding wound.

This is especially the case in those embodiments of the apparatus of this first aspect of the present invention for aspirating, irrigating and/or cleansing wounds where there is an inlet or outlet manifold from which tubules radiate and run to the wound bed to end in openings that deliver and collect the fluid directly from the wound bed over an extended area.

Such materials include cytokines, enzymes, nutrients for wound cells to aid proliferation, oxygen, and other molecules that are beneficially involved in wound healing (including such materials that have been added using cells or tissue), such as growth factors, and others having beneficial effects (which may be further enhanced) in causing chemotaxis.

In all embodiments of the apparatus of this first aspect of the present invention for aspirating, irrigating and/or cleansing wounds, a particular advantage is the tendency of the wound dressing to conform to the shape of the bodily part to which it is applied.

The wound dressing comprises
a backing layer with a wound-facing face which is capable of forming a relatively fluid-tight seal or closure over a wound and
at least one inlet pipe for connection to a fluid supply tube or recirculation tube, which passes through and/or under the wound-facing face, and
and at least one outlet pipe for connection to a fluid offtake tube, which passes through and/or under the wound-facing face,
the point at which the or each inlet pipe and the or each outlet pipe passes through and/or under the wound-facing face forming a relatively fluid-tight seal or closure.

The term 'relatively fluid-tight seal or closure' is used herein to indicate one which is fluid- and microbe-impermeable and permits a positive or negative pressure of up to 50% of up to 101,325 Pascals (atm.), more usually up to 15% of 101,325 Pascals (atm.) to be applied to the wound. The term 'fluid' is used herein to include gels, e.g. thick exudate, liquids, e.g. water, and gases, such as air, nitrogen, etc.

The shape of the backing layer that is applied may be any that is appropriate to aspirating, irrigating and/or cleansing the wound across the area of the wound.

Examples of such include a substantially flat film, sheet or membrane, or a bag, chamber, pouch or other structure of the backing layer, e.g. of polymer film, which can contain the fluid.

The backing layer may be a film, sheet or membrane, often with a (generally uniform) thickness of up to 100 micron, preferably up to 50 micron, more preferably up to 25 micron, and of 10 micron minimum thickness.

Its largest cross-dimension may be up to 500 mm (for example for large torso wounds), up to 100 mm (for example for axillary and inguinal wounds), and up to 200 mm for limb wounds (for example for chronic wounds, such as venous leg ulcers and diabetic foot ulcers.

Desirably the dressing is resiliently deformable, since this may result in increased patient comfort, and lessen the risk of inflammation of a wound. Suitable materials for it include synthetic polymeric materials that do not absorb aqueous fluids, such as polyolefins, such as polyethylene e.g. high-density polyethylene, polypropylene, copolymers thereof, for example with vinyl acetate and polyvinyl alcohol, and mixtures thereof; polysiloxanes; polyesters, such as polycarbonates; polyamides, e.g. 6-6 and 6 - 10, and hydrophobic polyurethanes.

They may be hydrophilic, and thus also include hydrophilic polyurethanes.

They also include thermoplastic elastomers and elastomer blends, for example copolymers, such as ethyl vinyl acetate, optionally or as necessary blended with high-impact polystyrene.

They further include elastomeric polyurethane, particularly polyurethane formed by solution casting.

Preferred materials for the present wound dressing include thermoplastic elastomers and curable systems.

The backing layer is capable of forming a relatively fluid-tight seal or closure over the wound and/or around the inlet and outlet pipe(s).

However, in particular around the periphery of the wound dressing, outside the relatively fluid-tight seal, it is preferably of a material that has a high moisture vapour permeability, to prevent maceration of the skin around the wound. It may also be a switchable material that has a higher moisture vapour permeability when in contact with liquids, e.g. water, blood or wound exudate. This may, e.g. be a material that is used in Smith & Nephew's Allevyn™, IV3000™ and OpSite™ dressings.

The periphery of the wound-facing face of the backing layer may bear an adhesive film, for example, to attach it to the skin around the wound.

This may, e.g. be a pressure-sensitive adhesive, if that is sufficient to hold the wound dressing in place in a fluid-tight seal around the periphery of the wound-facing face of the wound dressing.

Alternatively or additionally, where appropriate a light switchable adhesive could be used to secure the dressing in place to prevent leakage. (A light switchable adhesive is one the adhesion of which is reduced by photocuring. Its use can be beneficial in reducing the trauma of removal of the dressing.)

Thus, the backing layer may have a flanges or lip extending around the proximal face of the backing layer, of a transparent or translucent material (for which it will be understood that materials that are listed above are amongst those that are suitable).

This bears a film of a light switchable adhesive to secure the dressing in place to prevent leakage on its proximal face, and a layer of opaque material on its distal face.

To remove the dressing and not cause excessive trauma in removal of the dressing, the layer of opaque material on the distal face of the flange or lip extending around the proximal wound is removed prior to application of radiation of an appropriate wavelength to the flange or lip.

If the periphery of the wound dressing, outside the relatively fluid-tight seal, that bears an adhesive film to attach it to the skin around the wound, is of a material that has a high moisture vapour permeability or is a switchable material, then the adhesive film, if continuous, should also have a high or switchable moisture vapour permeability, e.g. be an adhesive such as used in Smith & Nephew's Allevyn™, IV3000™ and OpSite™ dressings.

Where a vacuum, is applied to hold the wound dressing in place in a fluid-tight seal around the periphery of the wound-facing face of the wound dressing, the wound dressing may be provided with a silicone flange or lip to seal the dressing around the wound. This removes the need for adhesives and associated trauma to the patient's skin.

Where the interior of, and the flow of irrigant and/or wound exudate to and through, the dressing is under any significant positive pressure, which will tend to act at peripheral points to lift and remove the dressing off the skin around the wound.

In such use of the apparatus, it may thus be necessary to provide means for forming and maintaining such a seal or closure over the wound against such positive pressure on the wound, to act at peripheral points for this purpose.

Examples of such means include light switchable adhesives, as above, to secure the dressing in place to prevent leakage.

Since the adhesion of a light switchable adhesive is reduced by photocuring, thereby reducing the trauma of removal of the dressing, a film of a more aggressive adhesive may be used, e.g. on a flange, as above.

Examples of suitable fluid adhesives for use in more extreme conditions where trauma to the patient's skin is tolerable include ones that consist essentially of cyanoacrylate and like tissue adhesives, applied around the edges of the wound and/or the proximal face of the backing layer of the wound dressing, e.g. on a flange or lip.

Further suitable examples of such means include adhesive (e.g. with pressure-sensitive adhesive) and non-adhesive, and elastic and non-elastic straps, bands, loops, strips, ties, bandages, e.g. compression bandages, sheets, covers, sleeves, jackets, sheathes, wraps, stockings and hose, e.g. elastic tubular hose or elastic tubular stockings that are a compressive fit over a limb wound to apply suitable pressure to it when the therapy is applied in this way; and inflatable cuffs, sleeves, jackets, trousers, sheathes, wraps, stockings and hose that are a compressive fit over a limb wound to apply suitable pressure to it when the therapy is applied in this way.

Such means may each be laid out over the wound dressing to extend beyond the periphery of the backing layer of the wound dressing.

It will, as appropriate, adhered or otherwise secured to the skin around the wound and/or itself and as appropriate will apply compression (e.g. with elastic bandages, stockings) to a degree that is sufficient to hold the wound dressing in place in a fluid-tight seal around the periphery of the wound,

Such means may each be integral with the other components of the dressing, in particular the backing layer.

Alternatively, it may be permanently attached or releasably attached to the dressing, in particular the backing layer, with an adhesive film, for example, or these components may be a Velcro ™, push snap or twist-lock fit with each other.

The means and the dressing may be separate structures, permanently unattached to each other.

In a more suitable layout for higher positive pressures on the wound, a stiff flange or lip extends around the periphery of the proximal face of the backing layer of the wound dressing as hereinbefore defined.

The flange or lip is concave on its proximal face to define a peripheral channel or conduit.

It has a suction outlet that passes through the flange or lip to communicate with the channel or conduit and may be connected to a device for applying a vacuum, such as a pump or a piped supply of vacuum.

The backing layer may be integral with or attached, for example by heat-sealing, to the flange or lip extending around its proximal face.

To form the relatively fluid-tight seal or closure over a wound that is needed and to prevent passage of irrigant and/or exudate under the periphery of the wound-facing face of the wound dressing, in use of the apparatus, the dressing is set on the skin around the wound.

The device then applies a vacuum to the interior of the flange or lip, thus forming and maintaining a seal or closure acting at peripheral points around the wound against the positive pressure on the wound.

With all the foregoing means of attachment, and means for forming and maintaining a seal or closure over the wound, against positive or negative pressure on the wound at peripheral points around the wound, the wound dressing sealing periphery is preferably of a generally round shape, such as an ellipse, and in particular circular.

To form the relatively fluid-tight seal or closure over a wound and around the inlet pipe(s) and outlet pipe(s) at the point at which they pass through and/or under the wound-facing face, the backing layer may be integral with these other components.

The components may alternatively just be a push, snap or twist-lock fit with each other, or adhered or heat-sealed together.

The or each inlet pipe or outlet pipe may be in the form of an aperture, such as a funnel, hole, opening, orifice, luer, slot or port for connection as a female member respectively to a mating end of
a fluid recirculation tube and/or fluid supply tube (optionally or as necessary via means for forming a tube, pipe or hose, or nozzle, hole, opening, orifice, luer, slot or port for connection as a male member respectively to a mating end of
a fluid recirculation tube and/or fluid supply tube (optionally or as necessary via means for flow switching between supply and recirculation) or
a fluid offtake tube.

Where the components are integral they will usually be made of the same material (for which it will be understood that materials that are listed above are amongst those that are suitable).

Where, alternatively, they are a push, snap or twist-lock fit, the may be of the same material or of different materials. In either case, materials that are listed above are amongst those that are suitable for all the components. The or each pipe will generally pass through, rather than under the backing layer.

In such case, the backing layer may often have a rigid and/or resiliently inflexible or stiff area to resist any substantial play between the or each pipe and the or each mating tube, or deformation under pressure in any direction.

It may often be stiffened, reinforced or otherwise strengthened by a boss projecting distally (outwardly from the wound) around each relevant tube, pipe or hose, or nozzle, hole, opening, orifice, luer, slot or port for connection to a mating end of a fluid recirculation tube and/or fluid supply tube or fluid offtake tube.

Alternatively or additionally, where appropriate the backing layer may have a stiff flange or lip extending around the proximal face of the backing layer to stiffen, reinforce or otherwise strengthen the backing layer.

The wound dressing may not comprise any integer under the backing layer in the wound in use.

However, this may not provide a system to distribute irrigant over a sufficient functional surface area to irrigate the wound at a practical rate. To be suitable for use, in particular in chronic wound dialysis, with relatively high concentrations of materials that are deleterious to wound healing, it may be advantageous to provide a system where wound irrigant and/or wound exudate may be distributed more evenly, or pass in a more convoluted path under the dressing over the wound bed.

Accordingly, one form of the dressing is provided with a 'tree' form of pipes, tubes or tubules that radiate from an inlet manifold to the wound bed to end in apertures and deliver the circulating fluid directly to the wound bed via the apertures. Similarly, there is an outlet manifold from which tubules radiate and run to the wound bed to end in openings and collect the fluid directly from the wound bed.

The pipes, etc. may radiate regularly or irregularly through the wound in use, respectively from the inlet or outlet manifold, although regularly may be preferred.

A more suitable layout for deeper wounds is one in which the pipes, etc. radiate hemispherically and concentrically, to the wound bed.

For shallower wounds, examples of suitable forms of such layout of the pipes, etc. include ones in which the pipes, etc. radiate in a flattened hemiellipsoid and concentrically, to the wound bed.

Other suitable forms of layout of the pipes, etc. include one which have pipes, tubes or tubules extending from the inlet pipe(s) and/or outlet pipe(s) at the point at which they pass through and/or under the wound-facing face of the backing layer to run over the wound bed. These may have a blind bore with perforations, apertures, holes, openings, orifices, slits or slots along the pipes, etc.

These pipes, etc. then effectively form an inlet pipe manifold that delivers the circulating fluid directly to the wound bed or outlet pipe or collects the fluid directly from the wound respectively.

It does so via the holes, openings, orifices, slits or slots in the tubes, pipes, tubules, etc. over most of the wound bed under the backing layer.

It may be desirable that the tubes, pipes or tubules are resiliently flexible, e.g. elastomeric, and preferably soft, structures with good conformability in the wound and the interior of the wound dressing.

When the therapy is applied in this way, the layout of the tubes, pipes, tubules, etc. may depend on the depth and/or capacity of the wound.

Thus, for shallower wounds, examples of suitable forms of such layout of the tubes, pipes, tubules, etc. include ones that consist essentially of one or more of the tubes, etc in a spiral.

A more suitable layout for deeper wounds when the therapy is applied in this way may be one which comprises one or more of the tubes, etc in a helix or spiral helix.

Other suitable layouts for shallower wounds include one which have blind-bore, perforated inlet pipe or outlet pipe manifolds that circulate fluid in the wound when the dressing is in use.

One or both of these may be such a form, the other may be, e.g. one or more straight blind-bore, perforated radial tubes, pipes or nozzles.

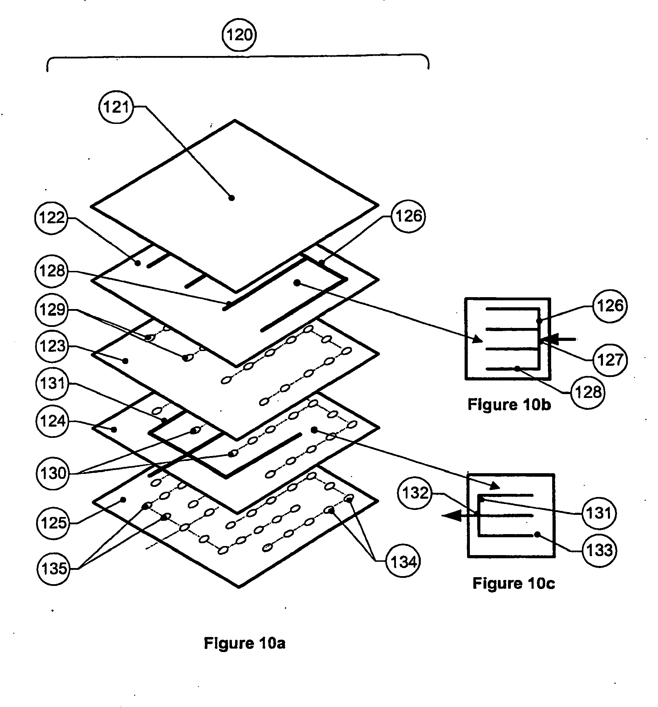

Another suitable layout is one in which
an inlet pipe and/or outlet pipe manifold that delivers the circulating fluid directly to the wound bed or collects the fluid directly from the wound respectively
via inlet and/or outlet tubes, pipes or tubules,
and the inlet manifold and/or outlet manifold is formed by slots in layers permanently attached to each other in a stack, and
the inlet and/or outlet tubes, pipes or tubules are formed by apertures through layers permanently attached to each other in a stack. (In Figure 10a there is shown an exploded isometric view of such a stack, which is non-limiting.)

As also mentioned herein, the backing layer that is applied may be any that is appropriate to the present system of therapy and permits a positive or negative pressure of up to 50% of 101,325 Pascals (atm.), more usually up to 25% of 101,325 Pascals (atm.) to be applied to the wound.

It is thus often a microbe-impermeable film, sheet or membrane, which is substantially flat, depending on any pressure differential on it, and often with a (generally uniform) thickness similar to such films or sheets used in conventional wound dressings, i.e. up to 100 micron, preferably up to 50 micron, more preferably up to 25 micron, and of 10 micron minimum thickness.

The backing layer may often have a rigid and/or resiliently inflexible or stiff area to resist any substantial play between other components that are not mutually integral, and may be stiffened, reinforced or otherwise strengthened, e.g. by a projecting boss.

Such a form of dressing would not be very conformable to the wound bed, and may effectively form a chamber, hollow or cavity defined by a backing layer and the wound bed under the backing layer.

It may be desirable that the interior of the wound dressing conform to the wound bed, even for a wound in a highly exuding state. Accordingly, one form of the dressing is provided with a wound filler under the backing layer.

This is favourably a resiliently flexible, e.g. elastomeric, and preferably soft, structure with good conformability to wound shape.

It is urged by its own resilience against the backing layer to apply gentle pressure on the wound bed.

The wound filler may be integral with the other components of the dressing, in particular the backing layer.

Alternatively, it may be permanently attached to them/it, with an adhesive film, for example, or by heat-sealing, e.g. to a flange or lip extending from the proximal face, so a not to disrupt the relatively fluid-tight seal or closure over the wound that is needed.

Less usually, the wound filler is releasably attached to the backing layer, with an adhesive film, for example, or these components may be a push, snap or twist-lock fit with each other.

The wound filler and the backing layer may be separate structures, permanently unattached to each other.

The wound filler may be or comprise a solid integer, favourably a resiliently flexible, e.g. elastomeric, and preferably soft, structure with good conformability to wound shape.

Examples of suitable forms of such wound fillers are foams formed of a suitable material, e.g. a resilient thermoplastic.

Preferred materials for the present wound dressing include reticulated filtration polyurethane foams with small apertures or pores.

Alternatively or additionally, it may be in the form of, or comprise one or more conformable hollow bodies defined by a film, sheet or membrane, such as a bag, chamber, pouch or other structure, filled with a fluid or solid that urges it to the wound shape.

The film, sheet or membrane, often has a (generally uniform) thickness similar to that of films or sheets used in conventional wound dressing backing layers.

That is, up to 100 micron, preferably up to 50 micron, more preferably up to 25 micron, and of 10 micron minimum thickness, and is often resiliently flexible, e.g. elastomeric, and preferably soft.

Such a filler is often integral with the other components of the dressing, in particular the backing layer, or permanently attached to them/it, with an adhesive film, for example, or by heat-sealing, e.g. to a flange

Examples of suitable fluids contained in the hollow body or bodies defined by a film, sheet or membrane include gases, such as air, nitrogen and argon, more usually air, at a small positive pressure above atmospheric; and liquids, such as water, saline.

Examples also include gels, such as silicone gels, e.g. CaviCare™ gel, or preferably cellulosic gels, for example hydrophilic cross-linked cellulosic gels, such as Intrasite ™ cross-linked materials.

Examples also include aerosol foams, where the gaseous phase of the aerosol system is air or an inert gas, such as nitrogen or argon, more usually air, at a small positive pressure above atmospheric; and solid particulates, such as plastics crumbs.

Of course, if the backing layer is a sufficiently conformable and/or e.g. an upwardly dished sheet, the backing layer may lie under the wound filler, rather than vice versa.

In this type of layout, in order for the wound filler to urge the wound dressing towards the wound bed, it will usually have to be firmly adhered or otherwise releasably attached to the skin around the wound. This is especially the case in those embodiments where the wound filler and the backing layer are separate structures, permanently unattached to each other.

In such a layout for deeper wounds when the therapy is applied in this way, the means for such attachment may also form and maintain a seal or closure over the wound.

Where the filler is over the backing layer, and the fluid inlet pipe(s) and outlet pipe(s) pass through the wound-facing face of the backing layer, they may run through or around the wound filler over the backing layer.

One form of the dressing is provided with a wound filler under the backing layer that is or comprises a resiliently flexible, e.g. elastomeric, and preferably soft, hollow body defined by a film, sheet or membrane, such as a bag, chamber, pouch or other structure, with apertures, holes, openings, orifices, slits or slots, or tubes, pipes, tubules or nozzles. It communicates with at least one inlet or outlet pipe through at least one aperture, hole, opening, orifice, slit or slot.

The fluid contained in the hollow body may then be the circulating fluid in the apparatus.

The hollow body or each of the hollow bodies then effectively forms an inlet pipe or outlet pipe manifold that delivers the circulating fluid directly to the wound bed or collects the fluid directly from the wound respectively via the holes, openings, orifices, slits or slots, or the tubes, pipes or hoses, etc. in the film, sheet or membrane.

When the therapy is applied in this way, the type of the filler may also be largely determined by the depth and/or capacity of the wound.

Thus, for shallower wounds, examples of suitable wound fillers as a component of a wound dressing include ones that consist essentially of one or more conformable hollow bodies defining an inlet pipe and/or outlet pipe manifold that delivers the circulating fluid directly to the wound bed or collects the fluid directly from the wound.

A more suitable wound filler for deeper wounds when the therapy is applied in this way may be one which comprises one or more conformable hollow bodies defined by, for example a polymer film, sheet or membrane, that at least partly surround(s) a solid integer. This may provide a system with better rigidity for convenient handling.

Unless the wound filler under the backing layer effectively forms an inlet pipe or outlet pipe manifold with a direct connection between the inlet pipe(s) and outlet pipe(s) at the point at which they pass through and/or under the wound-facing face and the wound bed is present, in order for aspiration and/or irrigation of the wound bed to occur, it is appropriate for one or more bores, channels, conduits, passages, pipes, tubes, tubules and/or spaces, etc. to run from the point at which the fluid inlet pipe(s) and outlet pipe(s) pass through and/or under the wound-facing face of the backing layer through or around the wound filler under the backing layer.

Less usually, the wound filler is an open-cell foam with pores that may form such bores, channels, conduits, passages and/or spaces through the wound filler under the backing layer.

Where the filler is or comprises one or more conformable hollow bodies defined by, for example a polymer film, sheet or membrane, it may be provided with means for admitting fluids to the wound bed under the wound dressing.

These may be in the form of pipes, tubes, tubules or nozzles running from the point at which the fluid inlet pipe(s) and outlet pipe(s) pass through and/or under the wound-facing face of the backing layer through or around the wound filler under the backing layer.

All of the suitable layouts for shallower wounds that comprise blind-bore, perforated inlet pipe or outlet pipe manifolds that circulate fluid in the wound when the dressing is in use, that are described hereinbefore, may be used under a wound filler under the backing layer.

In brief, suitable layouts include ones where one or both manifolds are annular or toroidal (regular, e.g. elliptical or circular, or irregular), optionally with blind-bore, perforated radial tubes, pipes or nozzles, branching from the annulus or torus; and/or
in a meandering, tortuous, winding, zigzag, serpentine or boustrophedic (i.e. in the manner of a ploughed furrow) pattern, or
defined by slots in and apertures through layers attached to each other in a stack.

The inlet and/or outlet tubes, the fluid recirculation tube and the fluid supply tube, etc. may be of conventional type, e.g. of elliptical or circular cross-section, and may suitably have a uniform cylindrical bore, channel, conduit or passage throughout their length.

Depending on the desired fluid volume flow rate of irrigant and/or wound exudate from the wound, and the desired amount in recirculation, suitably the largest cross-dimension of the bore may be up to 10 mm for large torso wounds, and up to 2 mm for limb wounds.

The tube walls should be suitably thick enough to withstand any positive or negative pressure on them, in particular if the volume of irrigant and/or wound exudate from the wound in recirculation is increased by continuing addition to it of wound exudate, and/or fluid passing from a cleansing fluid through a selectively permeable integer, for example in a dialysis unit.

However, as noted below with regard to pumps, the prime purpose of such tubes is to convey fluid irrigant and exudate through the length of the apparatus flow path, rather than to act as pressure vessels. The tube walls may suitably be at least 25 micron thick.

The bore or any perforations, apertures, holes, openings, orifices, slits or slots along the pipes, etc. or in the hollow body or each of the hollow bodies may be of small cross-dimension.

They may then effectively form a macroscopic and/or microscopic filter for particulates including cell debris and micro-organisms, whilst allowing proteins and nutrients to pass through.

Such tubes, pipes or hoses, etc. through and/or around the filler, whether the latter is a solid integer and/or one or more resiliently flexible or conformable hollow bodies, are described in further detail hereinbefore in connection with the inlet pipe(s) and outlet pipe(s).

The whole length of the apparatus for aspirating, irrigating and/or cleansing wounds should be microbe-impermeable once the wound dressing is over the wound in use.

It is desirable that the wound dressing and the interior of the apparatus for aspirating, irrigating and/or cleansing wounds of the present invention is sterile.

The fluid may be sterilised in the fluid reservoir and/or the rest of the system in which the fluid recirculates, including the means for fluid cleansing, by ultraviolet, gamma or electron beam irradiation.

(Excepted from this is the integer that contains the tissue or cell component, since this may adversely affect the viability and activity of the cells).

This way, in particular reduces or eliminates contact of internal surfaces and the fluid with any sterilising agent.

Examples of other methods of sterilisation of the fluid also include e.g. the use of
ultrafiltration through microapertures or micropores, e.g. of 0.22 to 0.45 micron maximum cross-dimension, to be selectively impermeable to microbes; and
fluid antiseptics, such as solutions of chemicals, such as chlorhexidine and povidone iodine; metal ion sources, such as silver salts, e.g. silver nitrate; and hydrogen peroxide;
although the latter involve contact of internal surfaces and the fluid with the sterilising agent.

It may be desirable that the interior of the wound dressing, the rest of the system in which the fluid recirculates, and/or the wound bed, even for a wound in a highly exuding state, are kept sterile after the fluid is sterilised in the fluid reservoir, or that at least naturally occurring microbial growth is inhibited.

Thus, materials that are potentially or actually beneficial in this respect may be added to the irrigant initially, and as desired the amount in recirculation increased by continuing addition.

Examples of such materials include antibacterial agents (some of which are listed above), and antifungal agents.

Amongst those that are suitable are, for example triclosan, iodine, metronidazole, cetrimide, chlorhexidine acetate, sodium undecylenate, chlorhexidine and iodine.

Buffering agents, such as potassium dihydrogen phosphate/ disodium hydrogen phosphate may be added to adjust the pH, as may local analgesics/anaesthetics, such as lidocaine/lignocaine hydrochloride, xylocaine (adrenoline, lidocaine) and/or anti-inflammatories, to reduce wound pain or inflammation or pain associated with the dressing.

It is also desirable to provide a system in which physiologically active components of the exudate that are beneficial to wound healing are not removed before or after the application of fluid cleansing, e.g. by the passive deposition of materials that are beneficial in promoting wound healing, such as proteins, e.g. growth factors.

This may occur at any point at least one inlet or outlet pipe through at least one aperture, hole, opening, orifice, slit or slot.

A material to combat the deposition of materials that are beneficial in promoting wound healing
a) may be added to the irrigant initially, and as desired the amount in recirculation increased by continuing addition, or
a) may be used at any point or on any integer in the recirculation path in direct contact with the fluid, e.g. on the means for fluid cleansing or any desired tube or pipe.

Examples of coating materials for surfaces over which the circulating fluid passes include
anticoagulants, such as heparin, and
high surface tension materials, such as PTFE, and polyamides,
which are useful for growth factors, enzymes and other proteins and derivatives.

The apparatus of the invention for aspirating, irrigating and/or cleansing wounds is provided with means for admitting fluids directly or indirectly to the wound under the wound dressing in the form of a fluid supply tube to a fluid reservoir.

The fluid reservoir may be of any conventional type, e.g. a tube, bag (such as a bag typically used for blood or blood products, e.g. plasma, or for infusion feeds, e.g. of nutrients), chamber, pouch or other structure, e.g. of polymer film, which can contain the irrigant fluid.

The reservoir may be made of a film, sheet or membrane, often with a (generally uniform) thickness similar to that of films or sheets used in conventional wound dressing backing layers, i.e. up to 100 micron, preferably up to 50 micron, more preferably up to 25 micron, and of 10 micron minimum thickness, and is often a resiliently flexible, e.g. elastomeric, and preferably soft, hollow body.

In all embodiments of the apparatus the type and material of the tubes throughout the apparatus of the invention for aspirating, irrigating and/or cleansing wounds and the container for cells or tissue and the fluid reservoir will be largely determined by their function.

To be suitable for use, in particular on chronic timescales, the material should be non-toxic and biocompatible, inert to any active components, as appropriate of the irrigant from the fluid reservoir and/or wound exudate in the apparatus flow path, and, in any use of a two-phase system dialysis unit, of the dialysate that moves into the circulating fluid in the apparatus.

When in contact with irrigant fluid, it should not allow any significant amounts of extractables to diffuse freely out of it in use of the apparatus.

It should be sterilisable by ultraviolet, gamma or electron beam irradiation and/or with fluid antiseptics, such as solutions of chemicals, fluid- and microbe-impermeable once in use, and flexible.

Examples of suitable materials for the fluid reservoir include synthetic polymeric materials, such as polyolefins, such as polyethylene, e.g. high-density polyethylene and polypropylene.

Suitable materials for the present purpose also include copolymers thereof, for example with vinyl acetate and mixtures thereof. Suitable materials for the present purpose further include medical grade poly(vinyl chloride).

Notwithstanding such polymeric materials, the fluid reservoir will often have a stiff area to resist any substantial play between it and components that are not mutually integral, such as the fluid supply tube.

It may be stiffened, reinforced or otherwise strengthened, e.g. by a projecting boss.

The device for moving fluid through the wound and means for fluid cleansing may be any appropriate for this purpose, and may act at any appropriate point for this purpose.

It may apply a positive or negative pressure to the wound, although its prime purpose is to move fluid (irrigant from the fluid reservoir and/or wound exudate through the length of the apparatus flow path, rather than to apply a positive or negative pressure to the wound.

If applied to the fluid in recirculation in the fluid recirculation tube upstream of and towards the wound dressing and/or the fluid in the fluid supply tube towards the wound dressing (optionally or as necessary via means for flow switching between supply and recirculation), it will usually apply positive pressure (i.e. above-atmospheric pressure) to the wound bed.

Often the means for fluid cleansing is (most appropriately for its purpose) downstream of the wound dressing, and provides the highest resistance in the flow path. This is especially the case where the means for fluid cleansing is a single-phase system, e.g. with ultrafiltration through microapertures or micropores, thus enhancing applied positive pressure to the wound.

Where the device is applied to the fluid in recirculation in the fluid recirculation tube and/or the fluid in the fluid offtake tube downstream of and away from the wound dressing, it will usually apply negative pressure (i.e. below-atmospheric pressure or vacuum) to the wound bed.

Again, often the means for fluid cleansing is (most appropriately for its purpose) downstream of the wound dressing, and provides the highest resistance in the flow path, thus enhancing applied negative pressure to the wound.

The following types of pump may be used as desired:
reciprocating pumps, such as:
   - shuttle pumps: - with an oscillating shuttle mechanism to move fluids at rates from 2 to 50 ml per minute;
   - diaphragm pumps: - where pulsations of one or two flexible diaphragms displace liquid while check valves control the direction of the fluid flow.
   - piston pumps: - where pistons pump fluids through check valves, in particular for positive and/or negative pressure on the wound bed;
rotary pumps, such as:
   - centrifugal pumps flexible impeller pumps: - where elastomeric impeller traps fluid between impeller blades and a moulded housing that sweeps fluid through the pump housing.
   - progressing cavity pumps: - with a cooperating screw rotor and stator, in particular for higher-viscosity and particulate-filled exudate;
   - rotary vane pumps: - with rotating vaned disk attached to a drive shaft moving fluid without pulsation as it spins. The outlet can be restricted without damaging the pump.
   - peristaltic pumps: - with peripheral rollers on rotor arms acting on a flexible fluid circulation tube to urge fluid current flow in the tube in the direction of the rotor.

The type and/or capacity of the device will be largely determined by
a) the appropriate or desired fluid volume flow rate of irrigant and/or wound exudate from the wound, and
b) whether it is appropriate or desired to apply a positive or negative pressure to the wound bed, and the level of such pressure to the wound bed
for optimum performance of the wound healing, and by factors such as portability, power consumption and isolation from contamination.

Such a device may also suitably be one that is capable of pulsed, continuous, variable, reversible and/or automated and/or programmable fluid movement. It may in particular be a pump of any of these types.

In practice, even from a wound in a highly exuding state, such a rate of exudate flow is only of the order of up to 75 microlitres / cm²/ hr (where cm² refers to the wound area).

The fluid can be highly mobile (owing to the proteases present). Exudate levels drop and consistency changes as the wound heals, e.g. to a level for the same wound that equates to 12.5 - 25 microlitres / cm² / hr.

Where materials deleterious to wound healing are removed by a two-phase system (see below.), such as a dialysis unit, fluid is also potentially lost to the system through the means for fluid cleansing.

This may occur, e.g. through a dialysis polymer film, sheet or membrane which is also permeable to water, in addition to materials deleterious to wound healing.

The balance of fluid in recirculation may thus further decrease, but may be adjusted to minimise this undesired loss in a routine manner as described hereinbefore.

Hence, it will be seen that the circulating fluid from the wound will typically contain a preponderance of irrigant over wound exudate in recirculation from the fluid reservoir.

The type and/or capacity of the device will thus be largely determined in this respect by the appropriate or desired fluid volume flow rate of irrigant, rather than that of exudate, from the wound.

In practice, such a rate of flow of total irrigant and/or wound exudate will be of the order of 1 to 1000, e.g. 3 to 300, and less preferably 1 to 10 ml / cm² /24 hour, where the cm² refers to the wound area.

The volume of irrigant and/or wound exudate in recirculation may vary over a wide range, but will typically be e.g. 1 to 8 l. (for example for large torso wounds), 200 to 1500 ml (for example for axillary and inguinal wounds), and 0.3 to 300 ml for limb wounds when the therapy is applied in this way.

In practice, suitable pressures are of the order of up to 25% atm such as up to 10% atm positive or negative pressure on the wound bed, the apparatus being operated as a closed recirculating system.

The higher end of these ranges are potentially more suitable for hospital use, where relatively high % pressures and/or vacua may be used safely under professional supervision.

The lower end is potentially more suitable for home use, where relatively high % pressures and/or vacua cannot be used safely without professional supervision, or for field hospital use.

The device may be a peristaltic pump or diaphragm pump, e.g. preferably a small portable diaphragm or peristaltic pump. These are preferred types of pump, in order in particular to reduce or eliminate contact of internal surfaces and moving parts of the pump with (chronic) wound exudate, and for ease of cleaning.

It may suitably be one that applies positive pressure to the wound and/or the means for fluid cleansing. A preferred pump when the applied pressure is positive is a peristaltic pump, e.g. a small, portable peristaltic pump, mounted upstream of the means for fluid cleansing.

Where the pump is a peristaltic pump, this may be e.g. an Instech Model P720 miniature peristaltic pump, with a flow rate: of 0.2 - 180ml/hr and a weight of < 0.5 k. This is potentially useful for home and field hospital use.

Where the pump is a peristaltic pump, this may be e.g. an Instech Model P720 miniature peristaltic pump, with a flow rate: of 0.2 - 180ml/hr and a weight of < 0.5 k. This is potentially useful for home and field hospital use.

The pump may suitably be one that applies negative pressure to the wound and/or the means for fluid cleansing. A preferred pump when the applied pressure is negative is a diaphragm pump, e.g. a small, portable diaphragm pump, mounted downstream of the dressing or the means for fluid cleansing.

Where the pump is a diaphragm pump, and preferably a small portable diaphragm pump, the one or two flexible diaphragms that displace liquid may each be, for example a polymer film, sheet or membrane that is connected to means for creating the pulsations. This may be provided in any form that is convenient, inter alia as a piezoelectric transducer, a core of a solenoid or a ferromagnetic integer and coil in which the direction of current flow alternates, a rotary cam and follower, and so on.

The outlet from the dressing passes to the means for fluid cleansing for removal of materials deleterious to wound healing from wound exudate, and in turn to the fluid recirculation tube(s).

In either form in which the two-phase system, such as a dialysis unit, is provided, in use typically the dialysate moves past the circulating fluid in the apparatus in a co- or preferably counter-current direction.

Pumps, such as peristaltic pumps, and/or valves control the direction of the two fluid flows.

However, the cleansing fluid may less usually be static, although this may not provide a system with sufficient (dynamic) surface area to remove materials deleterious to wound healing from wound exudate at a practical rate.

Typical dialysate flow rates in a dialytic means for fluid cleansing in the present apparatus for aspirating, irrigating and/or cleansing wounds are those used in the conventional type of two-phase system, such as a dialysis unit for systemic therapy.

The integer may be a film, sheet or membrane, often of the same type, and of the same (generally uniform) thickness, as those used in conventional two-phase system, such as a dialysis unit for systemic therapy.

The film, sheet or membrane may be substantially flat, and depending on any pressure differential across it may require other materials on or in it to stiffen, reinforce or otherwise strengthen it.

However, this may not provide a system with sufficient functional surface area to remove materials deleterious to wound healing.

To be suitable for use, in particular in chronic wound dialysis, with relatively high concentrations of materials that are deleterious to wound healing, it may be advantageous to provide a system in which the film, sheet or membrane of a polymeric material is in a more convoluted form.

This may be in the form of elongate structures, such as pipes, tubes hollow fibres or filaments or tubules of a round cross-section, e.g. elliptical or circular, e.g. in a parallel array with spaces therebetween.

The wound irrigant and/or wound exudate may recirculate through the inside and the cleansing fluid may pass into the spaces between adjacent pipes, tubes or tubules in a co- or preferably counter-current direction, or vice versa.

Again, materials deleterious to wound healing are removed into the dialysate, and the cleansed fluid, still containing materials that are beneficial in promoting wound healing (including such materials that have been added using cells or tissue) is returned via the recirculation tube to the wound.

Where the means for fluid cleansing is a two-phase system, e.g. in the form of a dialysis unit, or a biphasic extraction unit, the circulating fluid from the wound and the container for cells or tissue and the fluid reservoir passes across one surfaces of a significantly two-dimensional integer, for example a polymer film, sheet or membrane which is selectively permeable to materials deleterious to wound healing.

These are removed by passing a cleansing fluid across the other surface of the integer.

The integer may be a film, sheet or membrane that is selectively permeable to the foregoing materials deleterious to wound healing.

Examples of these as above include
oxidants, such as free radicals, e.g. peroxide and superoxide;
iron II and iron III;
all involved in oxidative stress on the wound bed;
proteases, such as serine proteases, e.g. elastase and thrombin; cysteine proteases; matrix metalloproteases, e.g. collagenase; and carboxyl (acid) proteases;
endotoxins, such as lipopolysaccharides;
bacterial autoinducer signalling molecules, such as homoserine lactone derivatives, e.g. oxo-alkyl derivatives;
inhibitors of angiogenesis such as thrombospondin-1 (TSP-1), plasminogen activator inhibitor, or angiostatin (plasminogen fragment);
pro-inflammatory cytokines such as tumour necrosis factor alpha (TNFα) and interleukin 1 beta (IL-1β); and
inflammatories, such as lipopolysaccharides, and e.g. histamine.

Examples of suitable materials for the film, sheet or membrane (typically in the form of conformable hollow bodies defined by the film, sheet or membrane, such as the structures described hereinbefore) include natural and synthetic polymeric materials.

The membrane may be of one or more hydrophilic polymeric materials, such as a cellulose derivative, e.g. regenerated cellulose, a cellulose mono-, di- or tri- esters, such as cellulose mono-, di- or tri-acetate, benzyl cellulose and Hemophan, and mixtures thereof.

Examples of other materials include hydrophobic materials, such as aromatic polysulphones, polyethersulphones, polyetherether-sulphones, polyketones, polyetherketones and polyetherether-ketones, and sulphonated derivatives thereof, and mixtures thereof.

Examples of other materials include hydrophobic materials, such as
polyesters, such as polycarbonates,
polyamides, e.g. 6-6 and 6 - 10;
polyacrylates, including, e.g. poly(methyl methacrylate),
polyacrylonitrile and
copolymers thereof, for example acrylonitrile - sodium metallosulphonate copolymers; and
poly(vinylidene chloride).

Suitable materials for the present membranes include thermoplastic polyolefins, such as polyethylene e.g. high-density polyethylene, polypropylene, copolymers thereof, for example with vinyl acetate and polyvinyl alcohol, and mixtures thereof.

Such use of the present apparatus, adding such materials using cells or tissue is, e.g. favourable to the wound healing process in chronic wounds, such as diabetic foot ulcers, and especially decubitus pressure ulcers.

As noted hereinafter, antagonists, for example degrading enzymes, or sequestrating agents for elastase on the dialysate side of the membrane, may be used to enhance the removal of this protease from wound exudate. Where it is desired to remove several different materials that are deleterious to wound healing, it may be advantageous to provide a system of modules in series, each of which removes a different material.

This allows incompatible cleansing materials to be used on the same fluid and/or wound exudates.

Preferably any such system is a conventional automated, programmable system which can cleanse the wound irrigant and/or wound exudate with minimal supervision.

As noted above in more detail, fluid passes from a cleansing fluid through a selectively permeable integer.

This may be the typical permeable polymer film, sheet or membrane of a two-phase system, such as a dialysis unit.

Additionally, solutes or disperse phase species will pass from the dialysate into the irrigant and/or wound exudate through the dialysis polymer film, sheet or membrane.

This property may be used to perfuse materials beneficial to wound healing into the irrigant and/or exudate from a dialysate.

In this less conventional type of infusion feed, a broad spectrum of species will usually pass into the exudate and/or irrigant fluid from the dialysate.

These include
ionic species, such as bicarbonate;
vitamins, such as ascorbic acid (vitamin C) and vitamin E, and stable derivatives thereof, and mixtures thereof; to relieve oxidative stress on the wound bed;
pH buffering agents, such as potassium dihydrogen phosphate/ disodium hydrogen phosphate,
local analgesics/anaesthetics, such as lidocaine/lignocaine hydrochloride and xylocaine (adrenoline lidocaine) and/or anti-inflammatories, to reduce wound pain or inflammation or pain associated with the dressing
nutrients to aid proliferation of wound cells, such as amino acids, sugars, low molecular weight tissue building blocks and trace elements; and other cell culture medium species; and
gases, such as air, nitrogen, oxygen and/or nitric oxide.

For the purposes of fluid cleansing in the apparatus of the present invention, both the single-phase system, such as an ultrafiltration unit, and two-phase system, such as a dialysis unit, may have captive (non-labile, insoluble and/or immobilised) species such as the following.

They are bound to an insoluble and/or immobilised) substrate over and/or through which the irrigant and/or wound exudate from, the wound dressing passes in turn to the fluid recirculation tube(s):
antioxidants and free radical scavengers, such as 3-hydroxytyramine (dopamine), ascorbic acid (vitamin C), vitamin E and glutathione, and stable derivatives thereof, and mixtures thereof; to relieve oxidative stress on the wound bed;
metal ion chelators and/or ion exchangers, such as transition metal ion chelators, such as iron III chelators (Fe III is involved in oxidative stress on the wound bed.), such as desferrioxamine (DFO), 3-hydroxytyramine (dopamine);
iron III reductants;
protease inhibitors, such as TIMPs and alpha 1-antitrypsin (AAT); serine protease inhibitors, such as 4-(2-aminoethyl)-benzene sulphonyl fluoride (AEBSF, PefaBloc) and Nα-*p*-tosyl-L-lysine chloro-methyl ketone (TLCK) and ε-aminocaproyl-*p*-chlorobenzylamide; cysteine protease inhibitors; matrix metalloprotease inhibitors; and carboxyl (acid) protease inhibitors;
sacrificial redox materials that are potentially or actually beneficial in promoting wound healing, by the removal of materials that trigger the expression into wound exudate of redox-sensitive genes that are deleterious to wound healing;
autoinducer signalling molecule degraders, which may be enzymes; and anti-inflammatory materials to bind or destroy lipopolysaccharides, e.g. peptidomimetics

Other physiologically active components of the exudate that are deleterious to wound healing may be removed in this way.

These may be removed with suitable chelators and/or ion exchangers, degraders, which may be enzymes, or other species.

The following types of functionalised substrate has sites on its surface that are capable of removing materials deleterious to wound healing on passing the circulating fluid from the wound and the container for cells or tissue and the fluid reservoir over them:
heterogeneous resins , for example silica-supported reagents such as:
   metal scavengers,
   3-(diethylenetriamino)propyl-functionalised silica gel
   2-(4-(ethylenediamino)benzene)ethyl-functionalised silica gel
   3-(mercapto)propyl-functionalised silica gel
   3-(1-thioureido)propyl-functionalised silica gel
   triamine tetraacetate-functionalised silica gel
or electrophilic scavengers,
   4-carboxybutyl-functionalised silica gel
   4-ethyl benzenesulfonyl chloride-functionalised silica gel
   propionyl chloride-functionalised silica gel
   3-(isocyano)propyl-functionalised silica gel
   3-(thiocyano)propyl-functionalised silica gel
   3-(2-succinic anhydride)propyl-functionalised silica gel
   3-(maleimido)propyl-functionalised silica gel
or nucleophilic scavengers,
   3-aminopropyl-functionalised silica gel
   3-(ethylenediamino)-functionalised silica gel
   2-(4-(ethylenediamino)propyl-functionalised silica gel
   3-(diethylenetriamino)propyl-functionalised silica gel
   4-ethyl-benzenesulfonamide-functionalised silica gel
   2-(4-toluenesulfonyl hydrazino)ethyl-functionalised silica gel
   3-(mercapto)propyl-functionalised silica gel
   dimethylsiloxy-functionalised silica gel
   or base or acid scavengers,
   3-(dimethylamino)propyl-functionalised silica gel
   3-(1,3,4,6,7,8-hexahydro-2H-pyrimido-[1,2-α]pyrimidino)propyl-functionalised silica gel
   3-(1-imidazol-1-yl)propyl-functionalised silica gel
   3-(1-morpholino)propyl-functionalised silica gel
   3-(1-piperazino)propyl-functionalised silica gel
   3-(1-piperidino)propyl-functionalised silica gel
   3-(4,4'-trimethyldipiperidino)propyl-functionalised silica gel
   2-(2-pyridyl)ethyl-functionalised silica gel
   3-(trimethylammonium)propyl-functionalised silica gel
or the reagents,
   3-(1-cyclohexylcarbodiimido)propyl-functionalised silica gel
   TEMPO-functionalised silica gel
   2-(diphenylphosphino)ethyl-functionalised silica gel
   2-(3,4-cyclohexyldiol)propyl-functionalised silica gel
   3-(glycidoxy)propyl-functionalised silica gel
   2-(3,4-epoxycyclohexyl)propyl-functionalised silica gel
   1-(allyl)methyl-functionalised silica gel
   4-bromopropyl-functionalised silica gel
   4-bromophenyl-functionalised silica gel
   3-chloropropyl-functionalised silica gel
   4-benzyl chloride-functionalised silica gel
   2-(carbomethoxy)propyl-functionalised silica gel
   3-(4-nitrobenzamido)propyl-functionalised silica gel
   3-(ureido)propyl-functionalised silica gel
or any combinations of the above.

The use of such captive (non-labile, insoluble and/or immobilised) species, such as the foregoing, bound to an insoluble and immobilised) substrate over and/or through which the irrigant and/or wound exudate from, the wound dressing passes has been described hereinbefore as suitable for the means for fluid cleansing.

However, they may additionally, where appropriate, be used in any part of the apparatus that is in contact with the irrigant and/or wound exudate, but often within the dressing, for removal of materials deleterious to wound healing.

The means for fluid cleansing may additionally, where appropriate, comprise one or more macroscopic and/or microscopic filters.

These are to retain particulates, e.g. cell debris and micro-organisms, allowing proteins and nutrients to pass through.

Circulating wound fluid aids in the quicker attainment of this equilibrium of materials beneficial in promoting wound healing (including such materials that have been added using cells or tissue)

It also returns them to the site where they can be potentially of most benefit, i.e. the wound bed.

It is believed that circulating wound fluid aids in removal from recirculation of the materials deleterious to wound healing, whilst retaining materials that are beneficial in promoting wound healing (including such materials that have been added using cells or tissue) in contact with the wound.

Both the single-phase system, such as an ultrafiltration unit, and two-phase system, such as a dialysis unit, may be in modular form that is relatively easily demountable from the apparatus of the invention. The system may suitably comprise one or more such modules.

The conduits through which respectively
d) the irrigant and/or wound exudate passes from the wound dressing and
e) the cleansed fluid is returned to the recirculation tube, and
f) (in the case where the means is provided in the form of a two-phase system, such as an dialysis unit) through which the cleansing fluid enters and exits the means
preferably have means for, on module disconnection and withdrawal,
iii) switching off the flow and
iv) providing an immediate fluid-tight seal or closure over the ends of the conduits and the cooperating tubes in the rest of the apparatus of the invention so exposed,
to prevent continuing passage of irrigant and/or exudate and cleansed fluid, and cleansing fluid.

The apparatus of the invention for aspirating, irrigating and/or cleansing wounds is provided with means for bleeding the offtake and/or recirculation tubes, such as a regulator, such as a valve or other control device for bleeding fluids from the wound.

The device for moving fluid through the wound and means for fluid cleansing is used to move irrigant to the wound dressing and apply the desired positive or negative pressure on the wound bed.

The desired balance of fluid in recirculation tube will typically be regulated by means of
a) the means for bleeding the offtake and/or recirculation tubes,
b) the means for flow switching between supply and recirculation, and/or
c) the means for moving fluid over the wound bed and through the means for fluid cleansing,
as appropriate.

Thus, e.g. if
a) the apparatus for aspirating, irrigating and/or cleansing wounds is a single-phase system, such as an ultrafiltration unit,
b) the wound is not in a highly exuding state and
c) it is not appropriate or desired to admit fluid into the wound from the fluid reservoir,
there is no or negligible change in the balance of fluid in recirculation. Once it has been primed throughout, e.g. to the desired positive or negative pressure on the wound bed, the apparatus may be operated as a closed recirculating system.

The means for flow switching between supply and recirculation tubes is set to close the wound to the fluid reservoir via the fluid supply tube, and the means for bleeding the offtake and/or recirculation tubes are also closed.

If
a) the apparatus for aspirating, irrigating and/or cleansing wounds is a single-phase system, such as an ultrafiltration unit,
b) the wound is in a highly exuding state and/or
c) it is appropriate or desired to admit fluid into the wound from the fluid reservoir,
there is a positive change in the balance of fluid in recirculation.

Once it has been primed throughout, e.g. to the desired positive or negative pressure on the wound bed, the apparatus cannot be operated as a closed recirculating system, without the pressure to the wound bed increasing, possibly undesirably.

The means for bleeding the offtake and/or recirculation tubes must be opened to some extent to relieve positive pressure on the wound bed. The bleed-off may be voided to waste, e.g. to a collection bag.

Materials that are beneficial in promoting wound healing, including such materials added using cells or tissue, may be lost to the site where they can be potentially of most benefit, i.e. the wound bed, when the therapy is applied in this way.

However, the balance of fluid in recirculation may be routinely adjusted to minimise this undesired loss.

The factors that determine the balance of fluid in recirculation in an apparatus with a two-phase system means for fluid cleansing in the form of a dialysis unit, or a biphasic extraction unit have been described hereinbefore in detail hereinbefore in connection with the operation of the apparatus.

It is sufficient to note here that at some point after steady state recirculation established through the length of the apparatus flow path, it may be necessary that any bleed valve is opened, if overall the fluid level is increasing by transfer from the dialysate to an undesirable extent.

Other combinations, and the necessary adjustments to maintain the desired balance of fluid in recirculation tube by means of
a) the means for bleeding the offtake and/or recirculation tubes,
b) the means for flow switching between supply and recirculation, and/or
c) the means for moving fluid
will be apparent to the skilled person.

The outlet from the means for bleeding the offtake and/or recirculation tubes may be collected and monitored and used to diagnose the status of the wound and/or its exudate.

The waste reservoir may be of any conventional type, e.g. a tube, bag (such as a bag typically used as an ostomy bag), chamber, pouch or other structure, e.g. of polymer film, which can contain the irrigant fluid that has been bled off. In all embodiments of the apparatus, the type and material of the waste reservoir will be largely determined by its function. To be suitable for use, the material need only be fluid-impermeable once in use, and flexible.

Examples of suitable materials for the fluid reservoir include synthetic polymeric materials, such as polyolefins, such as poly (vinylidene chloride).

Suitable materials for the present purpose also include polyethylene, e.g. high-density polyethylene, polypropylene, copolymers thereof, for example with vinyl acetate and mixtures thereof.

In a second aspect of the present invention there is provided a conformable wound dressing, characterised in that it comprises a backing layer with a wound-facing face which is capable of forming a relatively fluid-tight seal or closure over a wound and has
at least one inlet pipe for connection to a fluid supply tube, which passes through and/or under the wound-facing face, and
at least one outlet pipe for connection to a fluid offtake tube, which passes through and/or under the wound-facing face,
the point at which the or each inlet pipe and the or each outlet pipe passes through and/or under the wound-facing face forming a relatively fluid-tight seal or closure over the wound.

The dressing is advantageously provided for use in a bacteria-proof pouch.

Examples of suitable forms of such wound dressings are as described by way of example hereinbefore.

It is foreseen that the actives to be added to the wound bed maybe the nutrient medium, that human or mammalian cells e.g. keratinocytes, fibroblast or a mixture of these cells, or others for instance, have grown in (conditioned media). The cells will release beneficial actives to the media e.g. TGFβ that would benefit the wound bed and aid healing of the wound.

In some embodiments of the present invention the actual cells themselves with or without the cells growth media, maybe used as an active to the wound bed to aid healing. In particular embodiments of the present invention different types of cells maybe used as actives at different times of the healing process. For example, fibroblast type cells maybe used as an active to the wound bed to aid healing initially in order to help would remodelling and aid the wound to lay down structural fibres. Then keratinocytes or a larger proportion of keratinocytes than initially used before could be used as an active flowing along the wound bed to aid healing. Other cells could be used as well or combination thereof.

It is foreseen that the cells (keratinocytes or fibroblasts) can aid healing of the wound by giving beneficial healing components or by sticking to the wound bed and aiding healing directly.

When conditioned media is used, (the media that has had cells grown in it) different conditioned media from different cell source may be used and it is envisaged that having a particular order to which conditioned media to use may be important and aid healing. For example, conditioned media from fibroblast type cells or a mixture of cells comprising a high proportion of fibroblast cells may be used initially followed by a conditioned media from keratinocyte type cells or a mixture of cells comprising a higher proportion of keratinocyte than used before. It is foreseen that this will aid healing of the wound.

In some embodiments of the present invention there may be a wound contact layer. The wound contact layer may be made from any suitable material known in the art (e.g. gauze or foam) which will allow nutrients to reach the wound bed. Having a wound contact layer may prevent overgrowth of the granulation material.

In some embodiments of the present invention, a significant advantage, in particular in chronic wounds, is that in use granulation tissue is encouraged to grow onto and/or into the wound contact layer that lies between the wound film dressing and the wound bed.

The effect may be further enhanced by the circulation over the wound bed of irrigant from the fluid reservoir which contains nutrients for wound cells to aid proliferation, and other molecules that are beneficially involved in wound healing and/or that are favourable to the wound healing process.

A further particular advantage is that it is unnecessary to remove this granulation tissue in-growth on dressing change, as the wound contact layer may be left between the wound film dressing and the wound bed biodegrade. This minimises trauma and any need for debridement.

A particular advantage of this wound contact layer is its use with pressure sores: the device can be placed in the depths of the wound and the patient can lie upon it without either affecting the utility of the device or further damaging the wound. This becomes critical if the patient cannot be moved from this posture for other medical reasons.

The wound contact layer is placed over substantially the expanse of the wound, and its size and configuration can be adjusted to fit the individual wound. It can be formed from a variety of apertured, semi-rigid materials.

By 'apertured' herein is meant materials that are porous, apertured, holed, open-mesh, slit, incised and/or cut.

The material must be sufficiently apertured to allow for invasion by all manner of cells involved in the process of tissue repair and wound healing, and/or for the inward growth of blood vessels, and sufficiently rigid to prevent overgrowth and collapse under suction.

Suitable biomaterials for a biodegradable wound contact layer include poly(hydroxy acids) and esters thereof, such as poly(glycolic acid), poly(L-lactic acid), poly(D-lactic acid) and esters thereof, and copolymers and blends of the aforementioned.

Suitable biomaterials also include poly(acid anhydrides), such as poly(terephthalic acid), poly(adipic acid) and copolymers and blends of the aforementioned.

Additionally, biologically sourced biodegradable polymeric materials may be used, such as substantially protein based polymers, for example collagens, fibronectins, or fibrins, either as whole molecules or those subjected to proteolytic or chemical treatments, in either degraded or native conformations, or modified protein based polymers produced by nucleic acids recombinant techniques, for example, collagens, fibronectins, or fibrins, or fragments thereof, produced through recombinant DNA techniques; or blends thereof.

Further acceptable wound contact layers will be combinations of protein based scaffolds and carbohydrate based polymers such as glycosoaminoglycans, chitosans, cellulose or alginate molecules.

Suitable materials also include human or animal derived tissues processed in means to make them acceptable in placement into the wound such as skin, alimentary tract or connective tissues.

The wound contact layer/material may be formed in a variety of apertured, semi-rigid forms.

These forms may be essentially two-dimensional, such as sheets, layers, films, flexible panels, meshes, nets, webs or lattices. They may be planed in the wound as dry, hydrated or gel based formulations.

One embodiment of apertured or holed scaffold comprises a section of honeycombed polymer sheet cut to the shape of the wound.

Where the wound contact layer is in an essentially two-dimensional apertured, semi-rigid form, such as a sheet, layer, film, flexible panel, mesh, net, web or lattice, it may be designed in a configuration that is able to conform well to the wound bed on insertion into the wound.

This conforming to shape is then a particular advantage in those embodiments where the wound dressing is used on deeper wounds, especially where a wound filler is used to urge the wound dressing towards the wound contact layer and wound bed, as described hereinafter in connection with the wound dressing.

By way of example, such a wound contact layer may be in the form of a deeply indented circular disc much like a multiple Maltese cross or a stylised rose. This form is able to conform well to the wound bed on insertion into the wound, especially a deeper wound, by the arms closing in and possibly overlapping.

The form of the wound contact layer may also be three-dimensional, such as sheets, layers, films, flexible panels, meshes, nets, webs and lattices, folded, creased, pleated, tucked, crinkled, crumpled, screwed up or twisted into a three-dimensional form.

Alternatively, these forms may be inherently three-dimensional, such as multilayers of films, flexible panels, meshes, nets, webs and lattices, or three-dimensional meshes, nets, webs and lattices, and favourably foams.

They may be placed in the wound as dry, hydrated or gel based formulations.

Examples may also include:
a suction head having a first face;
a second face opposite said first face, wherein said second face is comprised of a plurality of projections, said projections defining a plurality of channels for facilitating flow of fluids to an opening in said second face and through said first face, wherein said opening is adapted for connection to a suction tube; and
a surgical drape having an aperture coincident said opening, said surgical drape extending over a region, and overlapping beyond the perimeter of said first face, and wherein said surgical drape comprises a flexible adhesive coated film adhered to said region of said first face and a release-coated backing extending over said second face and adhered to the overlapping portion of said surgical drape.

For distributing fluid across a wound surface, the present invention may also include:
a suction head having a first face;
a second face opposite said first face;
a plurality of projections coincident from said second face, wherein said projections form a contact surface with the wound surface, and wherein a plurality of channels for facilitating flow of fluids are defined between said projections, said channels remaining out of contact with the wound surface; and
an aperture in fluid communication with said channels formed by said projections and formed through said first face and second face.

The present invention will now be described by way of example only with reference to the accompanying drawings in which:
Figure 1 is a schematic view of an apparatus for aspirating, irrigating and/or cleansing a wound according to the first aspect of the present invention.
   It has a single-phase system means for fluid cleansing in the form of an ultrafiltration unit.
Figure 2 is a schematic view of an apparatus for aspirating, irrigating and/or cleansing a wound according to the first aspect of the present invention.
   It has a two-phase system means for fluid cleansing in the form of a dialysis unit, or a biphasic extraction unit.
Figures 3 to 7 are cross-sectional views of conformable wound dressings, of the second aspect of the present invention for aspirating and/or irrigating wounds.
   In these, Figures 3a to 6a are cross-sectional plan views of the wound dressings, and Figures 3b to 6b are cross-sectional side views of the wound dressings.
Figures 8 to 10 are various views of inlet and outlet manifold layouts for the wound dressings of the second aspect of the present invention for respectively delivering fluid to, and collecting fluid from, the wound.
Figure 11 is a schematic view of an apparatus for aspirating, irrigating and/or cleansing a wound according to the first aspect of the present invention.
   It has a single-phase system means for fluid cleansing in the form of an ultrafiltration unit.
Figure 12 is a schematic view of an apparatus for aspirating, irrigating and/or cleansing a wound according to the first aspect of the present invention.
   It has a two-phase system means for fluid cleansing in the form of a dialysis unit, or a biphasic extraction unit.
Figures 13 to 27 are cross-sectional views of conformable wound dressings of the second aspect of the present invention for aspirating and/or irrigating wounds.
Figure 28 is a schematic view of an apparatus for aspirating, irrigating and/or cleansing a wound according to the first aspect of the present invention.
Figure 29 shows a schematic representation Exudialysis flow system as used in Example 1, according to the present invention.
Figure 30 shows WST activity of fibroblasts with the addition of Dermagraft (the source of actives from live cells) in comparison to a media only control (TCM).
Figure 31 shows WST activity of fibroblasts
   (i) with an exudialysis system TCM + catalase
   (ii) in a media with the addition of Dermagraft (the source of actives from live cells) Dg and hydrogen peroxide H₂O₂
   (iii) in a media with the addition of Dermagraft (the source of actives from live cells) Dg, hydrogen peroxide (H₂O₂) and with an exudialysis system (+ catalase).

It has a single-phase system means for fluid cleansing in the form of an ultrafiltration unit.

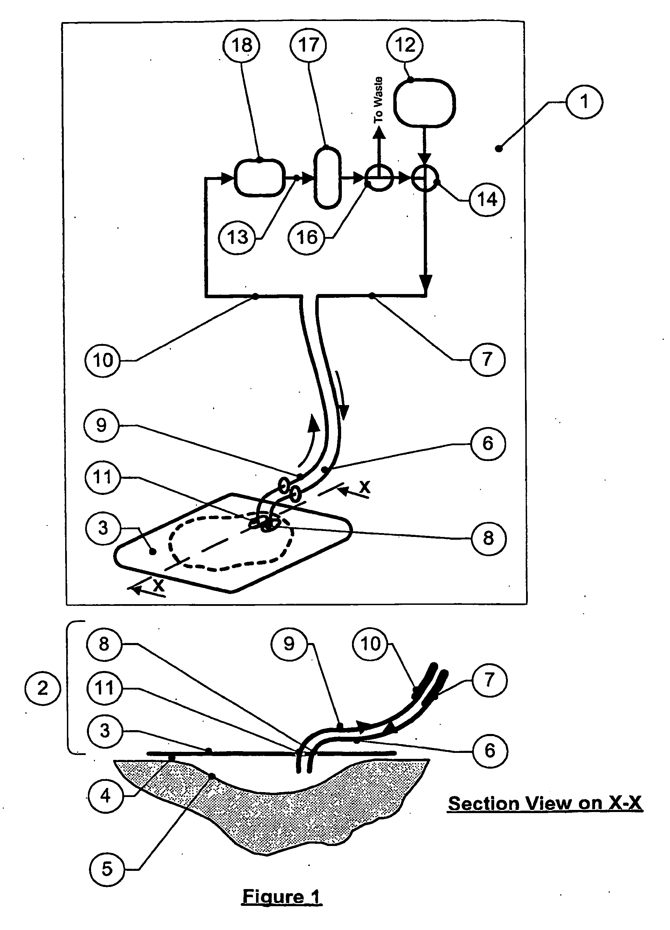

Referring to Figure 1, the apparatus (1) for aspirating, irrigating and/or cleansing wounds comprises
a conformable wound dressing (2), having
a backing layer (3) which is capable of forming a relatively fluid-tight seal or closure (4) over a wound (5) and
one inlet pipe (6) for connection to a fluid supply tube (7), which passes through the wound-facing face of the backing layer (5) at (8), and
one outlet pipe (9) for connection to a fluid offtake tube (10), which passes through the wound-facing face at (11),
the points (8), (11) at which the inlet pipe and the outlet pipe passes through and/or under the wound-facing face forming a relatively fluid-tight seal or closure over the wound,
the inlet pipe being connected via means for flow switching between supply and recirculation, here a T- valve (14), by the fluid supply tube (7) to a container for cells or tissue in series with a fluid reservoir (the container and reservoir being shown as a single integer (12)) and to a fluid recirculation tube (13) having a means for bleeding the tube, here a bleed T-valve (16) to waste, e.g. to a collection bag (not shown),
the outlet pipe (9) being connected to a fluid offtake tube (10), connected in turn to
means for fluid cleansing (17), here in the form of an ultrafiltration unit, connected to the inlet pipe (6) via the fluid recirculation tube (13) and T-valve (14), and
a device for moving fluid through the wound and means for fluid cleansing (17), here a peristaltic pump (18), e.g. preferably a small portable peristaltic pump, acting on the fluid circulation tube (13) with the peripheral rollers on its rotor (not shown) to apply a low negative pressure on the wound.

In use, the inlet pipe, means for flow switching between supply and recirculation T- valve (14), the fluid supply tube (7) and the container for cells or tissue (part of the integer (12)) contain physiologically active components from the cells or tissue in therapeutically active amounts to promote wound healing, and adds such materials into the flowpath.

The supply of such physiologically active materials may be effected at any appropriate point for this purpose along the apparatus flow path, but it is (as here) often convenient to effect such supply to the wound via the fluid in recirculation through the wound dressing from irrigant in the container that contains the cells or tissue.

The ultrafiltration unit (17) is a single-phase system. In this the circulating fluid from the wound and the container for cells or tissue and the fluid reservoir passes through a self-contained system in which materials deleterious to wound healing are removed and the cleansed fluid, still containing materials that are beneficial in promoting wound healing is returned via the recirculation tube to the wound bed.

(In a variant of this apparatus, there are two inlet pipes (6), which are connected respectively to a fluid supply tube (7) and fluid recirculation tube (13), respectively having a first valve (19) for admitting fluid into the wound from the container for cells or tissue and the fluid reservoir (together the integer (12)) and a second valve (20) for admitting fluid into the wound from the recirculation tube. Usually in use of the apparatus, when the first valve (19) is open, the second valve (20) is shut, and vice versa.) In use of the apparatus (1), the valve (16) is opened to a collection bag (not shown), and the T- valve (14) is turned to admit fluid from the container for cells or tissue and fluid reservoir (together the integer (12)) to the wound dressing through the fluid supply tube (7) and inlet pipe (6).

(In the variant of this apparatus having two inlet pipes (6), which are connected respectively to a fluid supply tube (7) and fluid recirculation tube (13), the first valve (19) for admitting fluid into the wound from the container for cells or tissue and the fluid reservoir (together the integer (12)) is opened and the second valve (20) is shut, and vice versa.)

The pump (18) is started to nip the fluid recirculation tube (13) with the peripheral rollers on its rotor (not shown) to apply a low positive pressure on the wound. It is allowed to run until the apparatus is primed throughout the whole length of the apparatus flow path and excess fluid is voided to waste via the bleed T-valve (16) into the collection bag (not shown).

The T-valve (14) is then turned to switch from supply and recirculation, i.e. is set to close the wound to the container for cells or tissue and the fluid reservoir (together the integer (12)) but to admit fluid into the wound from the fluid recirculation tube (13), and the bleed T-valve (16) is simultaneously closed.

(In the variant of this apparatus, where there are two inlet pipes (6), which are connected respectively to a fluid supply tube (7) and fluid recirculation tube (13), the first valve (19) is closed and a recirculating system set up by opening the second valve (20) for admitting fluid into the wound from the recirculation tube (13).

The circulating fluid from the wound and the container for cells or tissue and the fluid reservoir (together the integer (12)) passes through the ultrafiltration unit (17).

Materials deleterious to wound healing are removed and the cleansed fluid, still containing materials that are beneficial in promoting wound healing, is returned via the recirculation tube (13) to the wound bed.

The recirculation of fluid may be continued as long as desired.

Switching between supply and recirculation is then reversed, by turning the T- valve (14) to admit fluid from the fluid reservoir and the container for cells or tissue to the wound dressing through the fluid supply tube (7) and inlet pipe (6).

(In the variant of this apparatus having two inlet pipes (6), which are connected respectively to a fluid supply tube (7) and fluid recirculation tube (13), the first valve (19) for admitting fluid into the wound from the container for cells or tissue and the fluid reservoir (together the integer (12)) is opened and the second valve (20) is shut, and vice versa.)

The bleed valve (16) is simultaneously opened, so that fresh fluid flushes the recirculating system.

The running of the pump (18) may be continued until the apparatus is flushed, when it and the fluid recirculation is stopped.

If, e.g. the wound is in a highly exuding state, there is a positive change in the balance of fluid in recirculation. It may be necessary to bleed fluid from recirculation, by opening the bleed T-valve (16) to bleed fluid from the recirculation tube (13).

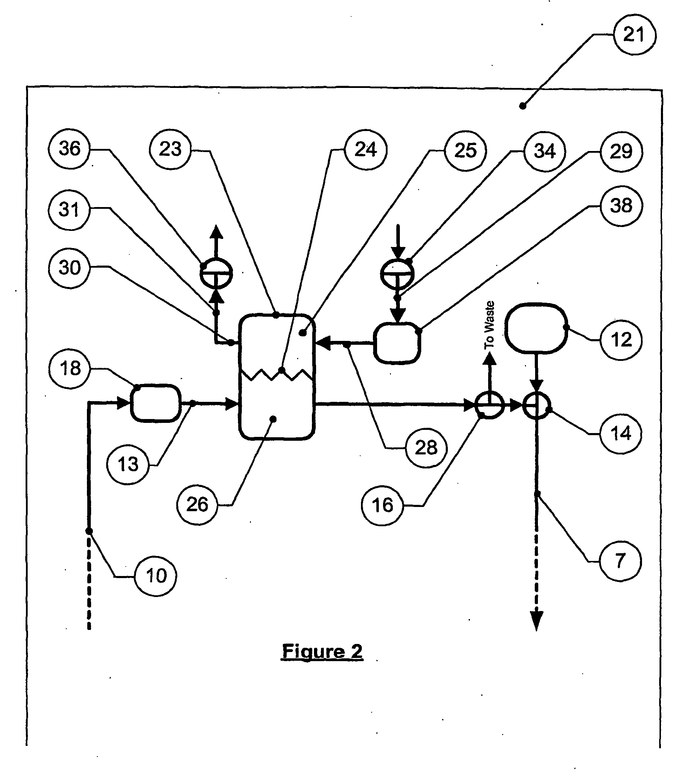

Referring to Figure 2, the apparatus (21) is a variant of that of Figure 1, with identical, and identically numbered, components, except for the means for fluid cleansing, which is in the form of a two-phase system, here a dialysis unit (23).

In this, there is one system through which the circulating fluid from the wound and the container for cells or tissue and the fluid reservoir passes and from which deleterious materials are removed by selectively permeable contact with a second system, through which passes a cleansing fluid.

The dialysis unit (23) thus has an internal polymer film, sheet or membrane (24), selectively permeable to materials deleterious to wound healing, which divides it into
a) a first chamber (25), through which passes a cleansing fluid across one surface of the polymer film, sheet or membrane, and
b) a second chamber (26), through which passes the circulating fluid from the wound and the container for cells or tissue and the fluid reservoir (together the integer (12)), and from which deleterious materials are removed

The dialysis unit (23) thus has a dialysate inlet pipe (28) connecting to a dialysate supply tube (29) which passes to a peristaltic pump (38), e.g. preferably a small portable peristaltic pump, acting on the dialysate supply tube (29) with the peripheral rollers on its rotor (not shown) to supply cleansing fluid across the surface of the polymer film, sheet or membrane (28) in the first chamber (25) from a dialysate reservoir (not shown) via a valve (34).

The dialysis unit (23) also has a dialysate outlet pipe (30) connecting to a dialysate outlet tube (31) which passes to waste via a second bleed T-valve (36) into, e.g. a collection bag (not shown).

Operation of this apparatus is similar to that of Figure 1, except for the dialysis unit (27), in that at some point after the irrigation system is primed and steady state recirculation established through the length of the apparatus flow path, the valve (34) and second bleed valve (36) are opened.

The pump (38) is started to nip fluid dialysate tube (29) with the peripheral rollers on its rotor (not shown) to pump cleansing fluid to the first chamber from a dialysate reservoir (not shown) and out to waste via the bleed valve (36) into the collection bag (not shown).

The dialysis unit (23) is a module (or scrubbing cartridge) with a substrate that changes colour to indicate the presence of detrimental factors in the cleansed fluid, and that the scrubbing cartridge is exhausted and should be renewed.

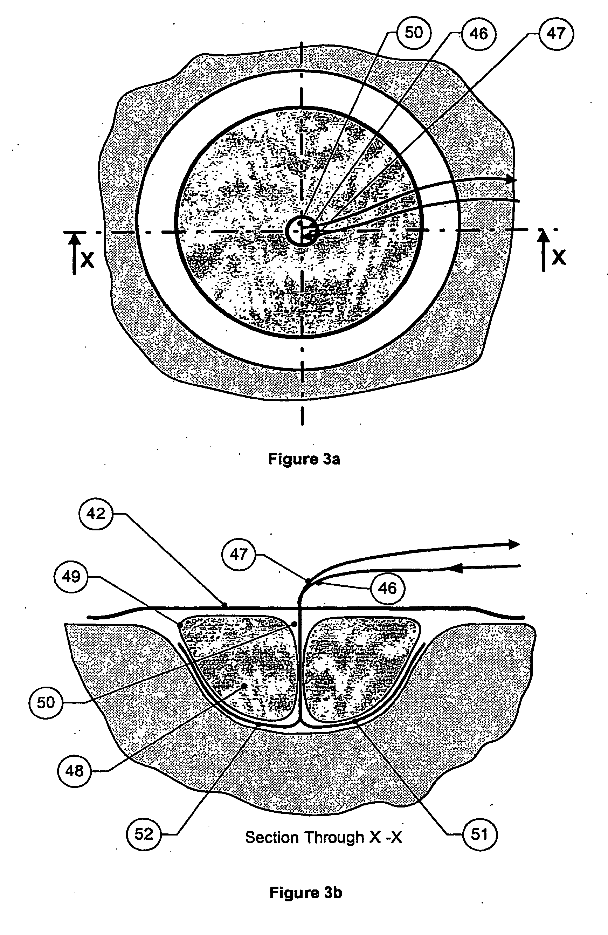

Referring to Figures 3 to 6, each dressing (41) is in the form of a conformable body defined by a microbe-impermeable film backing layer (42) with a uniform thickness of 25 micron, with a wound-facing face (43) which is capable of forming a relatively fluid-tight seal or closure over a wound.

The backing layer (42) extends in use on a wound over the skin around the wound. On the proximal face of the backing layer (43) on the overlap (44), it bears an adhesive film (45), to attach it to the skin sufficiently to hold the wound dressing in place in a fluid-tight seal around the periphery of the wound-facing face (43) of the wound dressing.

There is one inlet pipe (46) for connection to a fluid supply tube (not shown), which passes through and/or under the wound-facing face (43), and one outlet pipe (47) for connection to a fluid offtake tube (not shown), which passes through and/or under the wound-facing face (43).

Referring to Figures 3a and 3b, one form of the dressing is provided with a wound filler (48) under a circular backing layer (42).

This comprises a generally frustroconical, toroidal conformable hollow body, defined by a membrane (49) which is filled with a fluid, here air or nitrogen, that urges it to the wound shape.

The filler (48) may be permanently attached to the backing layer with an adhesive film (not shown) or by heat-sealing.

The inlet pipe (46) and outlet pipe (47) are mounted centrally in the backing layer (42) above the central tunnel (50) of the toroidal hollow body (48) and each passes through the backing layer (42), and each extends in pipes (51) and (52) respectively through the tunnel (50) of the toroidal hollow body (48) and then radially in diametrically opposite directions under the body (48).

This form of the dressing is a more suitable layout for deeper wounds.

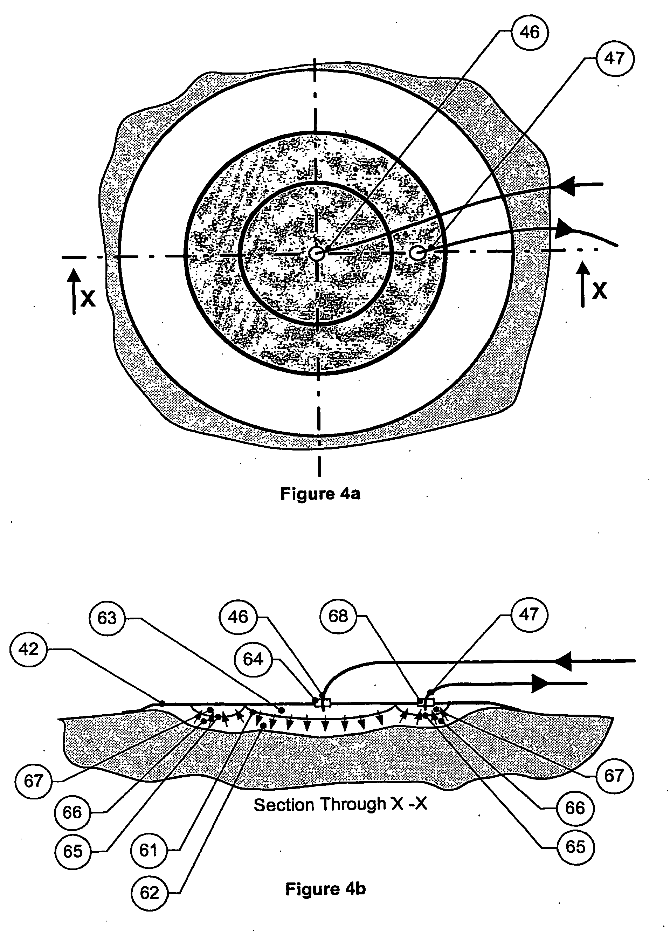

Referring to Figures 4a and 4b, a more suitable form for shallower wounds is shown. This comprises a circular backing layer (42) and a circular upwardly dished first membrane (61) with apertures (62) that is permanently attached to the backing layer (42) by heat-sealing to form a circular pouch (63).

The pouch (63) communicates with the inlet pipe (46) through a hole (64), and thus effectively forms an inlet pipe manifold that delivers the circulating fluid directly to the wound when the dressing is in use.

An annular second membrane (65) with openings (66) is permanently attached to the backing layer (42) by heat-sealing to form an annular chamber (67) with the layer (42).

The chamber (67) communicates with the outlet pipe (47) through an orifice (68), and thus effectively forms an outlet pipe manifold that collects the fluid directly from the wound when the dressing is in use.

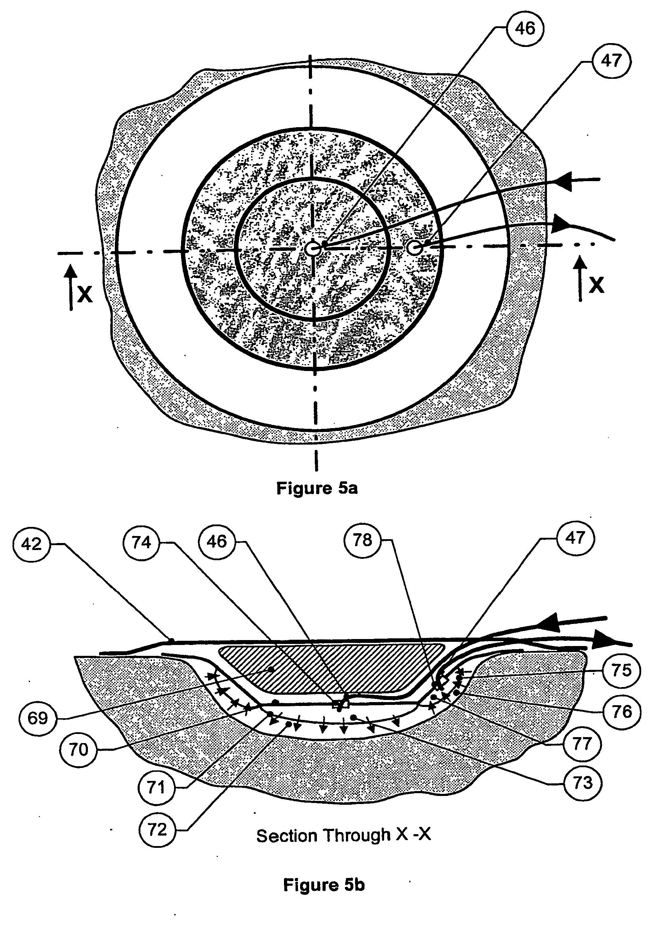

Referring to Figures 5a and 5b, a variant of the dressing of Figures 4a and 4b that is a more suitable form for deeper wounds is shown.

This comprises a circular backing layer (42) and a filler (69), in the form of an inverted frustroconical, solid integer, here a resilient elastomeric foam, formed of a thermoplastic, or preferably a cross-linked plastics foam.

It is permanently attached to the backing layer (42), with an adhesive film (not shown) or by heat-sealing.

A circular upwardly dished sheet (70) lies under and conforms to, but is a separate structure, permanently unattached to, the backing layer (42) and the solid integer (69).

A circular upwardly dished first membrane (71) with apertures (72) is permanently attached to the sheet (70) by heat-sealing to form a circular pouch (73) with the sheet (70).

The pouch (73) communicates with the inlet pipe (46) through a hole (74), and thus effectively forms an inlet pipe manifold that delivers the circulating fluid directly to the wound when the dressing is in use.

An annular second membrane (75) with openings (76) is permanently attached to the sheet (70) by heat-sealing to form an annular chamber (77) with the sheet (70).

The chamber (77) communicates with the outlet pipe (47) through an orifice (78), and thus effectively forms an outlet pipe manifold that collects the fluid directly from the wound when the dressing is in use.

Alternatively, where appropriate the dressing may be provided in a form in which the circular upwardly dished sheet (70) functions as the backing layer and the solid filler (69) sits on the sheet (70) as the backing layer, rather than under it. The filler (69) is held in place with an adhesive film or tape, instead of the backing layer (42).

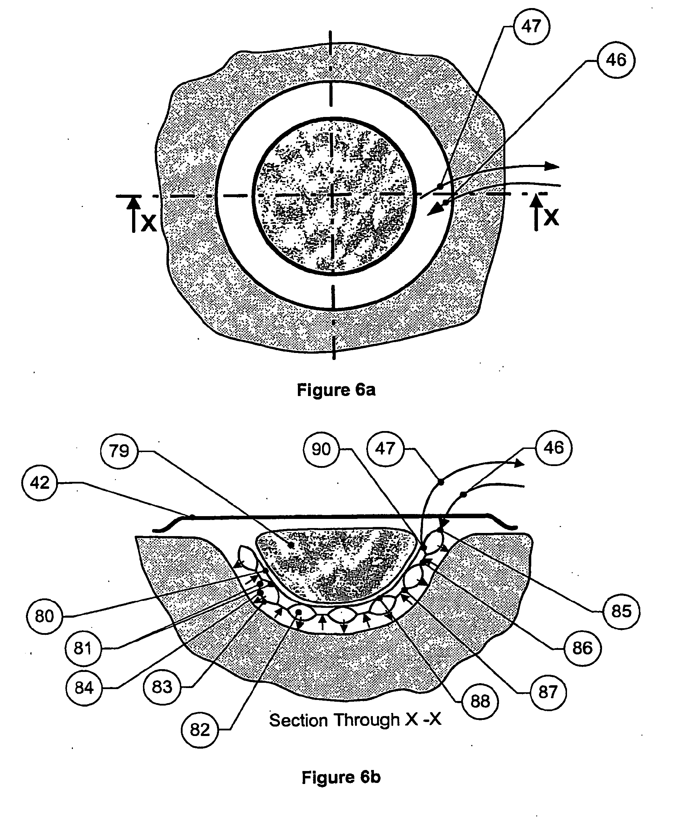

Referring to Figures 6a and 6b, a dressing that is a more suitable form for deeper wounds is shown.

This comprises a circular backing layer (42) and a filler (79), in the form of an inverted generally hemispherical integer, here a resilient elastomeric foam or a hollow body filled with a fluid, here a gel that urges it to the wound shape, and permanently attached to the backing layer with an adhesive film (not shown) or by heat-sealing.

The inlet pipe (46) and outlet pipe (47) are mounted peripherally in the backing layer (42).

A circular upwardly dished sheet (80) lies under and conforms to, but is a separate structure, permanently unattached to, the backing layer (42) and the filler (79).

A circular upwardly dished bilaminate membrane (81) has a closed channel (82) between its laminar components, with perforations (83) along its length on the outer surface (84) of the dish formed by the membrane (81) and
an opening (85) at the outer end of its spiral helix, through which the channel (82) communicates with the inlet pipe (46),
and thus effectively forms an inlet pipe manifold that delivers the circulating fluid directly to the wound when the dressing is in use.

The membrane (81) also has apertures (86) between and along the length of the turns of the channel (82).

The inner surface (87) of the dish formed by the membrane (81) is permanently attached at its innermost points (88) with an adhesive film (not shown) or by heat-sealing to the sheet (80). This defines a mating closed spirohelical conduit (89).

At the outermost end of its spiral helix, the conduit (89) communicates through an opening (90) with the outlet pipe (47) and is thus effectively an outlet manifold to collect the fluid directly from the wound via the apertures (86).

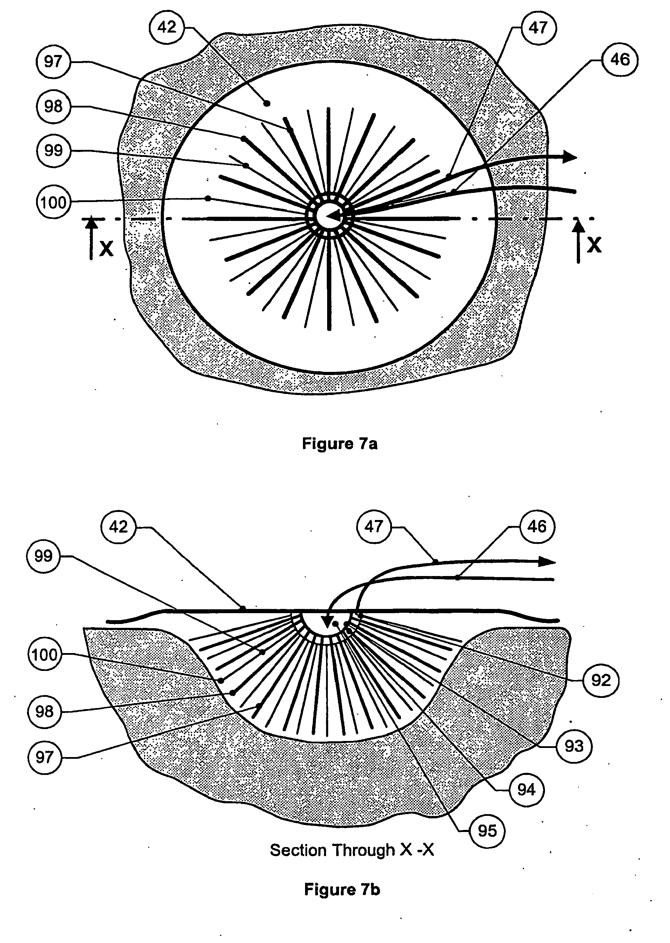

Referring to Figures 7a and 7b, one form of the dressing is provided with a circular backing layer (42). A first (larger) inverted hemispherical membrane (92) is permanently attached centrally to the layer (42) by heat-sealing to form a hemispherical chamber (94) with the layer (42). A second (smaller) concentric hemispherical membrane (93) within the first is permanently attached to the layer (42) by heat-sealing to form a hemispherical pouch (95).

The pouch (95) communicates with the inlet pipe (46) and is thus effectively an inlet manifold, from which pipes (97) radiate hemispherically and run to the wound bed to end in apertures (98).

The pipes (97) deliver the circulating fluid directly to the wound bed via the apertures (98).

The chamber (94) communicates with the outlet pipe (47) and is thus effectively an outlet manifold from which tubules (99) radiate hemispherically and run to the wound bed to end in openings (100). The tubules (99) collect the fluid directly from the wound via the openings (100).

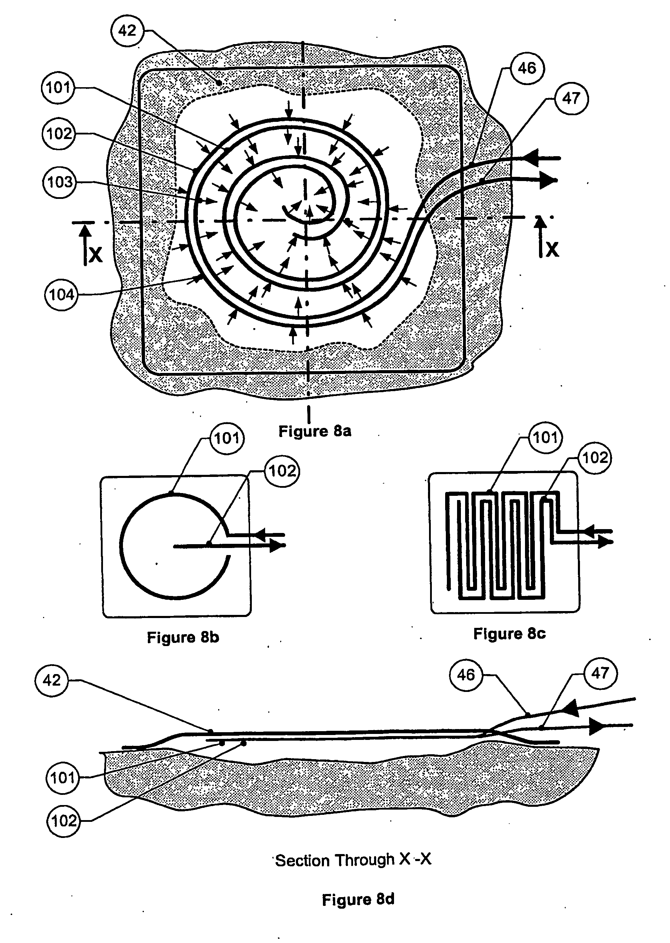

Referring to Figures 8a to 8d, one form of the dressing is provided with a square backing layer (42) and
first tube (101) extending from the inlet pipe (46), and
second tube (102) extending from the outlet pipe (47)
at the points at which they pass through the backing layer, to run over the wound bed.

These pipes (101), (102) have a blind bore with orifices (103), (104) along the pipes (101), (102).

These pipes (101), (102) respectively form an inlet pipe or outlet pipe manifold that delivers the circulating fluid directly to the wound bed or collects the fluid directly from the wound respectively via the orifices.

In Figures 8a and 8d, one layout of each of the pipes (101), (102) as inlet pipe and outlet pipe manifolds is a spiral.

In Figure 8b, the layout is a variant of that of Figures 8a and 8b, with the layout of the inlet manifold (101) being a full or partial torus, and the outlet manifold (102) being a radial pipe.

Referring to Figure 8c, there is shown another suitable layout in which the inlet manifold (101) and the outlet manifold (102) run alongside each other over the wound bed in a boustrophedic pattern, i.e. in the manner of ploughed furrows.

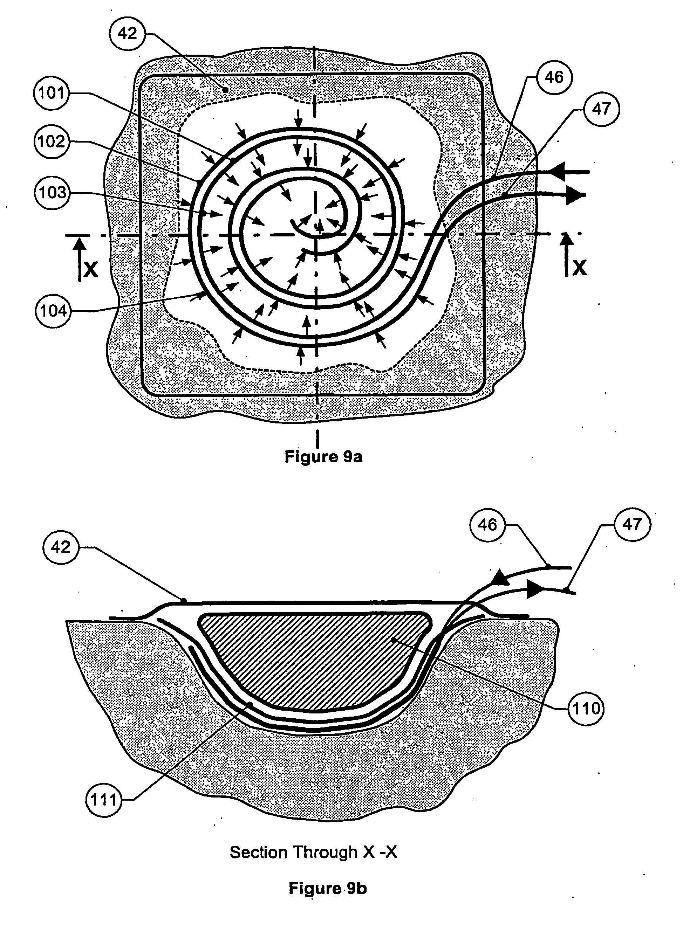

Referring to Figures 9a to 9d, there are shown other suitable layouts for deeper wounds, which are the same as shown in Figures 8a to 8d. The square backing layer (42) however has a wound filler (110) under, and may be permanently attached to, the backing layer (42), with an adhesive film (not shown) or by heat-sealing, which is an inverted hemispherical solid integer, here a resilient elastomeric foam, formed of a thermoplastic, preferably a cross-linked plastics foam.

Under the latter is a circular upwardly dished sheet (111) which conforms to, but is a separate structure, permanently unattached to, the solid filler (110). Through the sheet (111) pass the inlet pipe (46) and the outlet pipe (47), to run over the wound bed. These pipes (101), (102) again have a blind bore with orifices (103), (104) along the pipes (101), (102).

Alternatively (as in Figures 5a and 5b), where appropriate the dressing may be provided in a form in which the circular upwardly dished sheet (111) functions as the backing layer and the solid filler (110) sits on the sheet (42) as the backing layer, rather than under it. The filler (110) is held in place with an adhesive film or tape, instead of the backing layer (42).

In Figures 10a to 10c, inlet and outlet manifolds for the wound dressings for respectively delivering fluid to, and collecting fluid from, the wound, are formed by slots in and apertures through layers permanently attached to each other in a stack.

Thus, in Figure 10a there is shown an exploded isometric view of an inlet manifold and outlet manifold stack (120) of five square coterminous thermoplastic polymer layers, being first to fifth layers (121) to (125), each attached with an adhesive film (not shown) or by heat-sealing to the adjacent layer in the stack (120).

The topmost (first) layer (121) (which is the most distal in the dressing in use) is a blank square capping layer.

The next (second) layer (122), shown in Figure 10b out of the manifold stack (120), is a square layer, with an inlet manifold slot (126) through it. The slot (126) runs to one edge (127) of the layer (122) for connection to a mating end of a fluid inlet tube ((not shown), and spreads into four adjacent branches (128) in a parallel array with spaces therebetween.

The next (third) layer (123) is another square layer, with inlet manifold apertures (129) through the layer (123) in an array such that the apertures (129) are in register with the inlet manifold slot (126) through the second layer (122) (shown in Figure 10b).

The next (fourth) layer (124), shown in Figure 10c out of the manifold stack (120), is another square layer, with inlet manifold apertures (130) through the layer (124) in an array such that the apertures (130) are in register with the apertures (129) through the third layer (123).

It also has an outlet manifold slot (131) through it.

The slot (131) runs to one edge (132) of the layer (124) on the opposite side of the manifold stack (120) from the edge (127) of the layer (122), for connection to a mating end of a fluid outlet tube (not shown).

It spreads into three adjacent branches (133) in a parallel array in the spaces between the apertures (130) in the layer (124) and in register with the spaces between the apertures (129) in the layer (122).

The final (fifth) layer (125) is another square layer, with inlet manifold apertures (134) through the layer (125) in an array such that the apertures (134) are in register with the inlet manifold apertures (130) through the fourth layer (124) (in turn in register with the apertures (129) through the third layer (123). It also has outlet manifold apertures (135) in the layer (125) in an array such that the apertures (135) are in register with the outlet manifold slot (131) in the fourth layer (124).

It will be seen that, when the layers (121) to (125) are attached together to form the stack (120), the topmost (first) layer (121), the inlet manifold slot (126) through the second layer (122), and the third layer (123) cooperate to form an inlet manifold in the second layer (122), which is in use is connected to a mating end of a fluid inlet tube (not shown).

The inlet manifold slot (126) through the second layer (122), and the inlet manifold apertures (129), (130) and (134) through the layers (123), (124) and (125), all being mutually in register, cooperate to form inlet manifold conduits though the third to fifth layers (123), (124) and (125) between the inlet manifold in the second layer (122) and the proximal face (136) of the stack (120).

The third layer (121), the outlet manifold slot (131) through the fourth layer (124), and the fifth layer (125) cooperate to form an outlet manifold in the fourth layer (124), which is in use is connected to a mating end of a fluid outlet tube (not shown).

The outlet manifold slot (131) through the fourth layer (124), and the outlet manifold apertures (135) through the fifth layer (125), being mutually in register, cooperate to form outlet manifold conduits though the fifth layer (125) between the outlet manifold in the fourth layer (124) and the proximal face (136) of the stack (120).

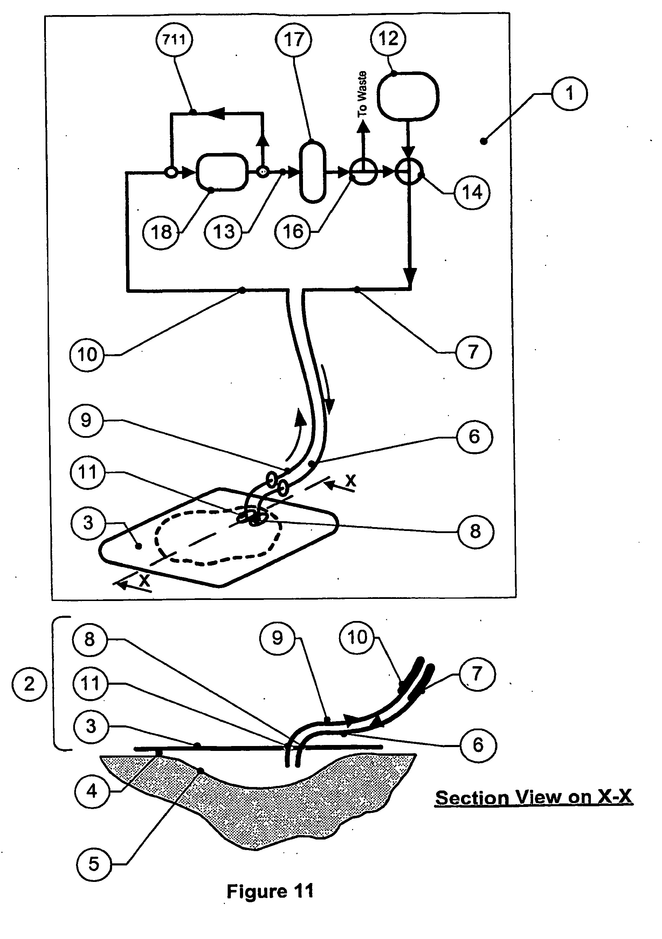

Referring to Figure 11, the apparatus (1) for aspirating, irrigating and/or cleansing wounds is a variant of the apparatus (1) of Figure 1.

It has bypass (711) around the pump (17), as a protection of the pump against any blockage in the system.

It is activated automatically by appropriate means, e.g. it is normally blocked by a bursting disc (not shown), or a pressure-activated motorised valve.

An alternative to the by-pass (711) is a pressure sensor in the system that will detect excessive load or pressure, and shut down the pump.

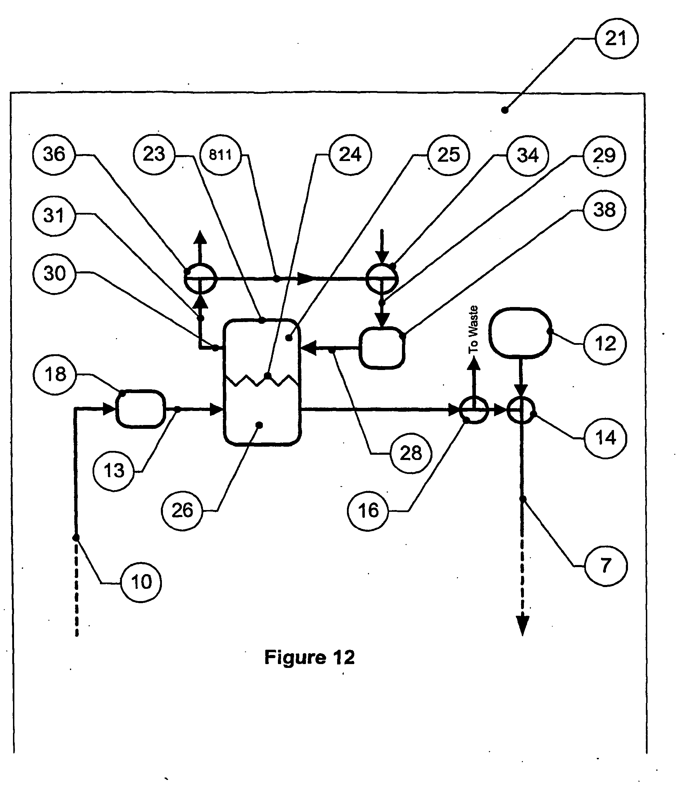

Referring to Figure 12, the apparatus (1) for aspirating, irrigating and/or cleansing wounds is a variant of the apparatus (1) of Figure 2.

The latter is a two-phase system with a dialysis unit (21), but is one in which dialytic fluid passes only once across the surface of the dialytic membrane (28) in the first chamber (25) from a dialysate reservoir (not shown) to waste via a second bleed T-valve (36) into, e.g. a collection bag (not shown).

This variant has a dialysate recirculation tube (811) running between a first T-valve (816) on the inlet side of the dialysate pump (23) and a second T-valve (817) to permit the pump (23) to recirculate the dialysate once the circuit is primed in multiple passes through the dialysis unit (21).

The operation of the system will be apparent to the skilled person.

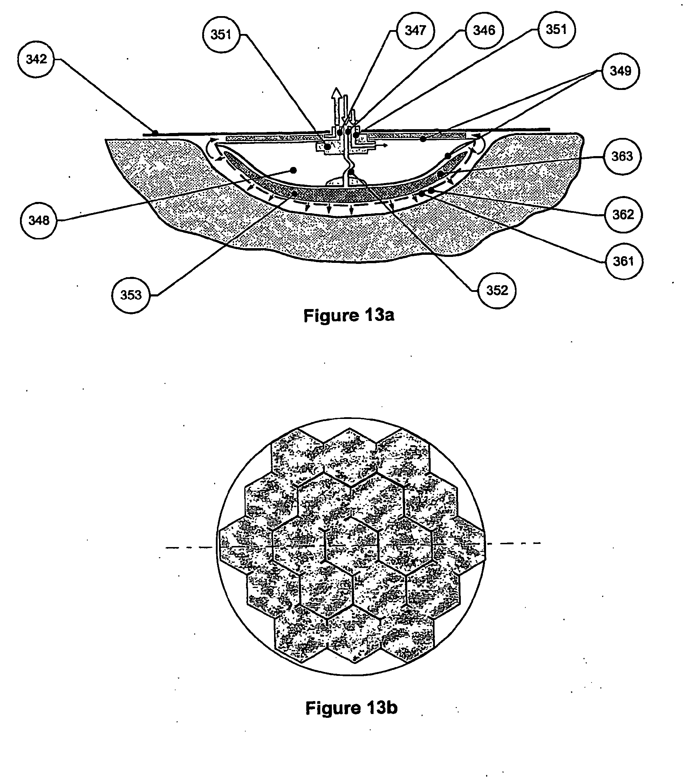

Referring to Figures 13 to 15, these forms of the dressing are provided with a wound filler (348) under a circular backing layer (342).

This comprises respectively a generally downwardly domed or toroidal, or oblately spheroidal conformable hollow body, defined by a membrane (349) which is filled with a fluid, here air or nitrogen, that urges it to the wound shape.

The filler (348) is permanently attached to the backing layer via a boss (351), which is e.g. heat-sealed to the backing layer (342).

An inflation inlet pipe (350), inlet pipe (346) and outlet pipe (347) are mounted centrally in the boss (351) in the backing layer (342) above the hollow body (348). The inflation inlet pipe (350) communicates with the interior of the hollow body (348), to permit inflation of the body (348). The inlet pipe (346) extends in a pipe (352) effectively through the hollow body (348). The outlet pipe (347) extends radially immediately under the backing layer (342).

In Figure 13, the pipe (352) communicates with an inlet manifold (353), formed by a membrane (361) with apertures (362) that is permanently attached to the filler (348) by heat-sealing. It is filled with foam (363) formed of a suitable material, e.g. a resilient thermoplastic.

Preferred materials include reticulated filtration polyurethane foams with small apertures or pores.

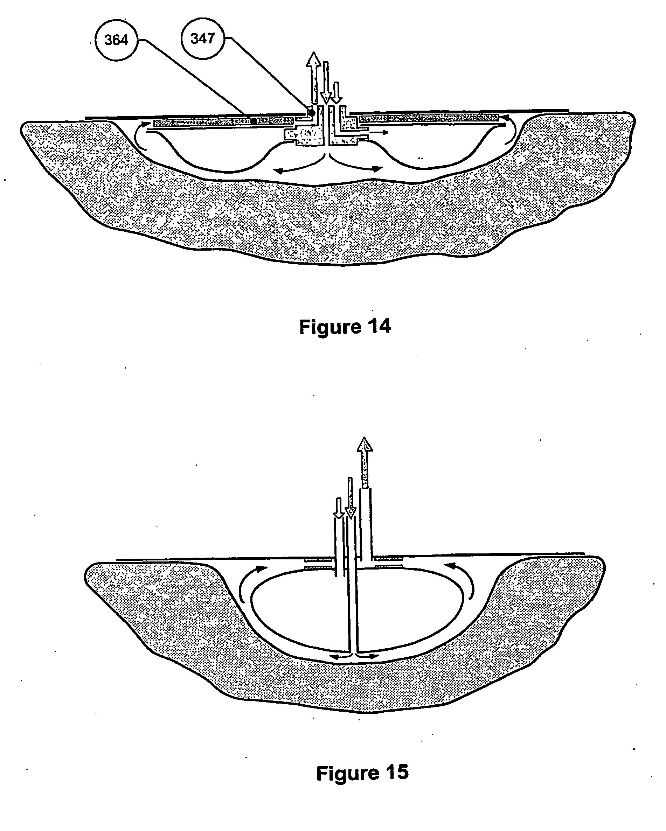

In Figure 14, the outlet pipe (347) communicates with a layer of foam (364) formed of a suitable material, e.g. a resilient thermoplastic. Again, preferred materials include reticulated filtration polyurethane foams with small apertures or pores.

In all of Figures 13, 14 and 15, in use, the pipe (346) ends in one or more openings that deliver the irrigant fluid directly from the wound bed over an extended area.

Similarly, the outlet pipe (347) effectively collects the fluid radially from the wound periphery when the dressing is in use.

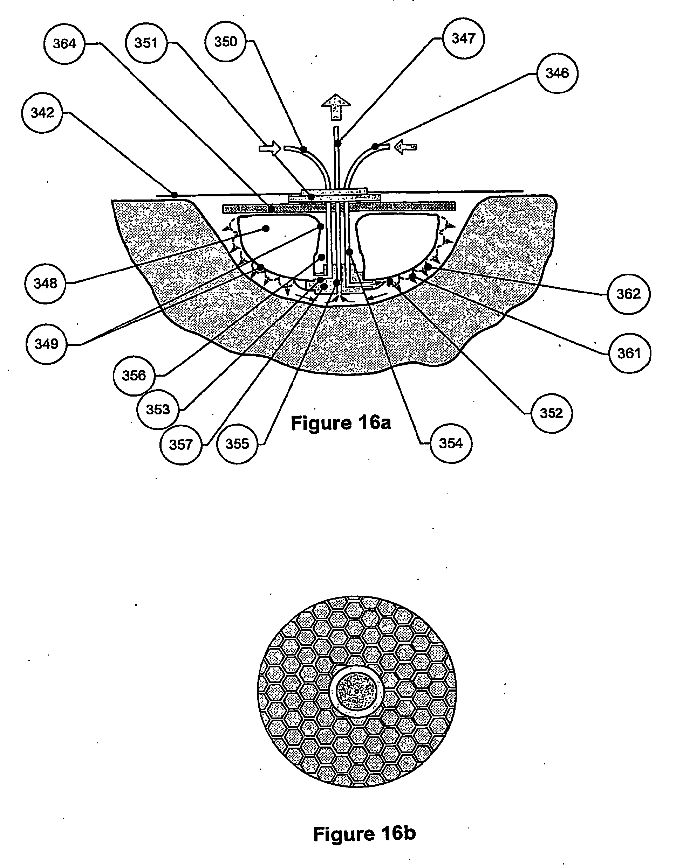

Referring to Figure 16, the dressing is also provided with a wound filler (348) under a circular backing layer (342).

This also comprises a generally toroidal conformable hollow body, defined by a membrane (349) which is filled with a fluid, here air or nitrogen, that urges it to the wound shape.

The filler (348) may be permanently attached to the backing layer (342) via a first boss (351) and a layer of foam (364) formed of a suitable material, e.g. a resilient thermoplastic. Again, preferred materials include reticulated filtration polyurethane foams with small apertures or pores.

The first boss (351) and foam layer (364) are respectively heat-sealed to the backing layer (342) and the boss (351).

An inflation inlet pipe (350), inlet pipe (346) and outlet pipe (347) are mounted centrally in the first boss (351) in the backing layer (342) above the toroidal hollow body (348).

The inflation inlet pipe (350), inlet pipe (346) and outlet pipe (347) respectively each extend in a pipe (353), (354) and (355) through a central tunnel (356) in the hollow body (348) to a second boss (357) attached to the toroidal hollow body (348).

The pipe (353) communicates with the interior of the hollow body (348), to permit inflation of the body (348). The pipe (354) extends radially through the second boss (357) to communicate with an inlet manifold (352), formed by a membrane (361) that is permanently attached to the filler (348) by heat-sealing in the form of a reticulated honeycomb with openings (362) that deliver the irrigant fluid directly to the wound bed over an extended area. The pipe (355) collects the fluid flowing radially from the wound centre when the dressing is in use.

This form of the dressing is a more suitable layout for deeper wounds

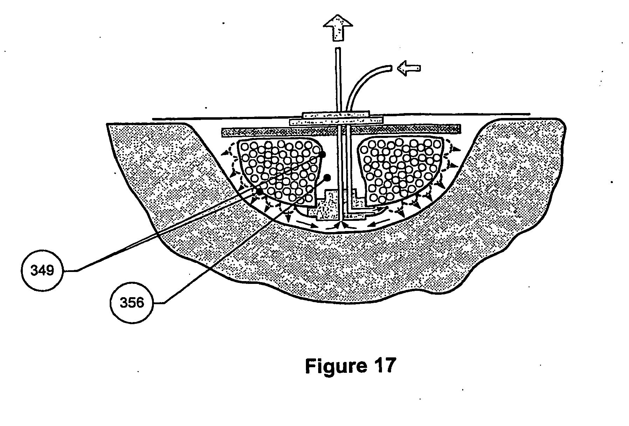

In Figure 17, the dressing is similar to that of Figure 16, except that the toroidal conformable hollow body, defined by a membrane (349), is filled with a fluid, here a solid particulates, such as plastics crumbs or beads, rather than a gas, such as air or an inert gas, such as nitrogen or argon, and the inflation inlet pipe (350) and pipe (353) are omitted from the central tunnel (356).

Examples of contents for the body (348) also include gels, such as silicone gels or preferably cellulosic gels, for example hydrophilic cross-linked cellulosic gels, such as Intrasite ™ cross-linked materials. Examples also include aerosol foams, and set aerosol foams, e.g. CaviCare™ foam.

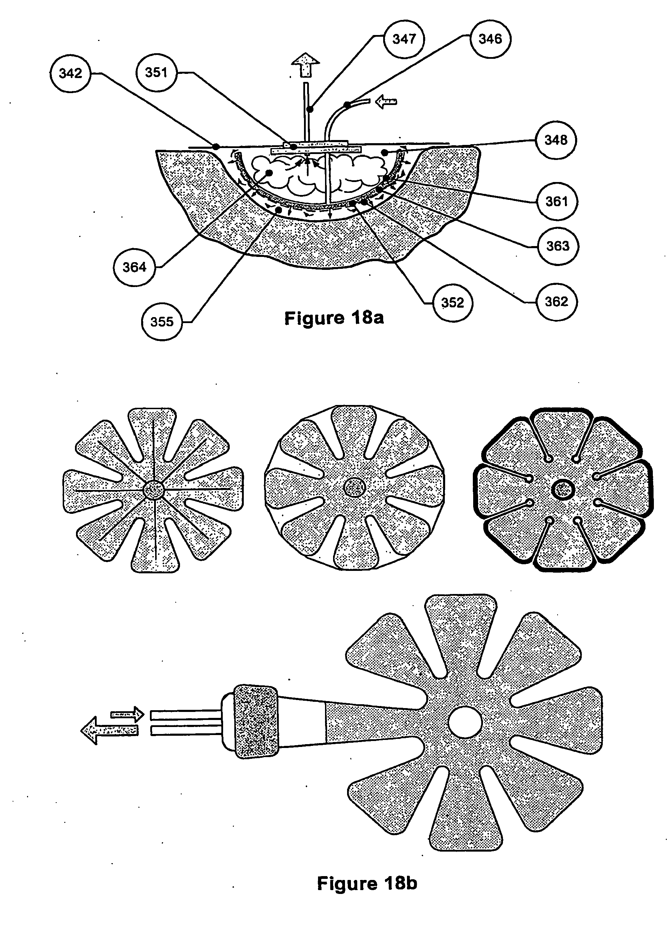

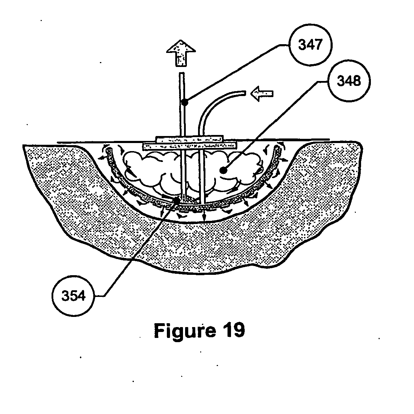

Referring to Figures 18 and 19, another form for deeper wounds is shown. This comprises a circular backing layer (342) and a chamber (363) in the form of a deeply indented disc much like a multiple Maltese cross or a stylised rose.

This is defined by an upper impervious membrane (361) and a lower porous film (362) with apertures (364) that deliver the irrigant fluid directly from the wound bed over an extended area.

A number of configurations of the chamber (363) are shown, all of which are able to conform well to the wound bed by the arms closing in and possibly overlapping in insertion into the wound.

In a particular design of the chamber (363), shown lowermost, on of the arms extended and provided with an inlet port at the end of the extended arm. This provides the opportunity for coupling and decoupling the irrigant supply remote from the dressing and the wound in use.

An inlet pipe (346) and outlet pipe (347) are mounted centrally in a boss (351) in the backing layer (342) above the chamber (363). The inlet pipe (346) is permanently attached to, and communicate with the interior of, the chamber (363), which thus effectively forms an inlet manifold. The space above the chamber (363) is filled with a loose gauze packing (364)..

In Figure 18, the outlet pipe (347) collects the fluid from the interior of the dressing from just under the wound-facing face (343) of the backing layer (342).

A variant of the dressing of Figure 18 is shown in Figure 19. The outlet pipe (347) is mounted to open at the lowest point of the space above the chamber (363) into a piece of foam (374).

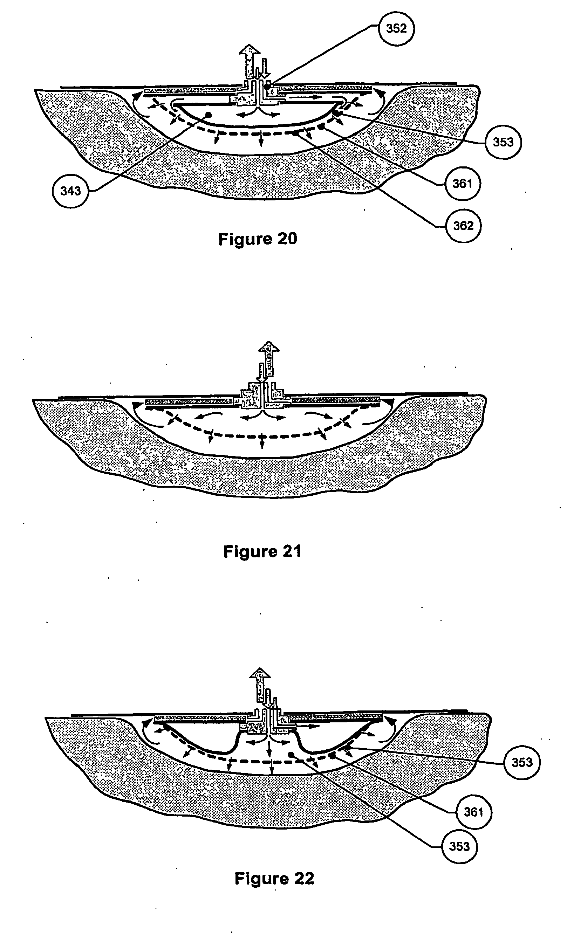

In Figure 20, the dressing is similar to that of Figure 13, except that the inlet pipe (352) communicates with an inlet manifold (353), formed by a membrane (361) with apertures (362), over the upper surface of the generally downwardly domed wound hollow filler (348), rather than through it.

In Figure 22, the dressing is similar to that of Figure 14, with the addition of an inlet manifold (353), formed by a membrane (361) with apertures (362), over the lower surface of the generally downwardly domed annular wound hollow filler.

In Figure 21, the generally downwardly domed annular wound hollow filler is omitted.

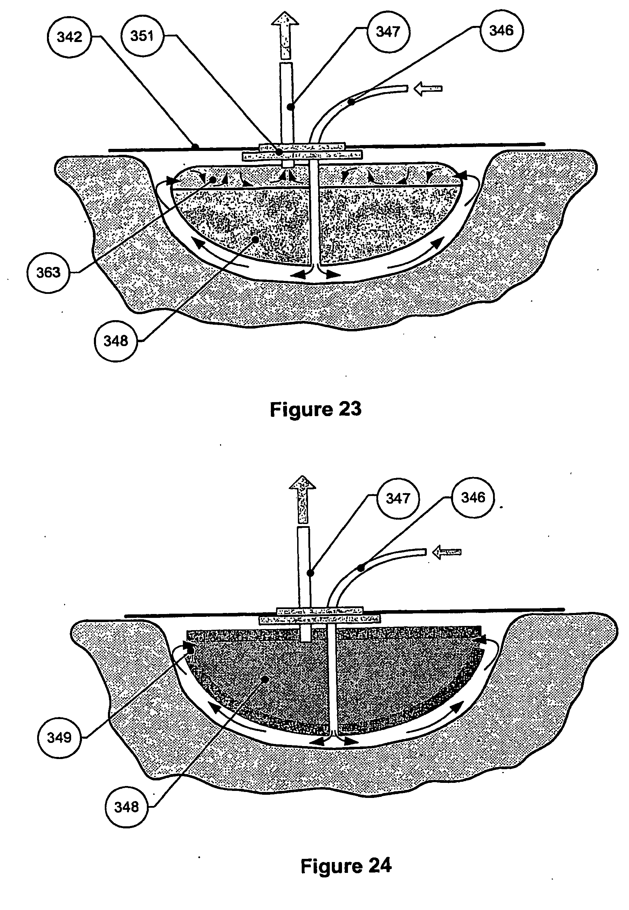

Referring to Figure 23, another form for deeper wounds is shown. An inlet pipe (346) and outlet pipe (347) are mounted centrally in a boss (351) in the backing layer (342) above a sealed-off foam filler (348). The inlet pipe (346) is permanently attached to and passes through the filler (348) to the wound bed. The outlet pipe (347) is attached to and communicates with the interior of, a chamber (363) defined by a porous foam attached to the upper periphery of the filler (348). The chamber (363) thus effectively forms an outlet manifold.

In Figure 24, the foam filler (348) is only partially sealed-off. The inlet pipe (346) is permanently attached to and passes through the filler (348) to the wound bed. The outlet pipe (347) is attached to and communicates with the interior of the foam of the filler (348). Fluid passes into an annular gap (349) near the upper periphery of the filler (348) into the foam, which thus effectively forms an outlet manifold.

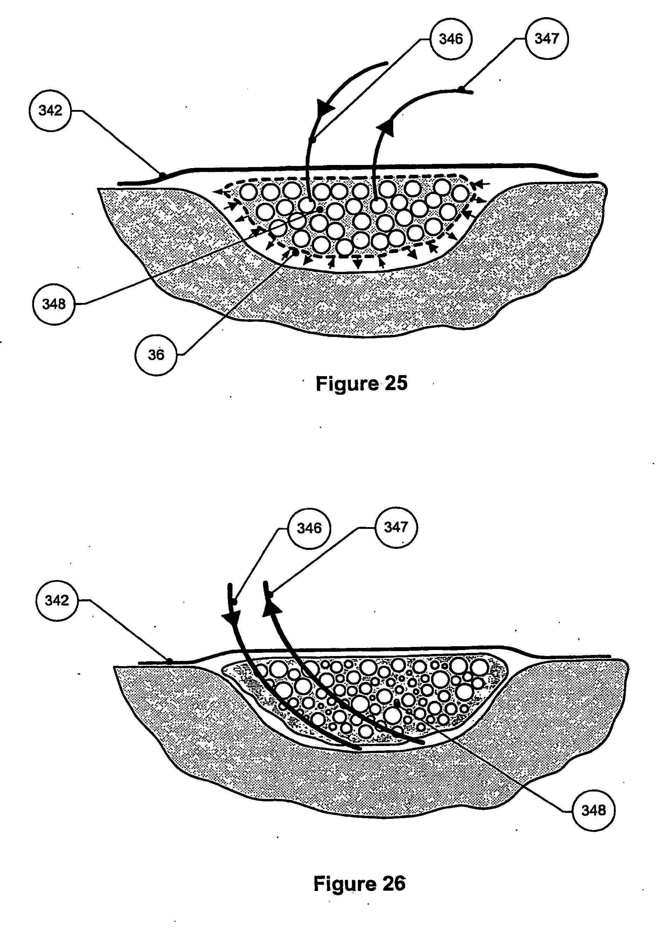

Figures 25 and 26 show dressings in which the inlet pipe (346) and outlet pipe (347) pass through the backing layer (342).

In Figure 25, they communicates with the interior of a porous bag filler (348) defined by a porous film (369) and filled with elastically resilient plastics bead or crumb.

In Figure 26, they communicate with the wound space just below a foam filler (348). The foam (348) may CaviCare ™ foam, injected and formed in situ around the pipes (346) and (347).

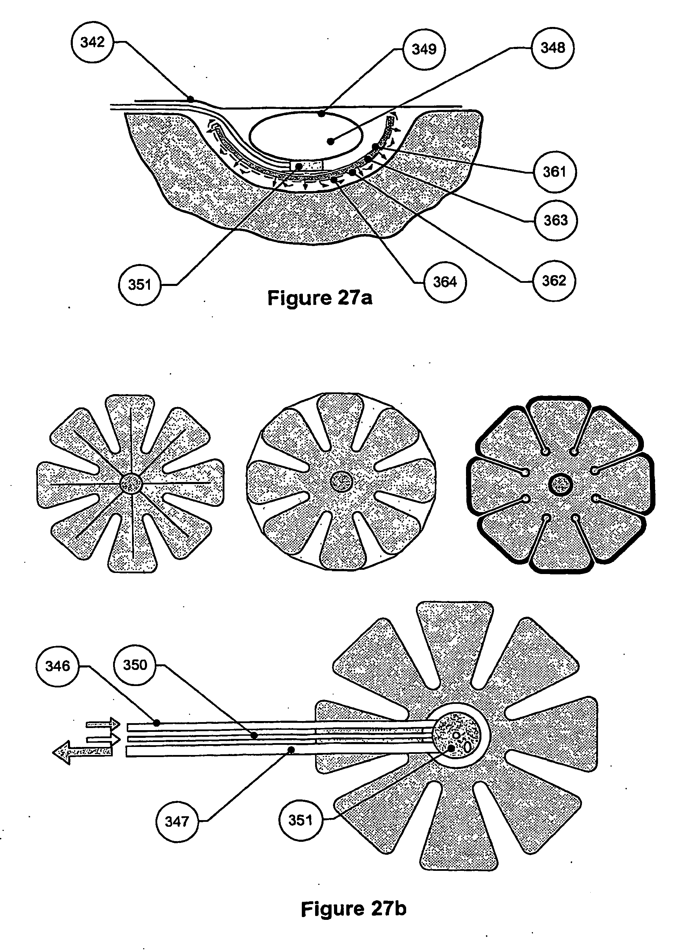

Referring to Figure 27, another form for deeper wounds is shown. This comprises a circular, or more usually square or rectangular backing layer (342) and a chamber (363) in the form of a deeply indented disc much like a multiple Maltese cross or a stylised rose.

This is defined by an upper impervious membrane (361) and a lower porous film (362) with apertures (364) that deliver the irrigant fluid directly to the wound bed over an extended area, and thus effectively forms an inlet manifold. Three configurations of the chamber (363) are shown in Figure 27b, all of which are able to conform well to the wound bed by the arms closing in and possibly overlapping in insertion into the wound.

The space above the chamber (363) is filled with a wound filler (348) under the backing layer (342). This comprises an oblately spheroidal conformable hollow body, defined by a membrane (349) that is filled with a fluid, here air or nitrogen, that urges it to the wound shape.

A moulded hat-shaped boss (351) is mounted centrally on the upper impervious membrane (361) of the chamber (363). It has three internal channels, conduits or passages through it (not shown), each with entry and exit apertures.

The filler (348) is attached to the membrane (361) of the chamber (363) by adhesive, heat welding or a mechanical fixator, such as a cooperating pin and socket.

An inflation inlet pipe (350), inlet pipe (346) and outlet pipe (347) pass under the edge of the proximal face of the backing layer (342) of the dressing, and extend radially immediately under the filler (348) and over the membrane (361) of the chamber (363) to each mate with an entry aperture in the boss (351).

An exit to the internal channel, conduit or passage through it that receives the inflation inlet pipe (350) communicates with the interior of the hollow filler (348), to permit inflation.

An exit to the internal channel, conduit or passage that receives the inlet pipe (346) communicates with the interior of the chamber (363) to deliver the irrigant fluid via the chamber (363) to the wound bed over an extended area.

Similarly, an exit to the internal channel, conduit or passage that receives the outlet pipe (347) communicates with the space above the chamber (363) and under the wound filler (348), and collects flow of irrigant and/or wound exudate radially from the wound periphery.

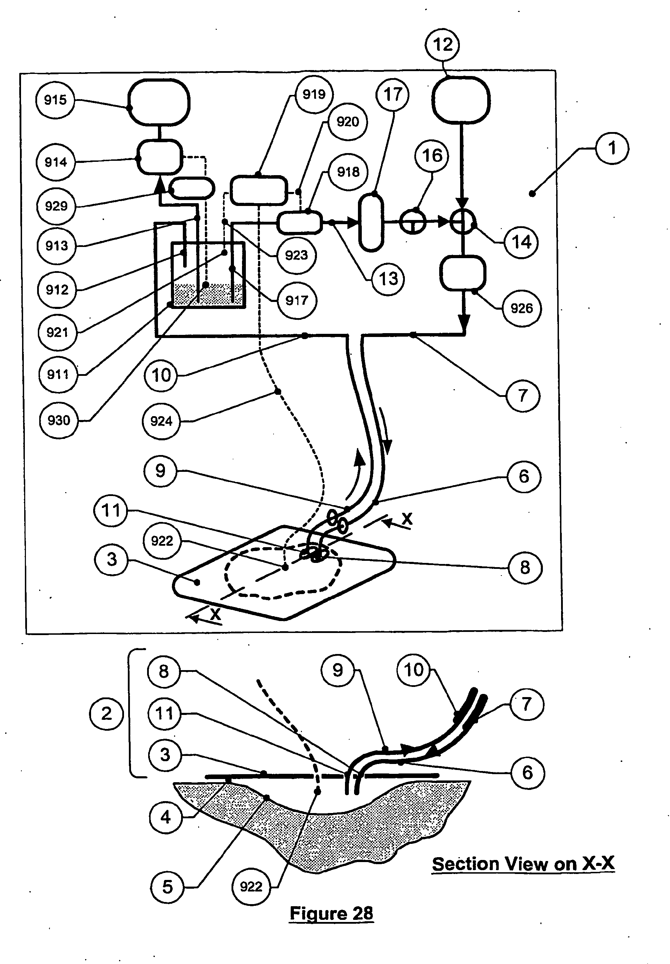

Referring to Figure 28, the apparatus (1) for aspirating, irrigating and/or cleansing using cells or tissue wounds is a major variant of the apparatus shown in Figure 1.

The device for moving fluid through the wound and means for fluid cleansing using cells or tissue (17) in Figure 1 is a peristaltic pump (18), e.g. preferably a small portable peristaltic pump, acting on the fluid circulation tube (13) downstream of the dressing (2) to apply a low negative pressure on the wound.

In the apparatus (1) shown in Figure 28, the peristaltic pump (18) is replaced by:
a) a peristaltic pump (926) acting on the fluid supply tube (7) upstream of the dressing (2), and
b) a vacuum pump assembly (918) with pressure regulating means, acting on the fluid circulation tube (13) downstream of the dressing (2),
to apply an overall low negative pressure in the wound space.

The vacuum pump assembly comprises a tank (911) with
an inlet tube (912) connecting to the fluid circulation tube (13) and communicating with the upper part of the tank (911),
a waste tube (913) connecting to a waste pump (914) with waste bag (915) and communicating with the lower part of the tank (911),
a pump tube (917) connecting to a vacuum pump (918) and communicating with the upper part of the tank (911), and connecting via the fluid circulation tube (13) to the means for cleansing using cells or tissue (17) and communicating with the lower part of the tank (911).

The vacuum pump (918) is controlled by a pressure feedback regulator (919) through an electrical line (920), the regulator receiving signals from a tank sensor (921) in the upper part of the tank (911), and a dressing sensor (922) in the wound space respectively via lines (923) and (924).

The waste pump (914) is controlled by a waste level feedback regulator (929) the regulator receiving signals from a tank sensor with electrical line (930) in the middle part of the tank (911).

The vacuum pump (918) either acts as a valve so that the pump tube 917 connecting to the vacuum pump (918) is normally blocked to prevent passage of air through it from the upper part of the tank (911) when the vacuum pump (918) is at rest, or the pump tube (917) is provided with a manual or motorised, e.g. pressure-activated motorised, valve (930) (not shown), so that the pump tube (917) connecting to the vacuum pump (918) may be blocked to prevent such passage.

The operation of the apparatus (1) is similar to that of the apparatus in Figure 1 mutatis mutandis.

In use of the apparatus (1), the valve (16) is opened to a collection bag (not shown), and the T- valve (14) is turned to admit fluid from the fluid reservoir to the wound dressing through the fluid supply tube (7) and inlet pipe (6).

The pump (926) is started to nip the fluid recirculation tube (7) with the peripheral rollers on its rotor (not shown) to apply a low positive pressure on the wound.

The vacuum pump (918) either acts as a valve since it is at rest, or the valve (930) (not shown) is closed, so that the pump tube 917 is blocked to prevent passage of air through it from the upper part of the tank (911).

Irrigant pumped from the wound dressing (2) through the fluid offtake tube (10) is pumped through the lower part of the tank (911) up the outlet tube (917) via the means for cleansing using cells or tissue (17) to the bleed T-valve (16) into, e.g. a collection bag (not shown).

The peristaltic pump (926) acting on the fluid supply tube (7) upstream of the dressing (2) is allowed to run until the apparatus is primed throughout the whole length of the apparatus flow path and excess fluid is voided to waste via the bleed T-valve (16) into the collection bag.

The T-valve (14) is then turned to switch from supply to recirculation, i.e. is set to close the wound to the fluid reservoir (part of the integer (12)) but to admit fluid into the wound from the fluid recirculation tube (13), and the bleed T-valve (16) is simultaneously closed.

The vacuum pump (918) is then activated, and, if the vacuum pump (918) does not act as a valve when at rest, the valve (930) in the pump tube 917 is opened, to apply a low negative pressure to the wound.

The circulating fluid from the wound and the fluid reservoir (part of the integer (12)) passes through the cleansing using cells or tissue unit (17). Materials deleterious to wound healing are removed and the cleansed fluid, still containing materials that are beneficial in promoting wound healing, is returned via the recirculation tube (13) to the wound bed.

The pressure feedback regulator (919) regulates the pressure at the wound and/or the tank (911).

If the amount of fluid in circulation becomes excessive, e.g. because the wound continues to exude heavily, the waste pump (914) may be started by the waste level feedback regulator (929) on the regulator receiving signals from the tank sensor with electrical line (930). The recirculation of fluid may be continued as long as desired.

The vacuum pump (918) is then deactivated, and, if the vacuum pump (918) does not act as a valve when at rest, the valve (930) in the pump tube (917) is closed, and the bleed T-valve (16) is opened to air to relieve the low negative pressure in the tank (911) via the means for cleansing using cells or tissue (17) and the outlet tube (917).

Switching between supply and recirculation is then reversed, by turning the T- valve (14) to admit fluid from the fluid reservoir to the wound dressing through the fluid supply tube (7) and inlet pipe (6).

The bleed valve (16) is left open, so that fresh fluid flushes the recirculating system. The running of the pump (918) may be continued until the apparatus is flushed, when it and the fluid recirculation is stopped.

### Example 1

### Method

### Cells

Human dermal fibroblasts (HS8/BS04) grown at 37°C/5% CO₂, in T175 flasks containing 35 ml DMEM /10% FCS media were washed in PBS and lifted using 1 x trypsin/EDTA (37°C for 5 min). Trypsin inhibition was achieved by adding 10 ml DMEM/10% FCS media and the cells were pelleted by centrifugation (Hereus Megafuge 1.0R; 1000 rpm for 5 min). The media was discarded and cells re-suspended in 10 ml DMEM/10% FCS. Cells were counted using a N haemocytometer (SOP/CB/007) and diluted in DMEM/10% FCS to obtain 100,000 cells per ml.

Cells (100 µl of diluted stock) were transferred to each 13mm Thermanox tissue culture coated cover slip (cat. 174950, lot 591430) in a 24 well plate and incubated for 1 hr at 37°C/5% CO₂ to allow cell adherence. After 1 h, 1 ml DMEM/10% FCS media was added per well. After 6 h, the media was removed, cells washed with 2 x 1 ml PBS and 1 ml DMEM/0%FCS added per well and the cells incubated overnight in the above conditions.

Following overnight incubation, cells were assessed visually for growth under the microscope and those with growth were inserted into cover slip holders (Vertriebs-Gmbh, cat no. 1300) for assembly in the Minucell chamber (Vertriebs-Gmbh, Cat no. 1301).

### Media

Cells were grown in DMEM media (Sigma, no. D6429) supplemented with 10% foetal calf serum; I-glutamine, non-essential amino acids and penicillin/streptomycin. Media used in the experimental systems was supplemented with 5% (v/v) foetal calf serum and buffered with 1% (v/v) Buffer-All media (Sigma, lot 51k2311) to ensure stable pH of the media.

### Minucell Flow systems

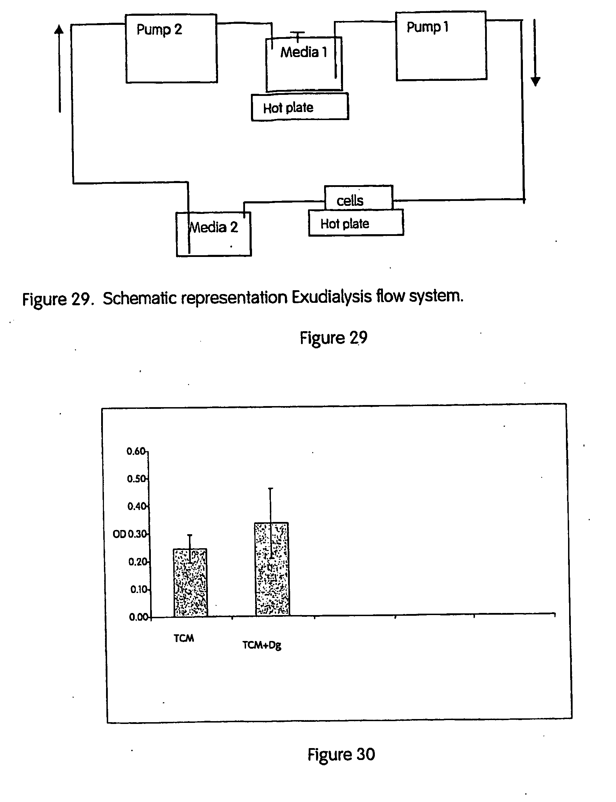

Systems (5) were made up as per figure 29.

| | Bottle 1 | Bottle 2 |
|---|---|---|
| System 1 | Media | Media. |
| System 2 | Media | Media and 6 Dermagraft squares |
| System 3 | Media + catalase | Media. |
| System 4 | Media + H₂O₂ | Media + Dermagraft |
| System 5 | Media +catalase + H₂O₂ | Media + Dermagraft |

Equipment used in the flow system was Ismatec IPC high precision peristaltic pumps with Ismatec pump tubing 1.02mm ID and high strength silicon tubing (HS-0152-009, Cole Palmer Instruments) and hot plates (asset number 6531 and 6532).

### Catalase

Snakeskin pleated dialysis tube (10kDa MWCO; Pierce, no. 68100, lot EB9446) containing 15 ml catalase (or 86200 units; Sigma, C3155, lot 014K7029). The dialysis tubing was placed in Media 1 bottle.

### H₂O₂

Hydrogen peroxide (Sigma, lot 074K3641; stock 8.8M, 30% soln) (250 µl) added to 21.75 ml DMEM/5% FCS media. 5.1ml of the media added to 39.9ml DMEM/5% FCS media and 5 ml of this was added to bottle 1 of the relevant systems giving a final concentration of 1.1 mM.

Hydrogen peroxide (H₂O₂) was used to mimic the chronic wound element, as it is a reactive oxygen species that causes oxidative stress to cells. The enzyme catalase is a natural antioxidant that degrades H₂O₂ into water and oxygen protecting cells against oxidative damage to proteins, lipids and nucleic acids. So, it was placed in dialysis tubing to mimic exudialysis. A source of cells as a source of actives was provided by using Vicryl mesh seeded with live fibroblast cells [Dermagraft]. The experiment ran for a total of 48 hours. A WST assay was used to measure fibroblast activity after 48 hours.

### Cells as a source of actives

The 'cells as a source of actives' was fibroblasts seeded on a Vicryl mesh (Dermagraft, Smith and Nephew). Dermagraft was defrosted in a water bath at 37°C for 1 minute and the cryoprotectant removed. The Dermagraft was washed with 3 x 50ml 0.9% saline and cut into 1.1cm squares using the clickerpress. 6 squares of Dermagraft were placed in bottle 2 of the systems described above.

*The final volume of media was made up to 50 ml in bottle 1 and bottle 2.*

### WST Assay

A WST assay to measure the cells mitochondrial activity was performed on 6 coverslips from each system. WST reagent (Roche, lot 102452000) was diluted to 10% v/v in DMEM/10% FCS/buffer all media. The coverslips were removed from the Minucell chamber and washed in 1 ml PBS. PBS was removed and 200 µl WST/DMEM media added. The coverslips were then incubated at 37°C for 45 min before transferring 150 µl of reagent to a 96 well plate. The absorbance at 450 nm with reference at 655 nm was determined using Ascent Multiskan Microtitre plate reader.

### Results and discussion

The mitochondrial activity of cells grown in exudialysis systems, with or without 'cells as actives' component was determined using the WST assay.

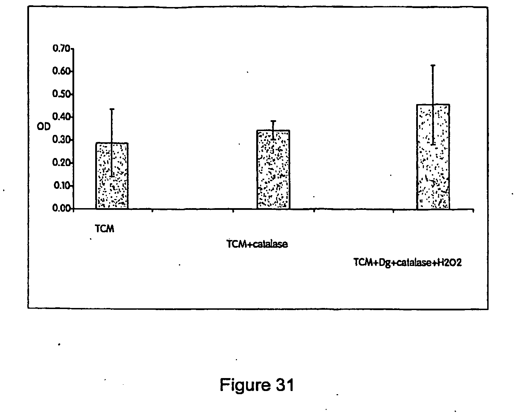

The WST activity of individual experiments is shown in figures 30 and 31, with the average WST activity represented by the bar and standard deviation by the error bars.

From the data in Figure 30 it is possible to see that the addition of Dermagraft (Dg) (the source of actives from live cells) resulted in an increased fibroblast activity, as measured by the WST assay.

Fibroblast activity within the Dermagraft squares was shown by assaying a number of Dermagraft squares from the media at the end of the experimental incubation period. Dermagraft activity was in the range 0.17 to 0.95 and was therefore alive.

From the data in Figure 31 it is possible to see that with the addition of exudialysis (+ catalase) resulted in an increased fibroblast activity over the control of media only (TCM).

This graph (Figure 31) also showed that the presence of H₂O₂ Hydrogen Peroxide even with Dermagraft (Dg) had no fibroblast activity. Thus in the absence of a removal system hydrogen peroxide, a source of toxic reactive oxygen species, kills the fibroblasts seeded on the coverslip and in the Dermagraft.

In contrast the data shows for, the actives from live cells (Dg) with Exudialysis ( + catalase) even with Hydrogen Peroxide (H₂O₂) a significant increase in fibroblast activity, as measured by the WST assay over the media only control, the hydrogen peroxide control and media and exudialysis result.

This increase was also greater than with media and actives from live cells (Figure 30).

### Conclusions

It is possible to see an increase fibroblast growth activity when the cells are in the flow system in conjunction with the live cells providing a source of actives along with the exudialysis system which removes the 'chrome wound element' from the media.

## Claims

1. An apparatus (1;21) for aspirating, irrigating and/or cleansing wounds, which comprises a fluid flowpath, comprising
i) a wound dressing (2), having a backing layer (3) and
at least one inlet pipe (16) for connection to a fluid supply tube (7), which passes through and/or under the backing layer and at least one outlet pipe (9) for connection to a fluid offtake tube (10), which passes through and/or under the backing layer,
at least one inlet pipe being connected to a fluid recirculation tube (13), and at least one outlet pipe being connected to a fluid offtake tube; and
ii) a means for fluid cleansing (17) having at least one inlet port connected to a fluid offtake tube and at least one outlet port connected to a fluid recirculation tube;
iii) a device for moving fluid (18;926;918) through the wound dressing and the means for fluid cleansing (17); and **characterised in that** the apparatus further comprises means for supplying physiologically active agents from cells or tissue to the wound, wherein said means comprises
(a) an irrigant reservoir connected to;
(b) a container that contains a cell or tissue component, in turn connected to;
(c) a supply tube in the flowpath.
such that fluid may be supplied to fill the flowpath and supply physiologically active agents from cells or tissue to the wound and recirculated by the device through the flow path.

2. An apparatus as claimed in claim 1, wherein the device for moving fluid through the wound dressing and the means for fluid cleansing, also moves fluid through the fluid supply tube.

3. An apparatus as claimed in claim 1 or claim 2, wherein the apparatus
further comprises a means for bleeding (15) the flowpath.

4. An apparatus as claimed in any preceding claims in which the backing layer is capable of forming a relatively fluid tight seal or closure over a wound.

5. An apparatus as claimed in any preceding claims in which the point (8;11) at which the/or each inlet pipe and the/or each outlet pipe passes through and/or under the backing layer is capable of forming a relatively fluid-tight seal or closure over the wound.

6. An apparatus as claimed in any preceding claims in which the wound dressing is a conformable wound dressing.

7. An apparatus as claimed in any one of the preceding claims in which the backing layer is semi permeable to allow a flow rate of gas through it.

8. An apparatus as claimed in any one of the preceding claims in which the apparatus comprises a wound contact layer.

9. An apparatus as claimed in claim 8 in which the wound contact layer is chosen from the group consisting of gauze, foam, a porous means, a semi-permeable porous means, an elastic filler or an inflatable device.

10. An apparatus as claimed in claim 1 in which the cell or tissue component is mounted under the backing layer.

11. An apparatus as claimed in any one of the preceding claims in which the apparatus is portable.

12. An apparatus as claimed in either one of claims 1 or 10 in which the cell or tissue component is bound on an insoluble and/or immobilised substrate.

13. An apparatus according to claim 2 comprising
a) a first device (918) for moving fluid through the wound applied to fluid downstream of and away from the wound dressing, and
b) a second device (926) for moving fluid through the wound applied to the irrigant in the fluid supply tube upstream of and towards the wound dressing.

14. An apparatus according to claim 13 in which the first device and/or second device is a fixed throughput device and the means for providing aspiration and irrigation of the wound also comprises at least one of: means for supply flow regulation (14), connected to a fluid supply tube, and means for aspirate flow regulation (16), connected to a fluid offtake tube.

15. An apparatus as claimed in claim 13 in which the irrigant fluid may be supplied to fill the flowpath from a fluid reservoir via the fluid supply tube while aspirate fluid is aspirated by a device through the fluid offtake tube.

16. An apparatus as claimed in any one of claims 13-15 in which the aspiration and irrigation of the wound is sequentially or simultaneously performed.

17. The apparatus of any preceding claim wherein the device for moving fluid through the wound is a diaphragm pump or a peristaltic pump (18).

18. The apparatus of any preceding claim in which the flow rate is a varied flow rate, either randomly or regularly cyclical.

19. The apparatus of claim 18 wherein the regular or random cycles of flow rate have a frequency of up to 48 per 24 hours.

20. The apparatus of claim 18 or 19 wherein the pulses of flow velocity have a frequency of from 1 to 60 per min.

21. The apparatus of any preceding claim wherein the device for moving fluid across the wound enables the fluid flow to be a parallel flow, radial streaming, spiral streaming, helical streaming, spirohelical streaming or circular streaming.

22. An apparatus according to any one of claims 13-16 in which the first device is also a vacuum means for creating a negative pressure on the area surrounding the wound.

23. An apparatus according to claim 22 in which the negative pressure is between about 1.01 and 100.3 kPa (0.01 and 0.99 atmospheres).

24. An apparatus according to claim 1, which comprises a means for fluid cleansing that is a single-phase system (17), in which the circulating fluid from the wound passes through the means for fluid cleansing and materials deleterious to wound healing are removed, without the circulating fluid coming into direct or indirect contact with another fluid in the means for fluid cleansing.

25. An apparatus according to claim 1, which comprises a means for fluid cleansing that is a two-phase system, in which the circulating fluid from the wound passes through the means for fluid cleansing and materials deleterious to wound healing are removed, by the circulating fluid coming into direct or indirect contact with another fluid in the means for fluid cleansing.

26. An apparatus according to claim 24, in which the means for fluid cleansing, the circulating fluid from the wound and the other fluid in the means for fluid cleansing are separated by an integer which is selectively permeable to materials deleterious to wound healing.

27. An apparatus according to claim 25, in which the means for fluid cleansing, the circulating fluid from the wound and the other fluid in the means for fluid cleansing are separated by an integer which is not selectively permeable to materials deleterious to wound healing, and the other fluid comprises and/or is in contact with a material that removes materials deleterious to wound healing.

28. An apparatus according to claim 25, in which the material that removes materials deleterious to wound healing is an antagonist, a binder and/or degrader, a chelator and/or ion exchanger for such deleterious materials, or an anti-oxidant.

29. An apparatus according to claim 25, in which the material that removes materials deleterious to wound healing is 4-(2-aminoethyl)-benzene sulphonyl fluoride (AEBSF, PefaBloc), Nα-ρ-tosyl-L-lysine chloromethyl ketone (TLCK), ε-aminocaproyl-ρ-chlorobenzylamide; a cysteine protease inhibitor; a matrix metalloprotease inhibitor; a carboxyl (acid) protease inhibitor; anti-inflammatory peptidomimetics; 3-hydroxytramine (dopamine), ascorbic acid (vitamin C), vitamin E; glutathione; desferrioxamine (DFO) and/or 3-hydroxytyramine (dopamine).

30. An apparatus according to claim 1, in which the materials deleterious to wound healing are oxidants; proteases; endotoxins; autoinducer signalling molecules; inhibitors of angiogenesis; pro-inflammatory cytokines; and inflammatories.

31. An apparatus according to any preceding claim which administers a reduced pressure treatment to the wound.

## Patentansprüche

1. Eine Vorrichtung (1; 21) zum Aspirieren, Spülen und/oder Reinigen von Wunden, die Folgendes beinhaltet:
einen Fluiddurchflussweg, der Folgendes beinhaltet:
i) einen Wundverband (2), der eine Unterschicht (3) und
mindestens ein Einlassrohr (16) zur Verbindung mit einer Fluidversorgungsröhre (7), das durch die und/oder unter der Unterschicht verläuft, und mindestens ein Auslassrohr (9) zur Verbindung mit einer Fluidableitungsröhre (10), das durch die und/oder unter der Unterschicht verläuft, aufweist,
wobei mindestens ein Einlassrohr mit einer Fluidumwälzungsröhre (13) verbunden ist und mindestens ein Auslassrohr mit einer Fluidableitungsröhre verbunden ist; und
ii) ein Mittel zum Reinigen von Fluid (17), das mindestens eine mit einer Fluidableitungsröhre verbundene Einlassöffnung und mindestens eine mit einer Fluidumwälzungsröhre verbundene Auslassöffnung aufweist;
iii) eine Einrichtung zum Bewegen von Fluid (18; 926; 918) durch den Wundverband und das Mittel zum Reinigen von Fluid (17), und **dadurch gekennzeichnet, dass** die Vorrichtung ferner ein Mittel zum Liefern von physiologisch aktiven Wirkstoffen von Zellen oder Gewebe zu der Wunde beinhaltet, wobei das Mittel Folgendes beinhaltet:
(a) einen Spülungsbehälter, verbunden mit
(b) einem Behältnis, das eine Zell- oder Gewebekomponente enthält, wiederum verbunden mit
(c) einer Versorgungsröhre in dem Durchflussweg,
so dass Fluid geliefert werden kann, um den Durchflussweg zu füllen und physiologisch aktive Wirkstoffe von Zellen oder Gewebe an die Wunde zu liefern, und durch die Einrichtung durch den Durchflussweg umgewälzt werden kann.

2. Vorrichtung gemäß Anspruch 1, wobei die Einrichtung zum Bewegen von Fluid durch den Wundverband und das Mittel zum Reinigen von Fluid ebenfalls Fluid durch die Fluidversorgungsröhre bewegt.

3. Vorrichtung gemäß Anspruch 1 oder Anspruch 2, wobei die Vorrichtung ferner ein Mittel zum Ablassen (15) des Durchflusswegs beinhaltet.

4. Vorrichtung gemäß einem der vorhergehenden Ansprüche, bei der die Unterschicht zum Bilden einer relativ fluiddichten Abdichtung oder Abschließung über einer Wunde fähig ist.

5. Vorrichtung gemäß einem der vorhergehenden Ansprüche, bei der der Punkt (8; 11), an dem das/oder jedes Einlassrohr und das/oder jedes Auslassrohr durch und/oder unter der Unterschicht verläuft, zum Bilden einer relativ fluiddichten Abdichtung oder Abschließung über der Wunde fähig ist.

6. Vorrichtung gemäß einem der vorhergehenden Ansprüche, bei der der Wundverband ein konformer Wundverband ist.

7. Vorrichtung gemäß einem der vorhergehenden Ansprüche, bei der die Unterschicht halbdurchlässig ist, um eine Durchflussrate von Gas durch sie zu ermöglichen.

8. Vorrichtung gemäß einem der vorhergehenden Ansprüche, bei der die Vorrichtung eine Wundkontaktschicht beinhaltet.

9. Vorrichtung gemäß Anspruch 8, bei der die Wundkontaktschicht aus der Gruppe ausgewählt ist, die aus Folgendem besteht: Gaze, Schaum, einem porösen Mittel, einem halbdurchlässigen porösen Mittel, einem elastischen Füllstoff oder einer aufblasbaren Einrichtung.

10. Vorrichtung gemäß Anspruch 1, bei der die Zell- oder Gewebekomponente unter der Unterschicht befestigt ist.

11. Vorrichtung gemäß einem der vorhergehenden Ansprüche, bei der die Vorrichtung tragbar ist.

12. Vorrichtung gemäß einem der Ansprüche 1 oder 10, bei der die Zell- oder Gewebekomponente auf einem unlöslichen und/oder immobilisierten Substrat gebunden ist.

13. Vorrichtung gemäß Anspruch 2, die Folgendes beinhaltet:
a) eine erste Einrichtung (918) zum Bewegen von Fluid durch die Wunde, angewandt auf Fluid stromabwärts des Wundverbands und von diesem weg, und
b) eine zweite Einrichtung (926) zum Bewegen von Fluid durch die Wunde, angewandt auf die Spülung in der Fluidversorgungsröhre stromaufwärts des Wundverbands und zu diesem hin.

14. Vorrichtung gemäß Anspruch 13, bei der die erste Einrichtung und/oder zweite Einrichtung eine Einrichtung mit festgelegtem Durchsatz ist und das Mittel zum Bereitstellen von Aspiration und Spülung der Wunde ebenfalls mindestens eines von Folgendem beinhaltet: ein Mittel zur Versorgungsdurchflussregulierung (14), verbunden mit einer Fluidversorgungsröhre, und ein Mittel zur Aspirationsdurchflussregulierung (16), verbunden mit einer Fluidableitungsröhre.

15. Vorrichtung gemäß Anspruch 13, bei der das Spülungsfluid geliefert werden kann, um den Durchflussweg von einem Fluidbehälter über die Fluidversorgungsröhre zu füllen, während Aspirationsfluid von einer Einrichtung durch die Fluidableitungsröhre aspiriert wird.

16. Vorrichtung gemäß einem der Ansprüche 13-15, bei der die Aspiration und Spülung der Wunde sequentiell oder simultan durchgeführt wird.

17. Vorrichtung gemäß einem der vorhergehenden Ansprüche, wobei die Einrichtung zum Bewegen von Fluid durch die Wunde eine Diaphragmapumpe oder eine peristaltische Pumpe (18) ist.

18. Vorrichtung gemäß einem der vorhergehenden Ansprüche, bei der die Durchflussrate eine variierte Durchflussrate ist, entweder wahllos oder regelmäßig zyklisch.

19. Vorrichtung gemäß Anspruch 18, wobei die regelmäßigen oder wahllosen Zyklen der Durchflussrate eine Frequenz von bis zu 48 pro 24 Stunden aufweisen.

20. Vorrichtung gemäß Anspruch 18 oder 19, wobei die Impulse der Durchflussgeschwindigkeit eine Frequenz von 1 bis 60 pro Minute aufweisen.

21. Vorrichtung gemäß einem der vorhergehenden Ansprüche, wobei die Einrichtung zum Bewegen von Fluid über die Wunde erlaubt, dass der Fluiddurchfluss ein paralleler Durchfluss, radiales Strömen, spiralförmiges Strömen, gewundenes Strömen, schraubenförmig gewundenes Strömen oder zirkulares Strömen ist.

22. Vorrichtung gemäß einem der Ansprüche 13-16, bei der die erste Einrichtung ebenfalls ein Vakuummittel zum Schaffen eines Unterdrucks auf dem die Wunde umgebenden Bereich ist.

23. Vorrichtung gemäß Anspruch 22, bei der der Unterdruck zwischen etwa 1,01 und 100,3 kPa (0,01 und 0,99 Atmosphären) ist.

24. Vorrichtung gemäß Anspruch 1, die ein Mittel zum Reinigen von Fluid beinhaltet, das ein Einphasensystem (17) ist, bei der das zirkulierende Fluid aus der Wunde durch das Mittel zum Reinigen von Fluid verläuft und Materialien, die schädlich für die Wundheilung sind, entfernt werden, ohne dass das zirkulierende Fluid in dem Mittel zum Reinigen von Fluid mit einem anderen Fluid in direkten oder indirekten Kontakt kommt.

25. Vorrichtung gemäß Anspruch 1, die ein Mittel zum Reinigen von Fluid beinhaltet, das ein Zweiphasensystem ist, bei der das zirkulierende Fluid aus der Wunde durch das Mittel zum Reinigen von Fluid verläuft und Materialien, die schädlich für die Wundheilung sind, entfernt werden, indem das zirkulierende Fluid in dem Mittel zum Reinigen von Fluid mit einem anderen Fluid in direkten oder indirekten Kontakt kommt.

26. Vorrichtung gemäß Anspruch 24, bei der in dem Mittel zum Reinigen von Fluid das zirkulierende Fluid aus der Wunde und das andere Fluid in dem Mittel zum Reinigen von Fluid durch ein Element, das wahlweise Materialien, die schädlich für die Wundheilung sind, gegenüber durchlässig ist, getrennt werden.

27. Vorrichtung gemäß Anspruch 25, bei der in dem Mittel zum Reinigen von Fluid, das zirkulierende Fluid aus der Wunde und das andere Fluid in dem Mittel zum Reinigen von Fluid durch ein Element, das für die Wundheilung schädlichen Materialien gegenüber wahlweise nicht durchlässig ist, getrennt werden, und das andere Fluid ein Material, das für die Wundheilung schädliche Materialien entfernt, beinhaltet und/oder mit diesem in Kontakt steht.

28. Vorrichtung gemäß Anspruch 25, bei der das Material, welches für die Wundheilung schädliche Materialien entfernt, ein Antagonist, ein Bindemittel und/oder ein Abbaumittel, eine chelatbildende Verbindung und/oder ein lonenaustauscher für derartige schädliche Materialien oder ein Antioxidationsmittel ist.

29. Vorrichtung gemäß Anspruch 25, bei der das Material, welches für die Wundheilung schädliche Materialien entfernt, 4-(2-Aminoethyl)-benzensulfonylfluorid (AEBSF, PefaBloc), Nα-p-Tosyl-L-lysinchlormethylketon (TLCK), ε-Aminocaproyl-p-chlorbenzylamid; ein Cysteinprotease-Hemmstoff; ein Matrix-Metallprotease-Hemmstoff; ein Carboxyl(säure)protease-Hemmstoff; entzündungshemmende Peptidomimetika; 3-Hydroxytramin (Dopamin), Ascorbinsäure (Vitamin C), Vitamin E; Glutathion; Desferrioxamin (DFO) und/oder 3-Hydroxytyramin (Dopamin) ist.

30. Vorrichtung gemäß Anspruch 1, bei der die für die Wundheilung schädlichen Materialien Oxidationsmittel, Proteasen, Endotoxine, Autoinduktor-Signalmoleküle, Angiogenese-Hemmstoffe, proinflammatorische Zytokine und Inflammatorika sind.

31. Vorrichtung gemäß einem der vorhergehenden Ansprüche, die eine Behandlung reduzierten Drucks an die Wunde verabreicht.

## Revendications

1. Un appareil (1 ; 21) pour aspirer, irriguer et / ou nettoyer des plaies, lequel comprend une voie d'écoulement de fluide, comprenant
i) un pansement pour plaie (2), possédant une couche de support (3) et
au moins un tuyau d'entrée (16) destiné à se raccorder à un tube d'apport de fluide (7), lequel passe à travers et / ou sous la couche de support et au moins un tuyau de sortie (9) destiné à se raccorder à un tube d'évacuation de fluide (10), lequel passe à travers et / ou sous la couche de support,
au moins un tuyau d'entrée étant raccordé à un tube de recirculation de fluide (13), et au moins un tuyau de sortie étant raccordé à un tube d'évacuation de fluide ; et
ii) un dispositif de nettoyage de fluide (17) possédant au moins un orifice d'entrée raccordé à un tube d'évacuation de fluide et au moins un orifice de sortie raccordé à un tube de recirculation de fluide ;
iii) un dispositif de déplacement de fluide (18 ; 926 ; 918) à travers le pansement pour plaie et le moyen de nettoyage de fluide (17) ; et **caractérisé en ce que** l'appareil comprend en outre un moyen pour apporter des agents physiologiquement actifs provenant de cellules ou de tissu à la plaie, ledit moyen comprenant
(a) un réservoir d'irriguant raccordé à ;
(b) un conteneur qui contient un composant cellulaire ou tissulaire, raccordé à son tour à ;
(c) un tube d'apport dans la voie d'écoulement,
de façon à ce que du fluide puisse être apporté pour remplir la voie d'écoulement et apporter des agents physiologiquement actifs provenant de cellules ou de tissu à la plaie et recirculé par le dispositif dans la voie d'écoulement.

2. Un appareil tel que revendiqué dans la revendication 1, dans lequel le dispositif de déplacement de fluide à travers le pansement pour plaie et le moyen de nettoyage de fluide déplace aussi du fluide dans le tube d'alimentation en fluide.

3. Un appareil tel que revendiqué dans la revendication 1 ou la revendication 2, dans lequel l'appareil comprend en outre un moyen de purge (15) de la voie d'écoulement.

4. Un appareil tel que revendiqué dans n'importe quelles revendications précédentes dans lequel la couche de support est capable de former un scellement ou une obturation relativement étanche à du fluide par-dessus une plaie.

5. Un appareil tel que revendiqué dans n'importe quelles revendications précédentes dans lequel le point (8 ; 11) auquel le / chaque tuyau d'entrée et le / chaque tuyau de sortie passe à travers et / ou sous la couche de support est capable de former un scellement ou une obturation relativement étanche à du fluide par-dessus la plaie.

6. Un appareil tel que revendiqué dans n'importe quelles revendications précédentes dans lequel le pansement pour plaie est un pansement pour plaie adaptable.

7. Un appareil tel que revendiqué dans l'une quelconque des revendications précédentes dans lequel la couche de support est semi-perméable afin de permettre un régime d'écoulement de gaz à travers celle-ci.

8. Un appareil tel que revendiqué dans l'une quelconque des revendications précédentes dans lequel l'appareil comprend une couche de contact avec la plaie.

9. Un appareil tel que revendiqué dans la revendication 8 dans lequel la couche de contact avec la plaie est choisie dans le groupe consistant en gaze, mousse, un moyen poreux, un moyen poreux semi-perméable, un produit de remplissage élastique ou un dispositif gonflable.

10. Un appareil tel que revendiqué dans la revendication 1 dans lequel le composant cellulaire ou tissulaire est monté sous la couche de support.

11. Un appareil tel que revendiqué dans l'une quelconque des revendications précédentes dans lequel l'appareil est portable.

12. Un appareil tel que revendiqué dans l'une ou l'autre des revendications 1 ou 10 dans lequel le composant cellulaire ou tissulaire est lié sur un substrat insoluble et /ou immobilisé.

13. Un appareil selon la revendication 2 comprenant
a) un premier dispositif (918) destiné à déplacer du fluide à travers la plaie appliqué à du fluide en aval et éloigné du pansement pour plaie, et
b) un deuxième dispositif (926) destiné à déplacer du fluide à travers la plaie appliqué à l'irriguant dans le tube d'apport de fluide en amont et en direction du pansement pour plaie.

14. Un appareil selon la revendication 13 dans lequel le premier dispositif et / ou le deuxième dispositif est un dispositif à débit fixe et le moyen destiné à fournir une aspiration et une irrigation de la plaie comprend aussi au moins un élément parmi : un moyen de régulation d'écoulement d'apport (14), raccordé à un tube d'apport de fluide, et un moyen de régulation d'écoulement d'aspirat (16), raccordé à un tube d'évacuation de fluide.

15. Un appareil tel que revendiqué dans la revendication 13 dans lequel le fluide formant irriguant peut être apporté pour remplir la voie d'écoulement à partir d'un réservoir de fluide via le tube d'apport de fluide tandis que le fluide formant aspirat est aspiré par un dispositif à travers le tube d'évacuation de fluide.

16. Un appareil tel que revendiqué dans l'une quelconque des revendications 13 à 15 dans lequel l'aspiration et l'irrigation de la plaie sont réalisées de façon séquentielle ou simultanée.

17. L'appareil de n'importe quelle revendication précédente dans lequel le dispositif pour déplacer du fluide à travers la plaie est une pompe à diaphragme ou une pompe péristaltique (18)

18. L'appareil de n'importe quelle revendication précédente dans lequel le régime d'écoulement est un régime d'écoulement varié, soit cyclique de façon aléatoire, soit régulièrement cyclique.

19. L'appareil de la revendication 18 dans lequel les cycles régulier ou aléatoire du régime d'écoulement ont une fréquence allant jusqu'à 48 par 24 heures.

20. L'appareil de la revendication 18 ou la revendication 19 dans lequel les impulsions de vitesse d'écoulement ont une fréquence allant de 1 à 60 par min.

21. L'appareil de n'importe quelle revendication précédente dans lequel le dispositif de déplacement de fluide de part et d'autre de la plaie permet à l'écoulement de fluide d'être un écoulement parallèle, un flux radial, un flux en spirale, un flux hélicoïdal, un flux spirohélicoïdal ou un flux circulaire.

22. Un appareil selon l'une quelconque des revendications 13 à 16 dans lequel le premier dispositif est aussi un moyen de vide destiné à créer une pression négative sur la zone entourant la plaie.

23. Un appareil selon la revendication 22 dans lequel la pression négative est comprise entre environ 1,01 et 100,3 kPa (0,01 et 0,99 atmosphères).

24. Un appareil selon la revendication 1, lequel comprend un moyen de nettoyage de fluide qui est un système à phase unique (17), dans lequel le fluide en circulation provenant de la plaie passe à travers le moyen de nettoyage de fluide et des matériaux délétères pour la cicatrisation de la plaie sont éliminés, sans que le fluide en circulation vienne en contact direct ou indirect avec un autre fluide dans le moyen de nettoyage de fluide.

25. Un appareil selon la revendication 1, lequel comprend un moyen de nettoyage de fluide qui est un système à phase double, dans lequel le fluide en circulation provenant de la plaie passe à travers le moyen de nettoyage de fluide et des matériaux délétères pour la cicatrisation de la plaie sont éliminés par le fluide en circulation venant en contact direct ou indirect avec un autre fluide dans le moyen de nettoyage de fluide.

26. Un appareil selon la revendication 24, dans lequel le moyen de nettoyage de fluide, le fluide en circulation provenant de la plaie et l'autre fluide dans le moyen de nettoyage de fluide sont séparés par une partie intégrante qui est perméable de manière sélective à des matériaux délétères pour la cicatrisation de la plaie.

27. Un appareil selon la revendication 25, dans lequel le moyen de nettoyage de fluide, le fluide en circulation provenant de la plaie et l'autre fluide dans le moyen de nettoyage de fluide sont séparés par une partie intégrante qui n'est pas perméable de manière sélective à des matériaux délétères pour la cicatrisation de la plaie, et l'autre fluide comprend et / ou est en contact avec un matériau qui élimine des matériaux délétères pour la cicatrisation de la plaie.

28. Un appareil selon la revendication 25, dans lequel le matériau qui élimine des matériaux délétères pour la cicatrisation de la plaie est un antagoniste, un liant et /ou un agent de dégradation, un chélateur et / ou un échangeur d'ions pour ces matériaux délétères, ou un anti-oxydant.

29. Un appareil selon la revendication 25, dans lequel le matériau qui élimine des matériaux délétères pour la cicatrisation de la plaie est 4-(2-aminoéthyl)-benzène sulfonyl fluorure (AEBSF, PefaBloc), Nα-ρ-tosyl-L-lysine chlorométhylcétone (TLCK), ε-aminocaproyl-ρ-chlorobenzylamide ; un inhibiteur de cystéine-protéase ; un inhibiteur de métalloprotéase matricielle ; un inhibiteur de la protéase (acide) carboxyle ; des peptidomimétiques anti-inflammatoires ; 3-hydroxytramine (dopamine), acide ascorbique (vitamine C), vitamine E ; glutathione; déferoxamine (DFO) et / ou 3-hydroxytyramine (dopamine).

30. Un appareil selon la revendication 1, dans lequel les matériaux délétères pour la cicatrisation de la plaie sont des oxydants ; protéases ; endotoxines ; molécules de signalisation auto-inductrices ; inhibiteurs d'angiogénèse ; cytokines pro-inflammatoires ; et inflammatoires.

31. Un appareil selon n'importe quelle revendication précédente qui administre un traitement sous pression réduite à la plaie.
